# EUROPEAN PATENT SPECIFICATION

(11) **EP 2 866 273 B1**
(45) Date of publication and mention of the grant of the patent: **18.10.2017**
(21) Application number: 14189545.8
(22) Date of filing: 20.10.2014
(51) Int. Cl.: H01L 51/54, C07F 15/00, C07D 403/04, C07D 403/10, C07D 471/04

(54) **Organic electrolumiescent materials and devices**
Organische elektrolumineszente Materialien und Vorrichtungen
Matériaux et dispositifs électroluminescents organiques

(30) Priority: 22.10.2013 US 201361894160 P; 24.12.2013 US 201361920544 P; 17.02.2014 US 201461940603 P; 15.04.2014 US 201414253471
(43) Date of publication of application: 29.04.2015
(73) Proprietor: Universal Display Corporation, Ewing, NJ 08618 (US)
(72) Inventor: Zeng, Lichang, Ewing, New Jersey 08618 (US); Adamovich, Vadim, Ewing, New Jersey 08618 (US); Wang, Ting-Chih, Ewing, New Jersey 08618 (US); Xia, Chuanjun, Ewing, New Jersey 08618 (US); Weaver, Michael S., Ewing, New Jersey 08618 (US); Yamamoto, Hitoshi, Ewing, New Jersey 08618 (US); Alleyne, Bert, Ewing, New York 08618 (US)
(74) Representative: Maiwald Patentanwalts GmbH

(56) References cited:
- EP-A1- 2 166 592
- WO-A1-2011/013843
- WO-A1-2011/136755
- WO-A1-2012/005363
- WO-A1-2012/165844

## Description

### CROSS-REFERENCE TO RELATED APPLICATIONS

This application claims priority under 35 U.S.C. § 119(e) to U.S. Provisional Applications No. 61/940,603, filed on February 17, 2014, No. 61/920,544, filed on December 24, 2013, No. 61/894,160, filed on October 22, 2013, No. 61/874,444, filed on September 06, 2013, and No. 61/867,858, filed on August 20, 2013.

### PARTIES TO A JOINT RESEARCH AGREEMENT

The claimed invention was made by, on behalf of, and/or in connection with one or more of the following parties to a joint university corporation research agreement: Regents of the University of Michigan, Princeton University, University of Southern California, and the Universal Display Corporation. The agreement was in effect on and before the date the claimed invention was made, and the claimed invention was made as a result of activities undertaken within the scope of the agreement.

### FIELD OF THE INVENTION

The present invention relates to organic light emitting devices (OLEDs), and more specifically to organic materials used in such devices. More specifically, the present invention relates to novel premixed host systems for phosphorescent organic light emitting devices (PHOLEDs).

### BACKGROUND

Opto-electronic devices that make use of organic materials are becoming increasingly desirable for a number of reasons. Many of the materials used to make such devices are relatively inexpensive, so organic opto-electronic devices have the potential for cost advantages over inorganic devices. In addition, the inherent properties of organic materials, such as their flexibility, may make them well suited for particular applications such as fabrication on a flexible substrate. Examples of organic opto-electronic devices include organic light emitting devices (OLEDs), organic phototransistors, organic photovoltaic cells, and organic photodetectors. For OLEDs, the organic materials may have performance advantages over conventional materials. For example, the wavelength at which an organic emissive layer emits light may generally be readily tuned with appropriate dopants.

OLEDs make use of thin organic films that emit light when voltage is applied across the device. OLEDs are becoming an increasingly interesting technology for use in applications such as flat panel displays, illumination, and backlighting. Several OLED materials and configurations are described in U.S. Pat. Nos. 5,844,363, 6,303,238, and 5,707,745.

One application for phosphorescent emissive molecules is a full color display. Industry standards for such a display call for pixels adapted to emit particular colors, referred to as "saturated" colors. In particular, these standards call for saturated red, green, and blue pixels. Color may be measured using CIE coordinates, which are well known to the art.

One example of a green emissive molecule is tris(2-phenylpyridine) iridium, denoted Ir(ppy)₃, which has the following structure:

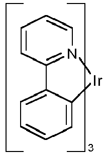

In this, and later figures herein, we depict the dative bond from nitrogen to metal (here, Ir) as a straight line.

As used herein, the term "organic" includes polymeric materials as well as small molecule organic materials that may be used to fabricate organic opto-electronic devices. "Small molecule" refers to any organic material that is not a polymer, and "small molecules" may actually be quite large. Small molecules may include repeat units in some circumstances. For example, using a long chain alkyl group as a substituent does not remove a molecule from the "small molecule" class. Small molecules may also be incorporated into polymers, for example as a pendent group on a polymer backbone or as a part of the backbone. Small molecules may also serve as the core moiety of a dendrimer, which consists of a series of chemical shells built on the core moiety. The core moiety of a dendrimer may be a fluorescent or phosphorescent small molecule emitter. A dendrimer may be a "small molecule," and it is believed that all dendrimers currently used in the field of OLEDs are small molecules.

As used herein, "top" means furthest away from the substrate, while "bottom" means closest to the substrate. Where a first layer is described as "disposed over" a second layer, the first layer is disposed further away from substrate. There may be other layers between the first and second layer, unless it is specified that the first layer is "in contact with" the second layer. For example, a cathode may be described as "disposed over" an anode, even though there are various organic layers in between.

As used herein, "solution processible" means capable of being dissolved, dispersed, or transported in and/or deposited from a liquid medium, either in solution or suspension form.

A ligand may be referred to as "photoactive" when it is believed that the ligand directly contributes to the photoactive properties of an emissive material. A ligand may be referred to as "ancillary" when it is believed that the ligand does not contribute to the photoactive properties of an emissive material, although an ancillary ligand may alter the properties of a photoactive ligand.

As used herein, and as would be generally understood by one skilled in the art, a first "Highest Occupied Molecular Orbital" (HOMO) or "Lowest Unoccupied Molecular Orbital" (LUMO) energy level is "greater than" or "higher than" a second HOMO or LUMO energy level if the first energy level is closer to the vacuum energy level. Since ionization potentials (IP) are measured as a negative energy relative to a vacuum level, a higher HOMO energy level corresponds to an IP having a smaller absolute value (an IP that is less negative). Similarly, a higher LUMO energy level corresponds to an electron affinity (EA) having a smaller absolute value (an EA that is less negative). On a conventional energy level diagram, with the vacuum level at the top, the LUMO energy level of a material is higher than the HOMO energy level of the same material. A "higher" HOMO or LUMO energy level appears closer to the top of such a diagram than a "lower" HOMO or LUMO energy level.

As used herein, and as would be generally understood by one skilled in the art, a first work function is "greater than" or "higher than" a second work function if the first work function has a higher absolute value. Because work functions are generally measured as negative numbers relative to vacuum level, this means that a "higher" work function is more negative. On a conventional energy level diagram, with the vacuum level at the top, a "higher" work function is illustrated as further away from the vacuum level in the downward direction. Thus, the definitions of HOMO and LUMO energy levels follow a different convention than work functions.

More details on OLEDs, and the definitions described above, can be found in US Pat. No. 7,279,704.

WO2011/136755 describes a combination of host materials suitable for co-evaporation or premix evaporation, and OLEDs containing the combination of host materials.

### SUMMARY OF THE INVENTION

According to the invention there is provided a composition as specified in claim 1, and a device as specified in claim 14.

The present disclosure provides a novel host composition comprising a mixture of a first compound and a second compound that is a stable co-evaporation mixture. In the mixture, the first compound has a different chemical structure than the second compound. The first compound is capable of functioning as a hole transporting material in an organic light emitting device at room temperature. Hole transporting material normally has a HOMO level between -5.0 and -6.0 eV. The first compound comprises at least one carbazole group, and has an evaporation temperature T1 of 150 to 350°C. The second compound has a evaporation temperature T2 of 150 to 350°C. In order to form the inventive composition comprising a mixture of a first compound and a second compound, the absolute value of T1-T2, the difference between T1 and T2, is less than 20°C. The evaporation temperatures are measured in a vacuum deposition tool at a constant pressure, between 1x10⁻⁶ Torr to 1x10⁻⁹ Torr, at a 2Å/sec deposition rate. The first compound has a concentration C1 in said mixture, and the first compound has a concentration C2 in a film formed by evaporating the mixture in a vacuum deposition tool at a constant pressure between 1x10⁻⁶ Torr to 1x10⁻⁹ Torr, at a 2Å/sec deposition rate on a surface positioned at a predefined distance away from the mixture being evaporated. The absolute value of (C1-C2)/C1 is less than 5%.

The first compound has a structure according to a formula (I): 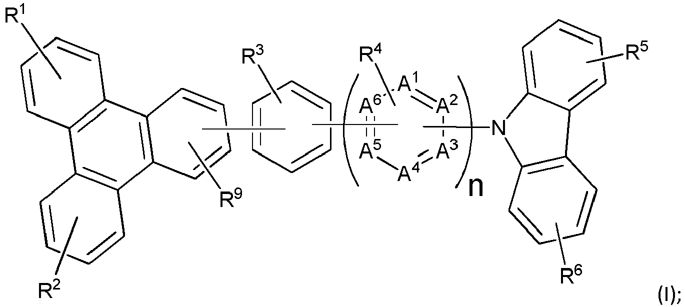 wherein
R¹, R², R³, R⁴, R⁵, and R⁶ each represent mono, di, tri, tetra substitutions, or no substitution;
R⁹ represents mono, di, tri substitutions, or no substitution;
R¹, R², R³, R⁴, R⁵, R⁶, and R⁹ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof;
A¹, A², A³, A⁴, A⁵, and A⁶ are each independently selected from N or C; and
n is an integer from 1 to 20.

The second compound has a structure according to a formula (II): 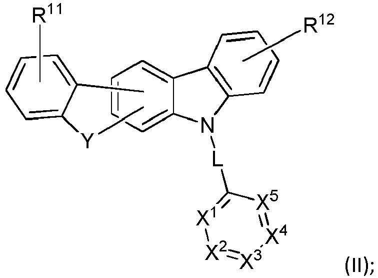 wherein
R¹¹ and R¹² each represent mono, di, tri, tetra substitutions, or no substitution;
Y is selected from the group consisting of O, S, Se, NR', and CR*"*R*"'*;
L is a single bond or comprises an aryl or heteroaryl group having from 5-24 carbon atoms, which is optionally further substituted;
X¹, X², X³, X⁴, and X⁵ are each independently selected from the group consisting of CR and N;
at least one of X¹, X², X³, X⁴, and X⁵ is N; and
R, R', R", and R*'"* are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof.

According to an embodiment of the present disclosure, a first device comprising a phosphorescent organic light-emitting device incorporating the novel host composition is disclosed. The phosphorescent organic light-emitting device comprises: an anode; a cathode; and an organic layer, disposed between the anode and the cathode, comprising an organic composition comprising the mixture of the first compound and the second compound described above.

### BRIEF DESCRIPTION OF THE DRAWINGS

FIG. 1 shows an organic light emitting device that can incorporate the inventive host material disclosed herein.

FIG. 2 shows an inverted organic light emitting device that can incorporate the inventive host material disclosed herein.

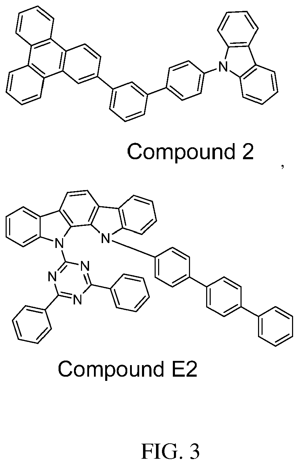

FIG. 3 shows Compounds 2 and E2 representing the first and second compounds which are example components of the novel organic host composition disclosed herein.

### DETAILED DESCRIPTION

Generally, an OLED comprises at least one organic layer disposed between and electrically connected to an anode and a cathode. When a current is applied, the anode injects holes and the cathode injects electrons into the organic layer(s). The injected holes and electrons each migrate toward the oppositely charged electrode. When an electron and hole localize on the same molecule, an "exciton," which is a localized electron-hole pair having an excited energy state, is formed. Light is emitted when the exciton relaxes via a photoemissive mechanism. In some cases, the exciton may be localized on an excimer or an exciplex. Non-radiative mechanisms, such as thermal relaxation, may also occur, but are generally considered undesirable.

The initial OLEDs used emissive molecules that emitted light from their singlet states ("fluorescence") as disclosed, for example, in U.S. Pat. No. 4,769,292. Fluorescent emission generally occurs in a time frame of less than 10 nanoseconds.

More recently, OLEDs having emissive materials that emit light from triplet states ("phosphorescence") have been demonstrated. Baldo et al., "Highly Efficient Phosphorescent Emission from Organic Electroluminescent Devices," Nature, vol. 395, 151-154, 1998; ("Baldo-I") and Baldo et al., "Very high-efficiency green organic light-emitting devices based on electrophosphorescence," Appl. Phys. Lett., vol. 75, No. 3, 4-6 (1999) ("Baldo-II"). Phosphorescence is described in more detail in US Pat. No. 7,279,704 at cols. 5-6.

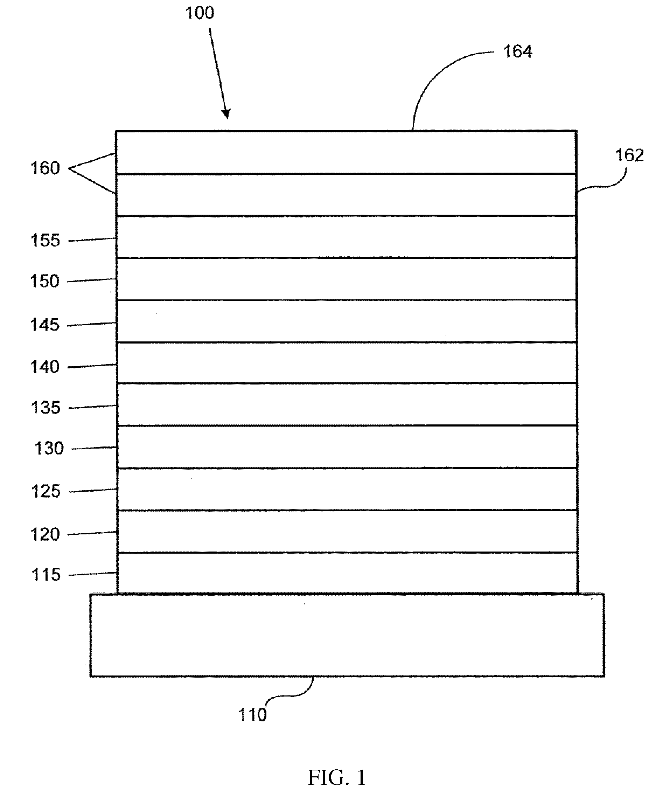

FIG. 1 shows an organic light emitting device **100.** The figures are not necessarily drawn to scale. Device **100** may include a substrate **110,** an anode **115,** a hole injection layer **120,** a hole transport layer **125,** an electron blocking layer **130,** an emissive layer **135,** a hole blocking layer **140,** an electron transport layer **145,** an electron injection layer **150,** a protective layer **155,** a cathode **160,** and a barrier layer **170.** Cathode **160** is a compound cathode having a first conductive layer **162** and a second conductive layer **164.** Device **100** may be fabricated by depositing the layers described, in order. The properties and functions of these various layers, as well as example materials, are described in more detail in US 7,279,704 at cols. 6-10.

More examples for each of these layers are available. For example, a flexible and transparent substrate-anode combination is disclosed in U.S. Pat. No. 5,844,363. An example of a p-doped hole transport layer is m-MTDATA doped with F₄-TCNQ at a molar ratio of 50:1, as disclosed in U.S. Patent Application Publication No. 2003/0230980. Examples of emissive and host materials are disclosed in U.S. Pat. No. 6,303,238 to Thompson et al.. An example of an n-doped electron transport layer is BPhen doped with Li at a molar ratio of 1:1, as disclosed in U.S. Patent Application Publication No. 2003/0230980. U.S. Pat. Nos. 5,703,436 and 5,707,745 disclose examples of cathodes including compound cathodes having a thin layer of metal such as Mg:Ag with an overlying transparent, electrically-conductive, sputter-deposited ITO layer. The theory and use of blocking layers is described in more detail in U.S. Pat. No. 6,097,147 and U.S. Patent Application Publication No. 2003/0230980. Examples of injection layers are provided in U.S. Patent Application Publication No. 2004/0174116. A description of protective layers may be found in U.S. Patent Application Publication No. 2004/0174116.

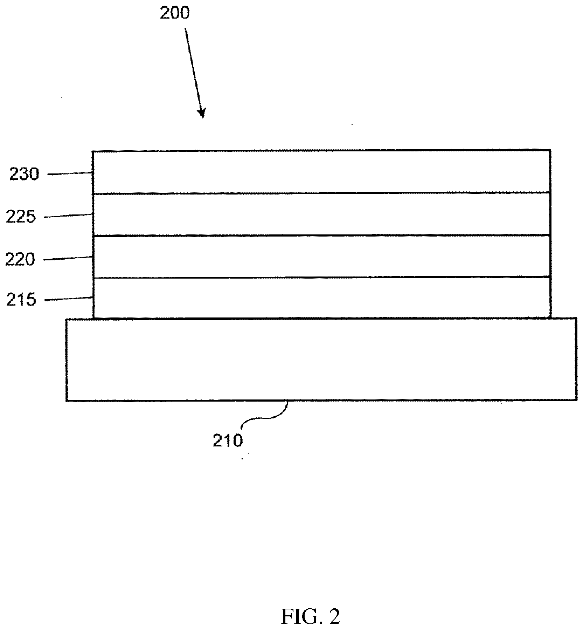

FIG. 2 shows an inverted OLED **200.** The device includes a substrate **210,** a cathode **215,** an emissive layer **220,** a hole transport layer **225,** and an anode **230.** Device **200** may be fabricated by depositing the layers described, in order. Because the most common OLED configuration has a cathode disposed over the anode, and device **200** has cathode **215** disposed under anode **230,** device **200** may be referred to as an "inverted" OLED. Materials similar to those described with respect to device **100** may be used in the corresponding layers of device **200.** FIG. 2 provides one example of how some layers may be omitted from the structure of device **100.**

The simple layered structure illustrated in FIGS. 1 and 2 is provided by way of non-limiting example, and it is understood that embodiments of the invention may be used in connection with a wide variety of other structures. The specific materials and structures described are exemplary in nature, and other materials and structures may be used. Functional OLEDs may be achieved by combining the various layers described in different ways, or layers may be omitted entirely, based on design, performance, and cost factors. Other layers not specifically described may also be included. Materials other than those specifically described may be used. Although many of the examples provided herein describe various layers as comprising a single material, it is understood that combinations of materials, such as a mixture of host and dopant, or more generally a mixture, may be used. Also, the layers may have various sublayers. The names given to the various layers herein are not intended to be strictly limiting. For example, in device **200,** hole transport layer **225** transports holes and injects holes into emissive layer **220,** and may be described as a hole transport layer or a hole injection layer. In one embodiment, an OLED may be described as having an "organic layer" disposed between a cathode and an anode. This organic layer may comprise a single layer, or may further comprise multiple layers of different organic materials as described, for example, with respect to FIGS. 1 and 2.

Structures and materials not specifically described may also be used, such as OLEDs comprised of polymeric materials (PLEDs) such as disclosed in U.S. Pat. No. 5,247,190 to Friend et al.. By way of further example, OLEDs having a single organic layer may be used. OLEDs may be stacked, for example as described in U.S. Pat. No. 5,707,745 to Forrest et al.. The OLED structure may deviate from the simple layered structure illustrated in FIGS. 1 and 2. For example, the substrate may include an angled reflective surface to improve out-coupling, such as a mesa structure as described in U.S. Pat. No. 6,091,195 to Forrest et al., and/or a pit structure as described in U.S. Pat. No. 5,834,893 to Bulovic et al..

Unless otherwise specified, any of the layers of the various embodiments may be deposited by any suitable method. For the organic layers, preferred methods include thermal evaporation, ink-jet, such as described in U.S. Pat. Nos. 6,013,982 and 6,087,196, organic vapor phase deposition (OVPD), such as described in U.S. Pat. No. 6,337,102 to Forrest et al., and deposition by organic vapor jet printing (OVJP), such as described in U.S. Pat. No. 7,431,968. Other suitable deposition methods include spin coating and other solution based processes. Solution based processes are preferably carried out in nitrogen or an inert atmosphere. For the other layers, preferred methods include thermal evaporation. Preferred patterning methods include deposition through a mask, cold welding such as described in U.S. Pat. Nos. 6,294,398 and 6,468,819, and patterning associated with some of the deposition methods such as ink-jet and OVJD. Other methods may also be used. The materials to be deposited may be modified to make them compatible with a particular deposition method. For example, substituents such as alkyl and aryl groups, branched or unbranched, and preferably containing at least 3 carbons, may be used in small molecules to enhance their ability to undergo solution processing. Substituents having 20 carbons or more may be used, and 3-20 carbons is a preferred range. Materials with asymmetric structures may have better solution processability than those having symmetric structures, because asymmetric materials may have a lower tendency to recrystallize. Dendrimer substituents may be used to enhance the ability of small molecules to undergo solution processing.

Devices in accordance with embodiments of the present invention may further optionally comprise a barrier layer. One purpose of the barrier layer is to protect the electrodes and organic layers from damaging exposure to harmful species in the environment including moisture, vapor and/or gases. The barrier layer may be deposited over, under or next to a substrate, an electrode, or over any other parts of a device including an edge. The barrier layer may comprise a single layer, or multiple layers. The barrier layer may be formed by various known chemical vapor deposition techniques and may include compositions having a single phase as well as compositions having multiple phases. Any suitable material or combination of materials may be used for the barrier layer. The barrier layer may incorporate an inorganic or an organic compound or both. The preferred barrier layer comprises a mixture of a polymeric material and a non-polymeric material as described in U.S. Pat. No. 7,968,146, PCT Pat. Application Nos. PCT/US2007/023098 and PCT/US2009/042829. To be considered a "mixture", the aforesaid polymeric and non-polymeric materials comprising the barrier layer should be deposited under the same reaction conditions and/or at the same time. The weight ratio of polymeric to non-polymeric material may be in the range of 95:5 to 5:95. The polymeric material and the non-polymeric material may be created from the same precursor material. In one example, the mixture of a polymeric material and a non-polymeric material consists essentially of polymeric silicon and inorganic silicon.

Devices in accordance with embodiments of the invention may be incorporated into a wide variety of consumer products, including flat panel displays, computer monitors, medical monitors, televisions, billboards, lights for interior or exterior illumination and/or signaling, heads up displays, fully transparent displays, flexible displays, laser printers, telephones, cell phones, personal digital assistants (PDAs), laptop computers, digital cameras, camcorders, viewfinders, micro-displays, 3-D displays, vehicles, a large area wall, theater or stadium screen, or a sign. Various control mechanisms may be used to control devices in accordance with the present invention, including passive matrix and active matrix. Many of the devices are intended for use in a temperature range comfortable to humans, such as 18 degrees C to 30 degrees C, and more preferably at room temperature (20-25 degrees C), but could be used outside this temperature range, for example, from -40 degree C to + 80 degree C.

The materials and structures described herein may have applications in devices other than OLEDs. For example, other optoelectronic devices such as organic solar cells and organic photodetectors may employ the materials and structures. More generally, organic devices, such as organic transistors, may employ the materials and structures.

The term "halo" or "halogen" as used herein includes fluorine, chlorine, bromine, and iodine.

The term "alkyl" as used herein contemplates both straight and branched chain alkyl radicals. Preferred alkyl groups are those containing from one to fifteen carbon atoms and include methyl, ethyl, propyl, isopropyl, butyl, isobutyl, tert-butyl, and the like. Additionally, the alkyl group may be optionally substituted.

The term "cycloalkyl" as used herein contemplates cyclic alkyl radicals. Preferred cycloalkyl groups are those containing 3 to 7 carbon atoms and include cyclopropyl, cyclopentyl, cyclohexyl, and the like. Additionally, the cycloalkyl group may be optionally substituted.

The term "alkenyl" as used herein contemplates both straight and branched chain alkene radicals. Preferred alkenyl groups are those containing two to fifteen carbon atoms. Additionally, the alkenyl group may be optionally substituted.

The term "alkynyl" as used herein contemplates both straight and branched chain alkyne radicals. Preferred alkyl groups are those containing two to fifteen carbon atoms. Additionally, the alkynyl group may be optionally substituted.

The terms "aralkyl" or "arylalkyl" as used herein are used interchangeably and contemplate an alkyl group that has as a substituent an aromatic group. Additionally, the aralkyl group may be optionally substituted.

The term "heterocyclic group" as used herein contemplates aromatic and non-aromatic cyclic radicals. Hetero-aromatic cyclic radicals also refer to heteroaryl. Preferred hetero-non-aromatic cyclic groups are those containing 3 or 7 ring atoms which include at least one hetero atom, and include cyclic amines such as morpholino, piperidino, pyrrolidino, and the like, and cyclic ethers, such as tetrahydrofuran, tetrahydropyran, and the like. Additionally, the heterocyclic group may be optionally substituted.

The term "aryl" or "aromatic group" as used herein contemplates single-ring groups and polycyclic ring systems. The polycyclic rings may have two or more rings in which two carbons are common to two adjoining rings (the rings are "fused") wherein at least one of the rings is aromatic, e.g., the other rings can be cycloalkyls, cycloalkenyls, aryl, heterocycles, and/or heteroaryls. Additionally, the aryl group may be optionally substituted.

The term "heteroaryl" as used herein contemplates single-ring hetero-aromatic groups that may include from one to three heteroatoms, for example, pyrrole, furan, thiophene, imidazole, oxazole, thiazole, triazole, pyrazole, pyridine, pyrazine and pyrimidine, and the like. The term heteroaryl also includes polycyclic hetero-aromatic systems having two or more rings in which two atoms are common to two adjoining rings (the rings are "fused") wherein at least one of the rings is a heteroaryl, e.g., the other rings can be cycloalkyls, cycloalkenyls, aryl, heterocycles, and/or heteroaryls. Additionally, the heteroaryl group may be optionally substituted.

The alkyl, cycloalkyl, alkenyl, alkynyl, aralkyl, heterocyclic group, aryl, and heteroaryl may be optionally substituted with one or more substituents selected from the group consisting of hydrogen, deuterium, halogen, alkyl, cycloalkyl, heteroalkyl, arylalkyl, alkoxy, aryloxy, amino, cyclic amino, silyl, alkenyl, cycloalkenyl, heteroalkenyl, alkynyl, aryl, heteroaryl, acyl, carbonyl, carboxylic acid, ether, ester, nitrile, isonitrile, sulfanyl, sulfinyl, sulfonyl, phosphino, and combinations thereof.

As used herein, "substituted" indicates that a substituent other than H is bonded to the relevant position, such as carbon. Thus, for example, where R¹ is mono-substituted, then one R¹ must be other than H. Similarly, where R¹ is di-substituted, then two of R¹ must be other than H. Similarly, where R¹ is unsubstituted, R¹ is hydrogen for all available positions.

The "aza" designation in the fragments described herein, i.e. aza-dibenzofuran, aza-dibenzothiophene, etc. means that one or more of the C-H groups in the respective fragment can be replaced by a nitrogen atom, for example, and without any limitation, azatriphenylene encompasses both dibenzo[*f,h*]quinoxaline and dibenzo[*f,h*]quinoline.

It is to be understood that when a molecular fragment is described as being a substituent or otherwise attached to another moiety, its name may be written as if it were a fragment (e.g. naphthyl, dibenzofuryl) or as if it were the whole molecule (e.g. naphthalene, dibenzofuran). As used herein, these different ways of designating a substituent or attached fragment are considered to be equivalent.

Often, the emissive layer (EML) of OLED devices exhibiting good lifetime and efficiency requires more than two components (e.g. 3 or 4 components). For this purpose, 3 or 4 source materials are required to fabricate such an EML, which is very complicated and costly compared to a standard two-component EML with a single host and an emitter, which requires only two sources. Premixing two or more materials and evaporating them from one source can reduce the complexity of the fabrication process. Selected hole transporting hosts and another type of hosts, such as electron transporting hosts can be mixed and co-evaporated from one crucible and achieve stable evaporation.

However, the co-evaporation must be stable, *i.e.* the composition of the evaporated film should remain constant during the manufacturing process. Any composition change may affect the device performance adversely. In order to obtain a stable co-evaporation from a mixture of compounds under vacuum, one would assume that the materials must have the same evaporation temperature under the same condition. However, this may not be the only parameter one has to consider. When the two compounds are mixed together, they may interact with each other and their evaporation properties may differ from their individual properties. On the other hand, materials with slightly different evaporation temperatures may form a stable co-evaporation mixture. Therefore, it is extremely difficult to achieve a stable co-evaporation mixture. So far, there have been very few stable co-evaporation mixture examples despite that there have been tens of thousands of hosts materials available in the literature. "Evaporation temperature" of a material is measured in a vacuum deposition tool at a constant pressure, between 1x10⁻⁶ Torr to 1x10⁻⁹ Torr, at a 2Å/sec deposition rate on a surface positioned at a set distance away from the evaporation source of the material being evaporated, e.g. sublimation crucible in a VTE tool. The various measured values such as temperature, pressure, deposition rate, etc. disclosed herein are expected to have nominal variations because of the expected tolerances in the measurements that produced these quantitative values as understood by one of ordinary skill in the art.

This disclosure describes the new class of hole transporting and electron transporting hosts which can be premixed to provide a stable co-evaporation mixture that is useful as a host material for green phosphorescent emitters. Many factors other than temperatures can contribute to the evaporation, such as miscibility of different materials, different phase transition. The inventors found that when two materials have similar evaporation temperature, and similar mass loss rate or similar vapor pressure, the two materials can co-evaporate consistently. Mass loss rate is defined as percentage of mass lost over time (minute) and is determined by measuring the time it takes to lose the first 10% of the mass as measured by thermal gravity analysis (TGA) under same experimental condition at a same constant given temperature for each compound after the composition reaches a steady evaporation state. The constant given temperature is one temperature point that is chosen so that the value of mass loss rate is between about 0.05 to 0.50 percentage/min. A skilled person in this field should appreciate that in order to compare two parameters, the experimental condition should be consistent. The method of measuring mass loss rate and vapor pressure is well known in the art and can be found, for example, in Bull. et al. Mater. Sci. 2011, 34, 7.

Current state of the art green phosphorescent host materials consist of two components: a hole transporting cohost (h-host) and an electron transporting cohost (e-host). Some examples of such green phosphorescent host materials are disclosed for example in United States patent No. 6,803,720. In order to fabricate EML of a PHOLED with such host material, three evaporation sources are required: one for h-host, another one for e-host, and a third one for the emitter. The concentration of the cohost and the emitter is critical for the device performance and typically, the rate of deposition of each component is measured individually during the deposition. This makes the fabrication process complicated and costly. Thus, it is desired to mix at least two of the components to reduce the number of sources.

The host compounds Compound 2 and Compound E2 show stable premixability, which means they can be premixed and codeposited from one source without changing the composition. Uniform co-evaporation of the two hosts is critical for the consistency of the devices performance fabricated from this mixture.

The premixability of these two compounds was tested by high pressure liquid chromatography (HPLC) analysis of evaporated films. For this purpose, 0.15 g of Compound 2 and 0.15 g of Compound E2 were mixed and ground. 0.3 g of the mixture was loaded into the evaporation source of a VTE vacuum chamber. The chamber was pumped down to 10⁻⁷ Torr pressure. The premixed components were deposited at the rate of 2 Å/s onto glass substrates. The substrates were replaced continuously after deposition of 600 Å film without stopping the deposition process so that the source is maintained at proper temperature and avoid cooling the source. The deposited films were analyzed by HPLC and results are shown in Table 1 below. Seven such substrate samples were taken and are labeled as Plate1 to Plate7 in Table 1. The composition of the components Compound 2 and Compound E2 did not change significantly from Plate1 to Plate7. Some fluctuations in the concentrations do not reveal any trend and can be explained by the accuracy of sample collection and HPLC analysis. Normally, the change of the concentration before and after depositions within 5% throughout the process is considered to be good and useful for commercial OLED application.

**Table 1. HPLC composition (%) of sequentially deposited films from premixed hosts, (h-host:e-host), with ratio 1:1. (HPLC Conditions C18, 100 45 min, Detected wavelength 254 nm)**

| Films (600Å) | Compound 2 (h-host) | Compound E2 (e-host) |
|---|---|---|
| Plate1 | 54.1 | 45.9 |
| Plate2 | 57.3 | 42.7 |
| Plate3 | 56.9 | 43.1 |
| Plate4 | 56.7 | 43.3 |
| Plate5 | 56.4 | 43.6 |
| Plate6 | 56.1 | 43.9 |
| Plate7 | 54.6 | 45.4 |

This is evidence that Compound 2 and Compound E2 form a stable co-evaporation mixture. Inventors conclude that other hosts from these families can be premixed to be used as hosts for PHOLEDs. Table 2 below shows some representative examples which the inventors believe are suitable for pre-mixing and evaporation from a common source based on the compounds' evaporation temperature and their similar chemical structures.

**Table 2. Examples of possible premix pairs.**

| Mixture number | Hole transporting host | Electron transporting host |
|---|---|---|
| 1 | Compound 2 | Compound E2 |
| 2 | Compound 3 | Compound E4 |
| 3 | Compound 1 | Compound E5 |
| 4 | Compound 6 | Compound E7 |
| 5 | Compound 7 | Compound E7 |
| 6 | Compound 9 | Compound E8 |
| 7 | Compound 17 | Compound E8 |
| 8 | Compound 15 | Compound E18 |

HOMO and LUMO levels of selected compounds for the possible premix pairs are listed in Table 3 below. The HOMO/LUMO levels were obtained through differential pulse voltammetry in DMF solutions at a concentration of 10⁻³ M, with 0.1 M tetrabutylammonium hexafluorophosphate as the supporting electrolyte. A glass carbon disk, a platinum wire and a silver wire are used as the working, counter and pseudo reference electrodes, respectively. Ferrocene is added into the solution to serve as the internal standard for each measurement. The resultant oxidation potential (Eₒₓ) and reduction potential (E_{red}), adjusted to ferrocene, are used to calculate the HOMO/LUMO levels as-4.8 eV - q Eₒₓ and -4.8 eV - qE_{red}, respectively, where q is the electron charge.

**Table 3. HOMO and LUMO levels of selected compounds for premix pairs.**

| Compound | HOMO (eV) | LUMO (eV) |
|---|---|---|
| Compound 1 | -5.7 | -2.1 |
| Compound 2 | -5.7 | -2.1 |
| Compound E2 | -5.6 | -2.7 |
| Compound E69 | -5.6 | -2.7 |

Having HOMO and LUMO levels at -5.7 eV and -2.1 eV, respectively, both Compounds 1 and 2 are considered as hole-transporting hosts. On the other hand, Compounds E2 and E69 have deep LUMO levels at-2.7 eV and are used as electron-transporting hosts.
Structures of the host compounds:

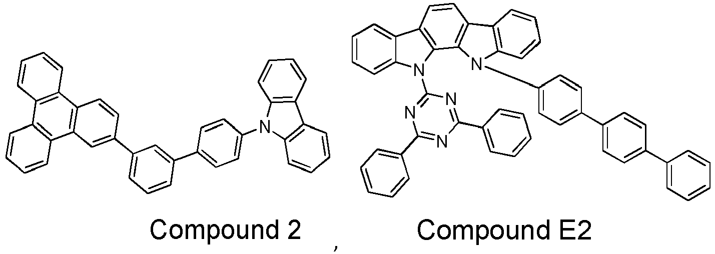

According to an embodiment of the present disclosure, a novel host composition comprising a mixture of a first compound and a second compound that is a stable co-evaporation mixture is disclosed. In the mixture, the first compound has different chemical structure than the second compound. The first compound is capable of functioning as a hole transporting material in an organic light emitting device at room temperature. The first compound can have a HOMO level between -5.0 and -6.0 eV. The first compound comprises at least one carbazole group, and has an evaporation temperature T1 of 150 to 350°C. The second compound has an evaporation temperature T2 of 150 to 350°C. In order to form the inventive composition comprising a mixture of a first compound and a second compound, the absolute value of T1-T2, the difference between T1 and T2, is less than 20°C. Preferably, the absolute value of T1-T2 is less than 10 °C and more preferably less than 5 °C.

The first compound has a concentration C1 in the mixture, and the first compound has a concentration C2 in a film formed by evaporating the mixture in a vacuum deposition tool, such as a VTE tool, at a constant pressure between 1x10⁻⁶ Torr to 1x10⁻⁹ Torr, at a 2Å/sec deposition rate on a surface positioned at a predefined distance away from the evaporation source of the mixture being evaporated; and wherein the absolute value of (C1-C2)/C1 is less than 5%. The concentrations C1 and C2 are relative concentrations of the first compound. Therefore, the conditional requirement for the two compounds forming the mixture described above means that the relative concentration of the first compound in the as-deposited film (C2) should be as close to the original relative concentration of the first compound (C1) in the evaporation source mixture. One of ordinary skill in this field should realize that the concentration of each component is expressed as a relative percentage. The concentration of each component in the mixture can be measured by a suitable analytical method such as high pressure liquid chromatography (HPLC) and nuclear magnetic resonance spectroscopy (NMR).

The inventors used HPLC and the percentage was calculated by dividing the integration area under the HPLC trace of each component by the total integration area. HPLC can use different detectors such as UV-vis, photo diode array detector, refractive index detector, fluorescence detector, and light scattering detector. Due to different materials properties, each component in the mixture may respond differently. Therefore, the measured concentration may differ from their real concentration in the mixture, however the relative ratio value of (C1-C2)/C1 is independent of these variables as long as the experimental condition is kept consistent, for example, all concentrations should be calculated under the exact same HPLC parameters for each component. It is sometimes preferred to select a measurement condition that gives calculated concentration close to the real concentration. However, it is not necessary. It is important to select a detecting condition that accurately detects each component. For example, a fluorescence detector should not be used if one of the components does not fluoresce.

In another embodiment of the host composition, the first compound has a evaporation temperature T1 of 200 to 350 °C and the second compound has a evaporation temperature T2 of 200 to 350°C. Preferably, the absolute value of (C1-C2)/C1 is less than 3%.

In one embodiment of the host composition, the first compound has a vapor pressure of P1 at T1, the second compound has vapor pressure of P2 at T2, and the ratio of P1/P2 is within the range of 0.90 to 1.10.

The first compound has a first mass loss rate and the second compound has a second mass loss rate, wherein the ratio between the first mass loss rate and the second mass loss rate is within the range of 0.90 to 1.10, including the endpoints. In one embodiment, the ratio between the first mass loss rate and the second mass loss rate is within the range of 0.95 to 1.05, including the endpoints. Preferably, the ratio between the first mass loss rate and the second mass loss rate is within the range of 0.97 to 1.03, including the endpoints.

The second compound is capable of functioning as an electron transporting material in an organic light emitting device at room temperature. The second compound can have a LUMO level between -2.3 and -3.3 eV.

In one embodiment, the first compound has HOMO energy level higher than -5.7 eV. In another embodiment, the first compound has HOMO energy level higher than -5.5 eV. Preferably, the first compound has HOMO energy level higher than -5.8 eV.

Preferably, the first compound and the second compound each has a purity in excess of 99 % as determined by high pressure liquid chromatography.

According to one embodiment, the novel composition can further comprise a third compound. The third compound has a different chemical structure than the first compound and the second compound, wherein the third compound has an evaporation temperature T3 of 150 to 350 ºC; and wherein the absolute value of T1-T3 is less than 20 ºC.

According to one embodiment, the composition is in liquid form at a temperature lower than T1 and T2. In other words, where T1 and T2 are not equal, the composition is in liquid form at a temperature lower than the lower of T1 and T2.

The first compound has the formula (I): 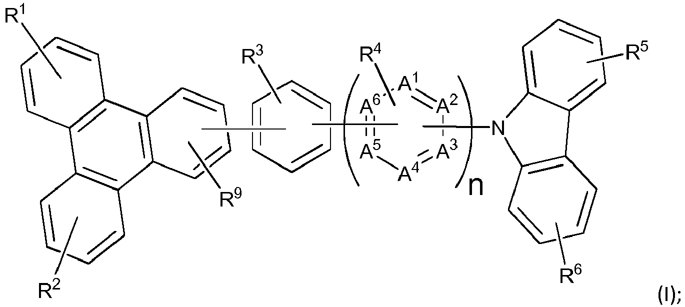 wherein
R¹, R², R³, R⁴, R⁵, and R⁶ each represent mono, di, tri, tetra substitutions, or no substitution;
R⁹ represents mono, di, tri substitutions, or no substitution;
R¹, R², R³, R⁴, R⁵, R⁶, and R⁹ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof;
A¹, A², A³, A⁴, A⁵, and A⁶ are each independently selected from N or C; and
n is an integer from 1 to 20.

The second compound has the formula (II): 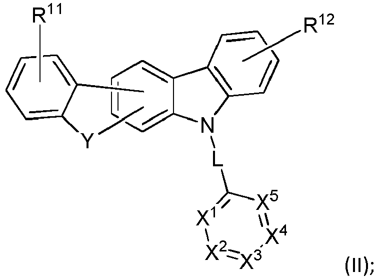 wherein
R¹¹ and R¹² each represent mono, di, tri, tetra substitutions, or no substitution;
Y is selected from the group consisting of O, S. Se, NR', and CR*"*R*"'*;
L is a single bond or comprises an aryl or heteroaryl group having from 5-24 carbon atoms, which is optionally further substituted;
X¹, X², X³, X⁴, and X⁵ are each independently selected from group consisting of CR and N;
at least one of X¹, X², X³, X⁴, and X⁵ is N; and
R, R', R", and R*'"* are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof.

In one embodiment, in the formula (II) for the second compound, X¹, X³, and X⁵ are N; and X² and X⁴ are CR.

According to an embodiment of the host composition, the first compound has the formula selected from the group consisting of: 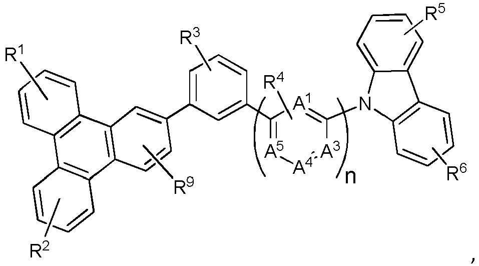 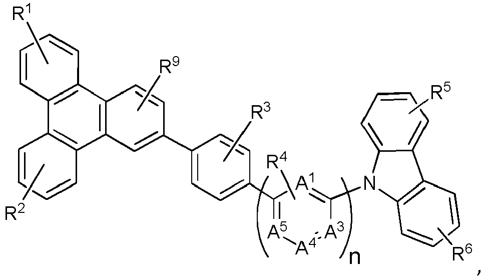 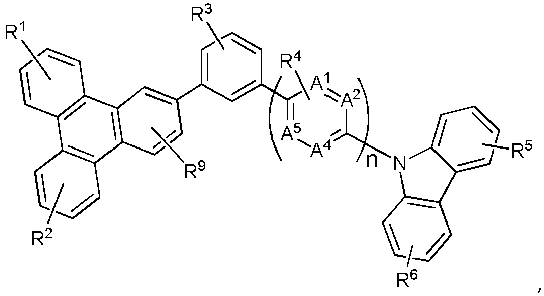 and

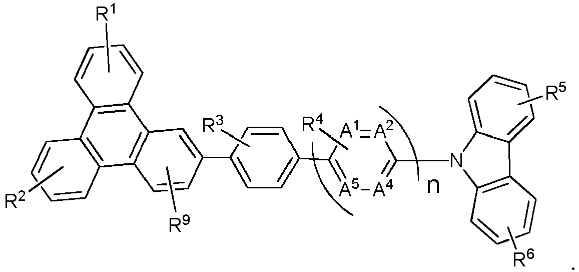

In another embodiment of the host composition, the first compound has the formula selected from the group consisting of: 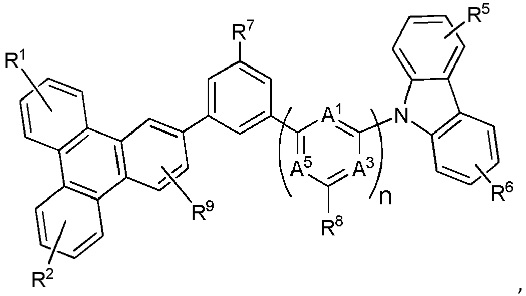 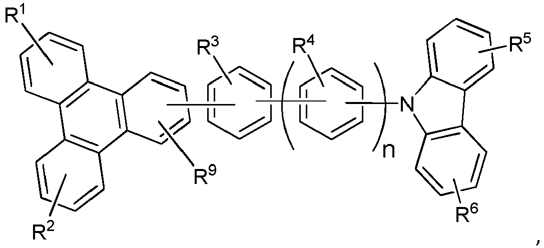 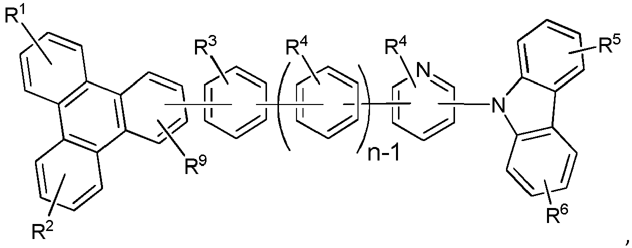 and 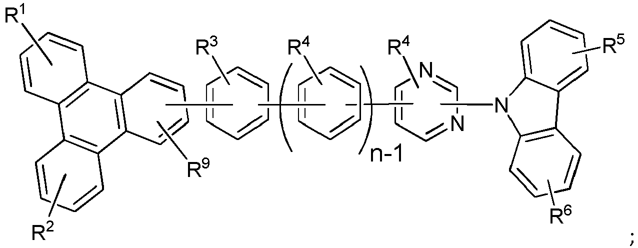 wherein R⁷ and R⁸ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof.

In one embodiment, the first compound is selected from the group consisting of: 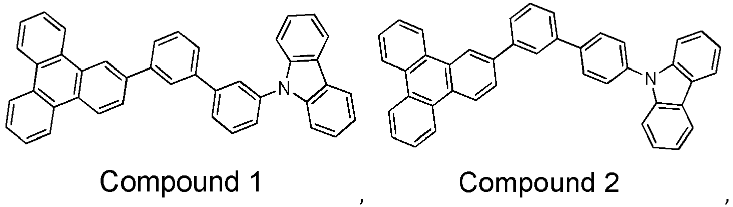 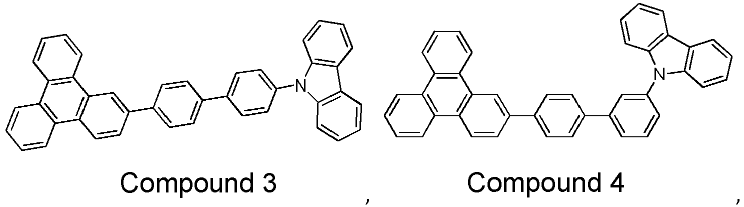 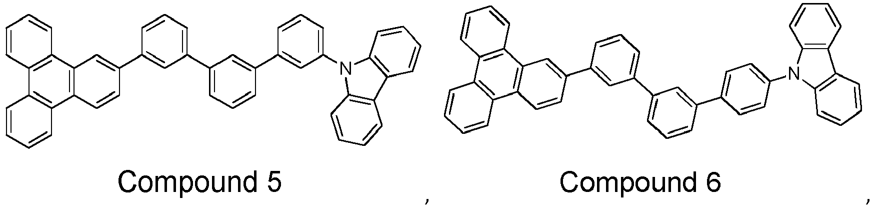 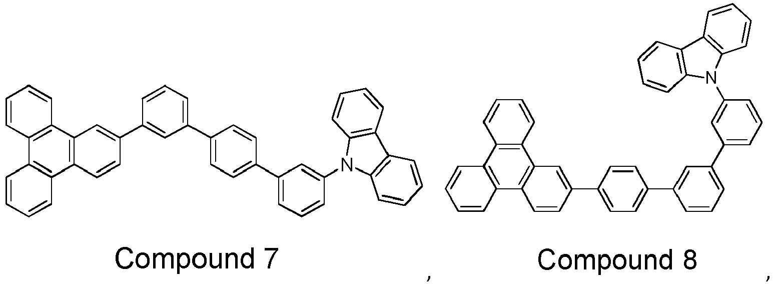 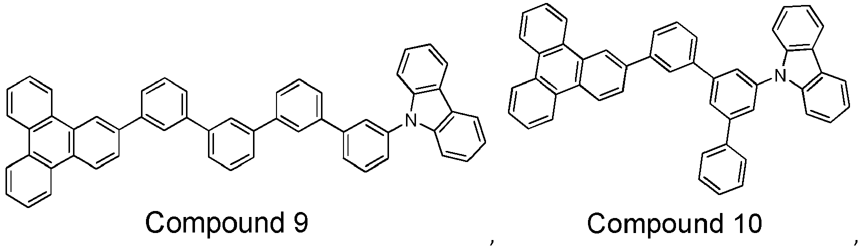 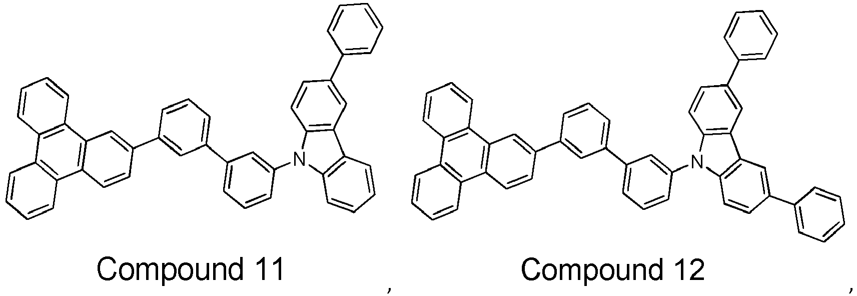 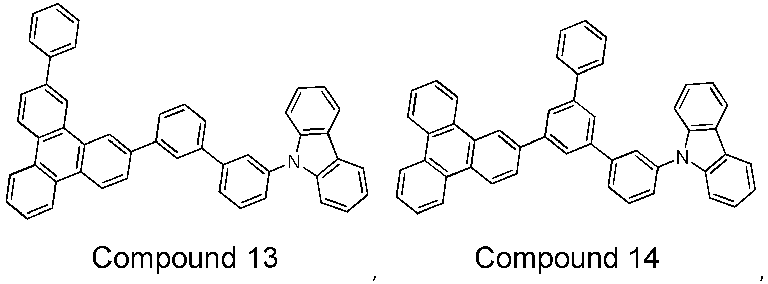 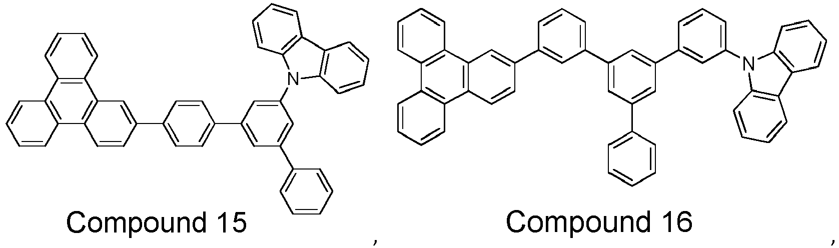 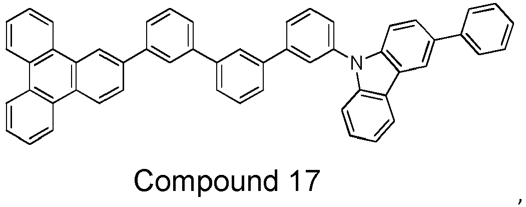 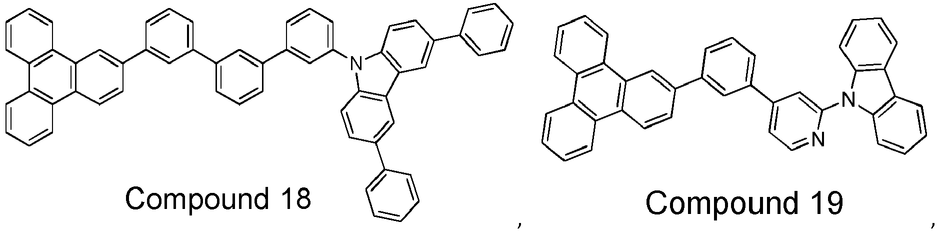 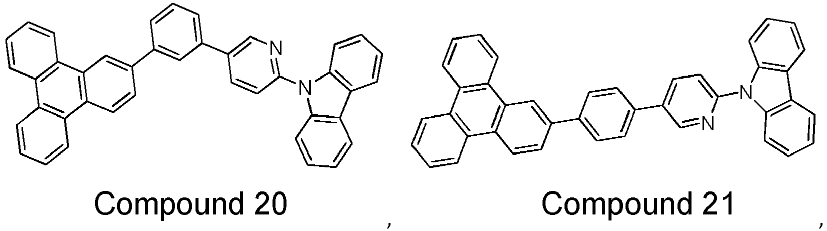 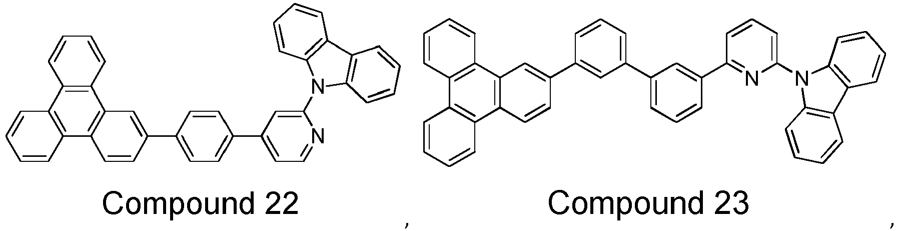 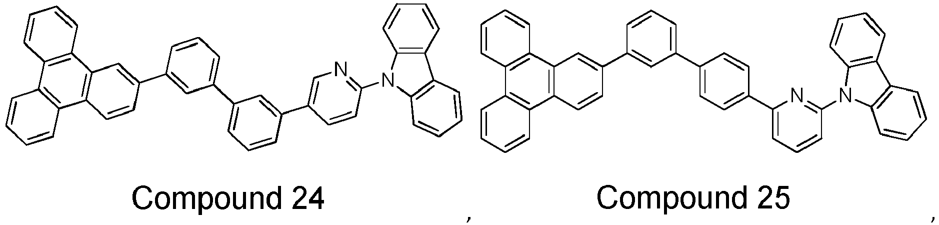 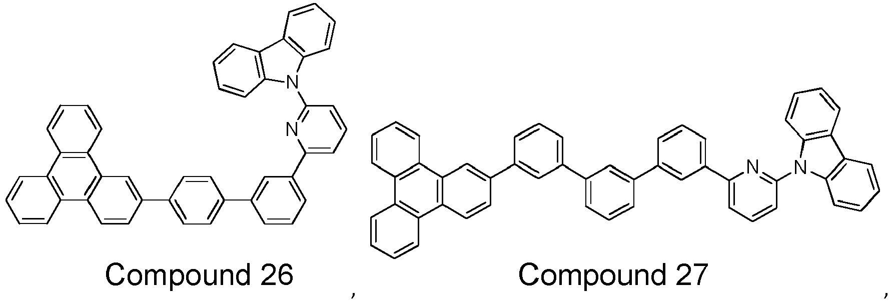 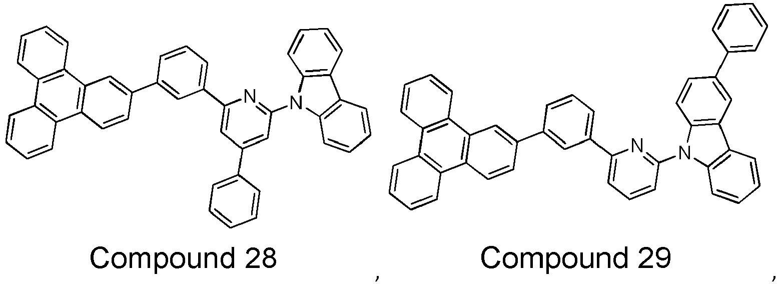 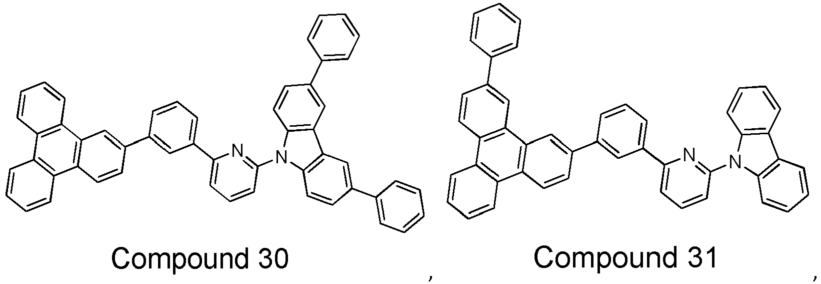 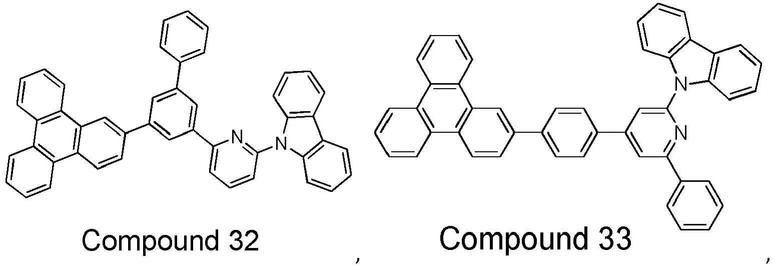 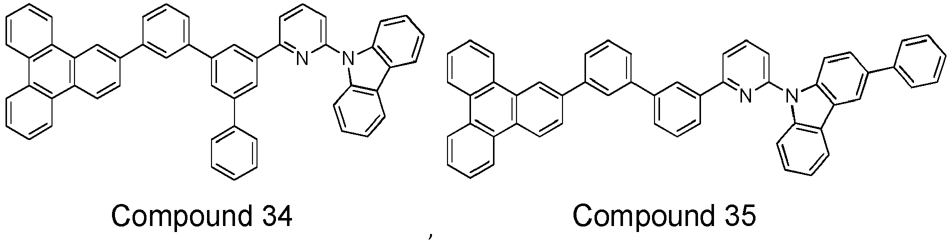 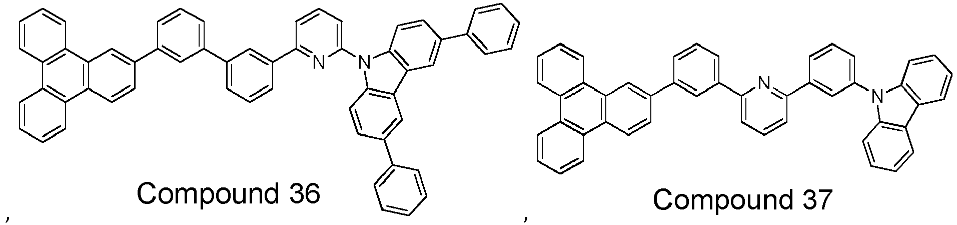 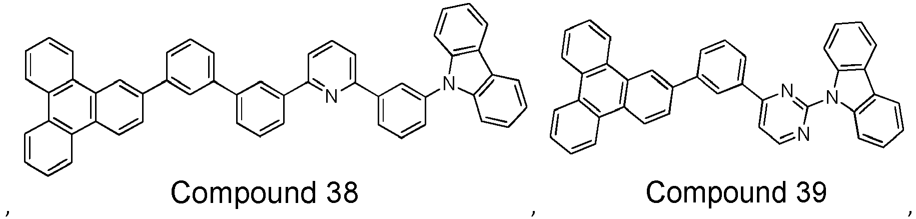 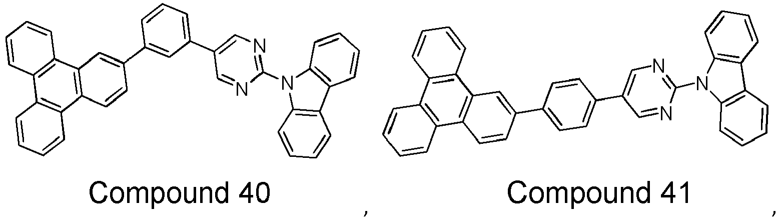 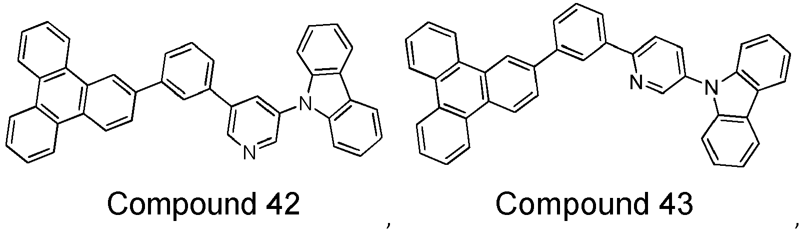 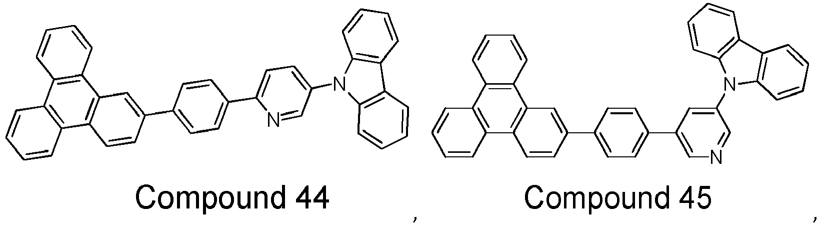 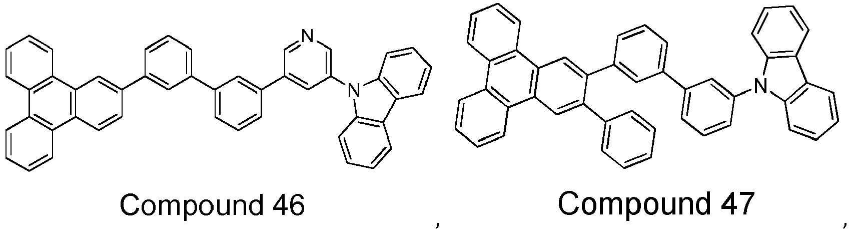 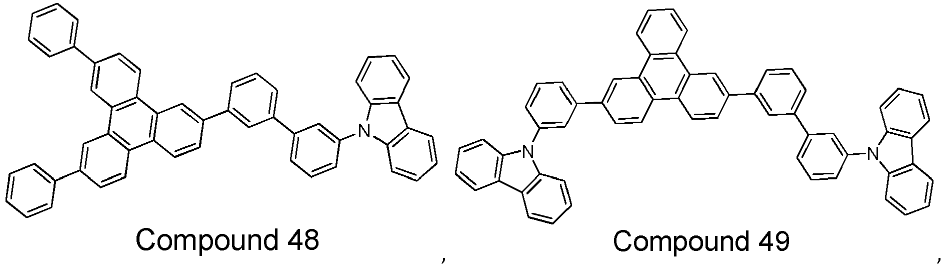 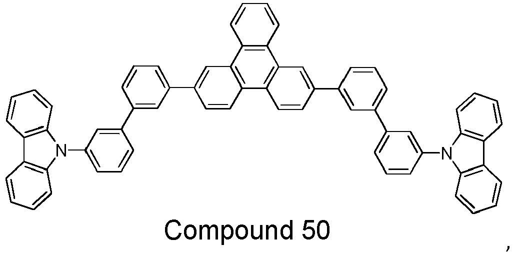 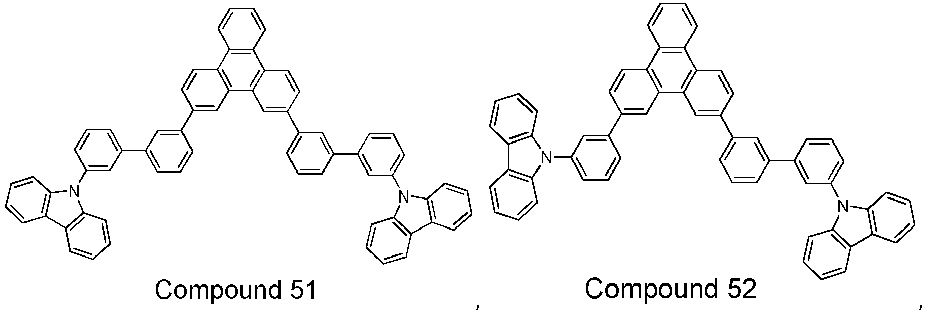 and

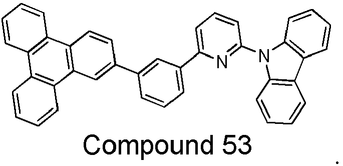

In one embodiment of the host composition, the second compound is selected from the group consisting of:

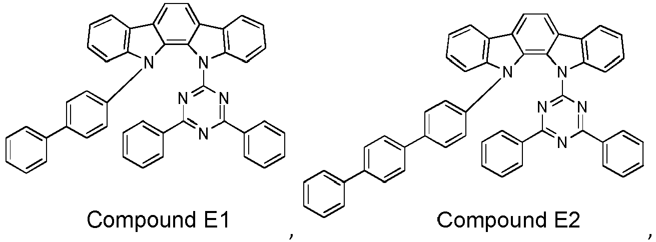

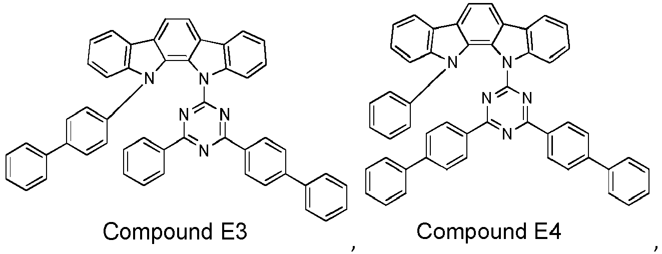

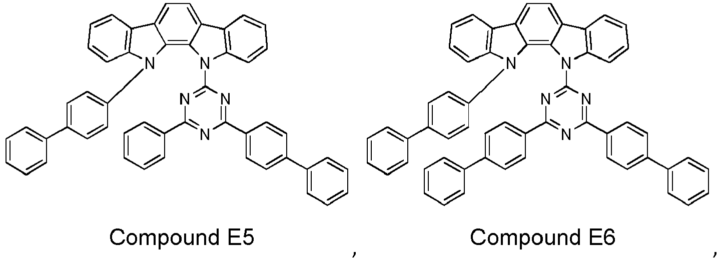

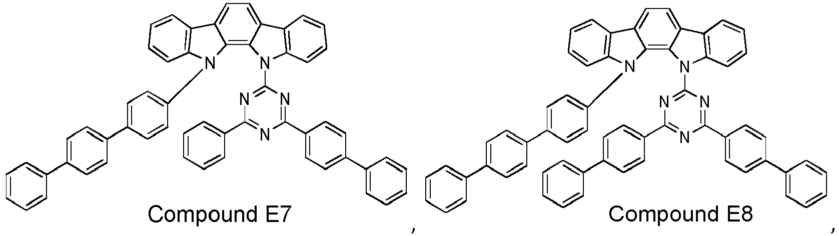

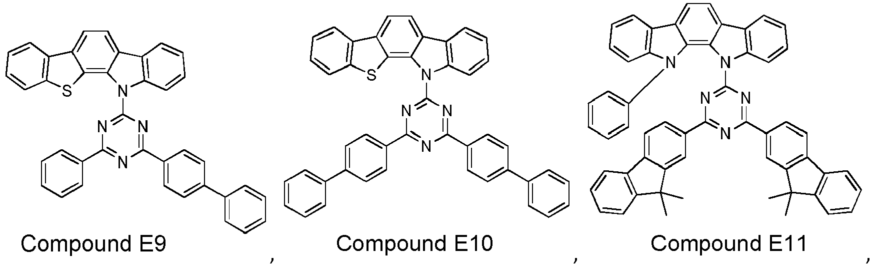

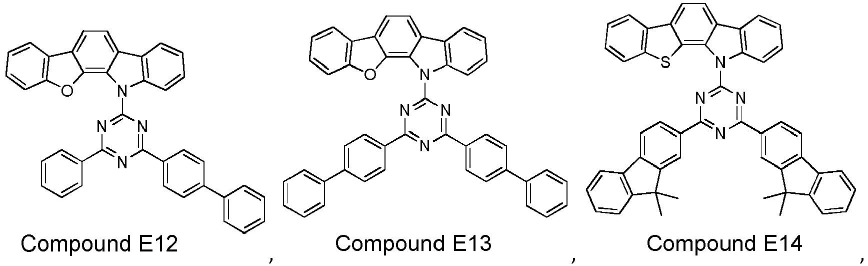

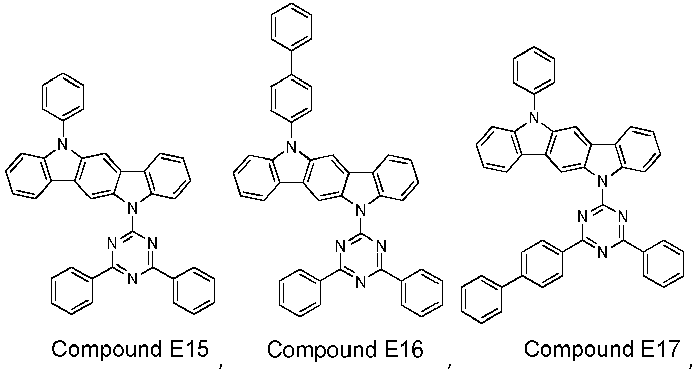

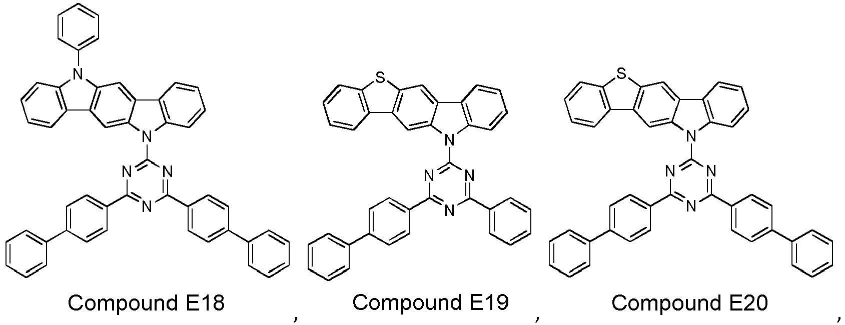

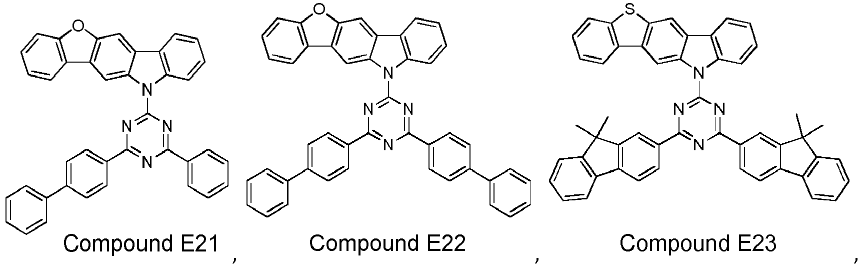

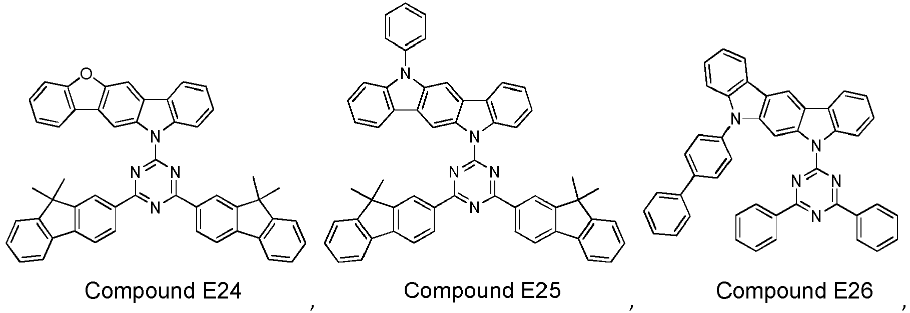

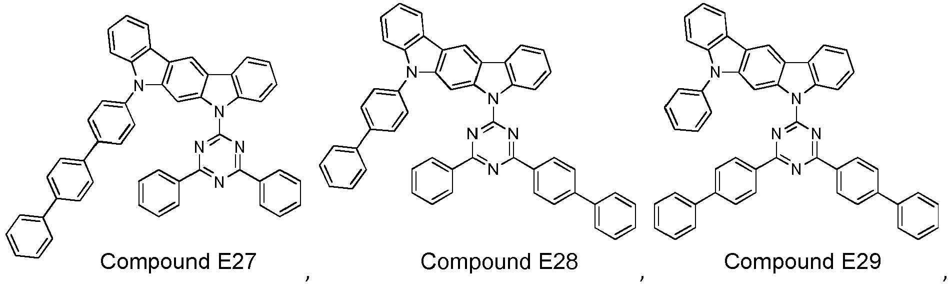

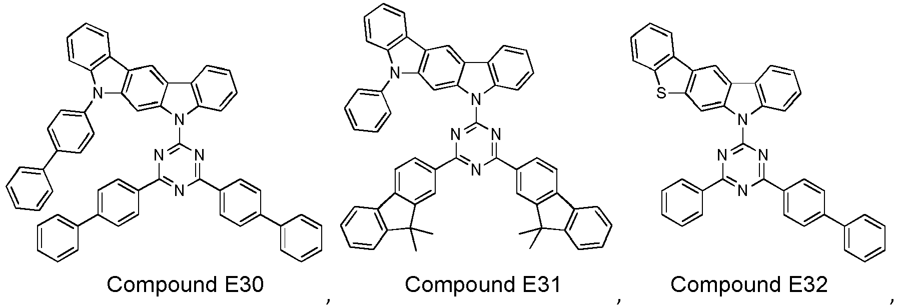

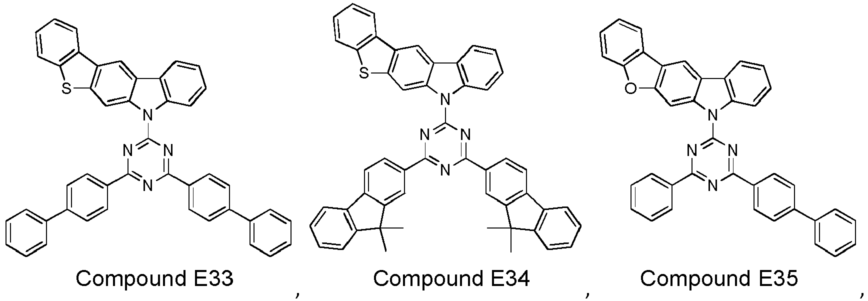

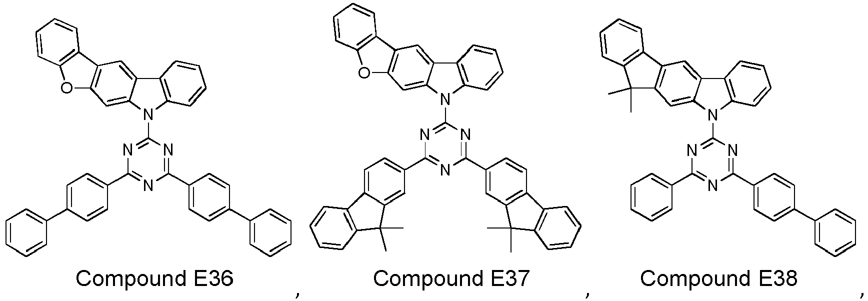

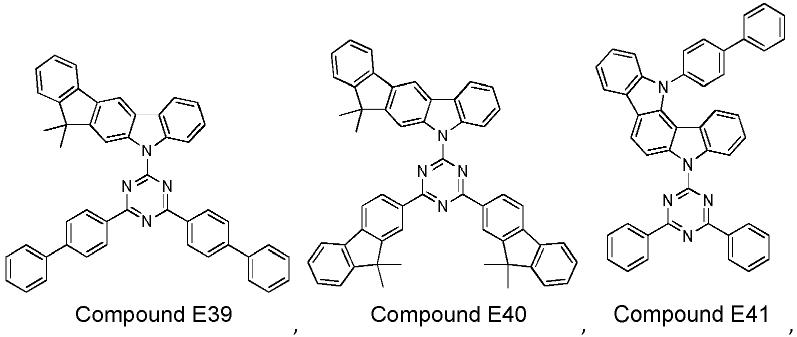

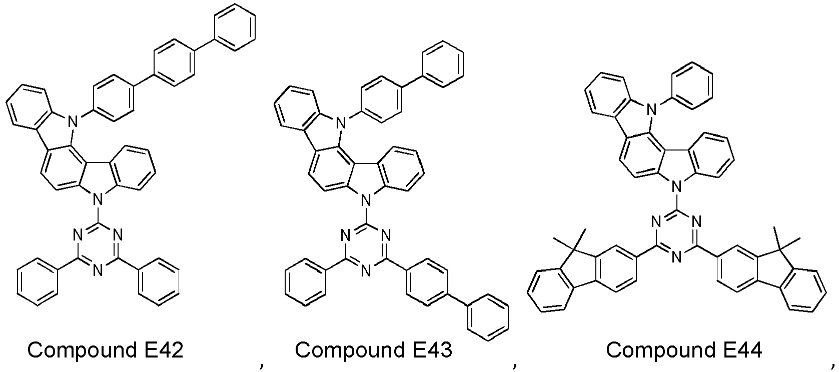

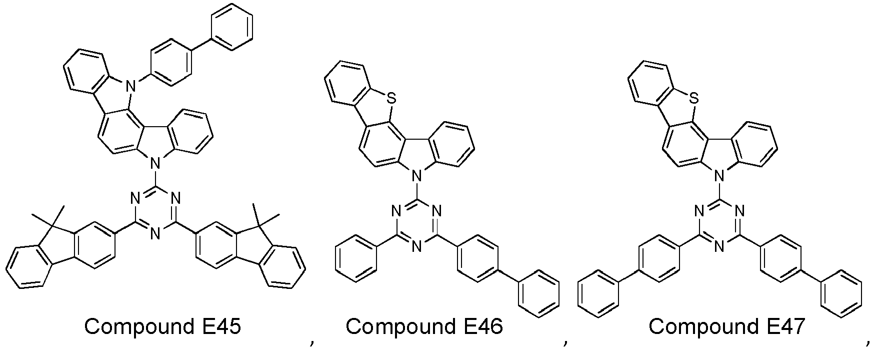

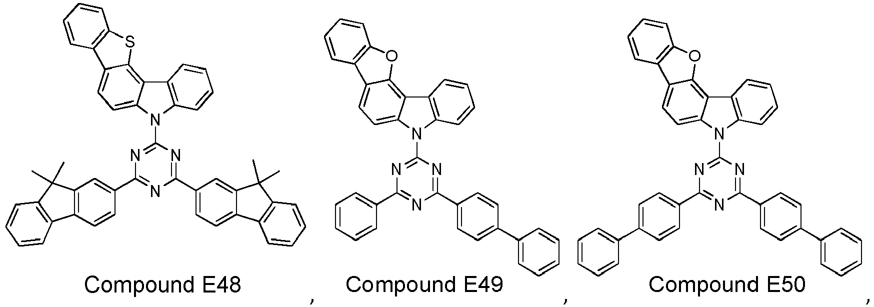

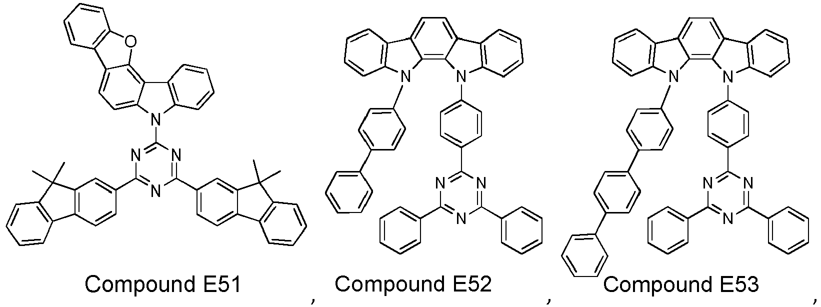

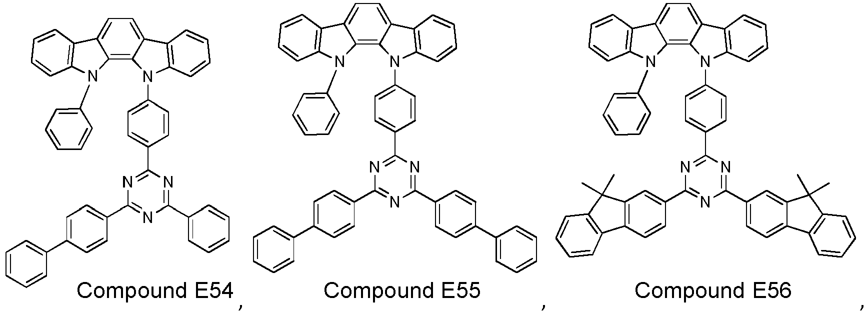

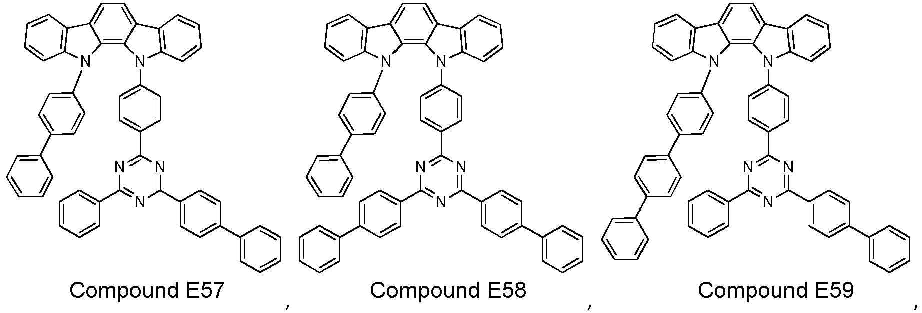

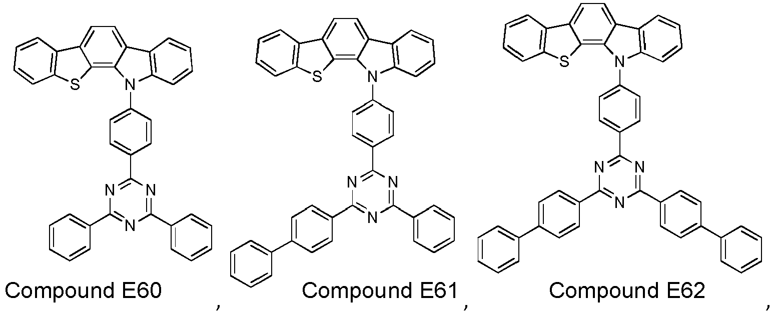

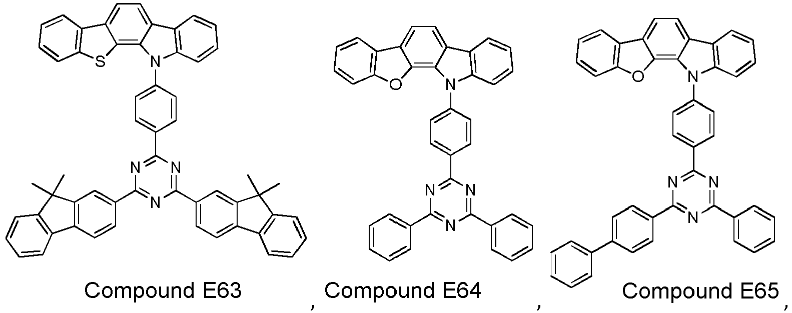

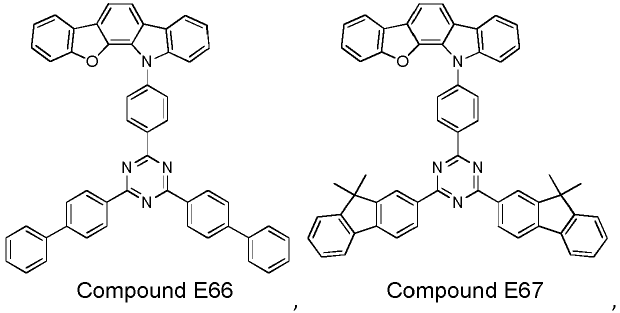

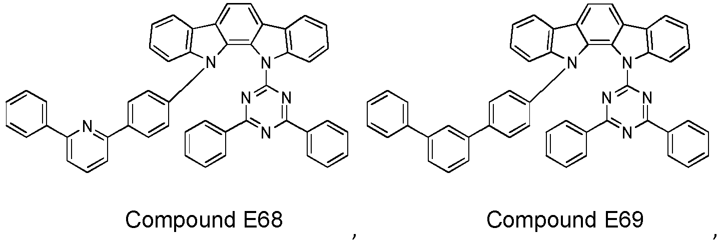

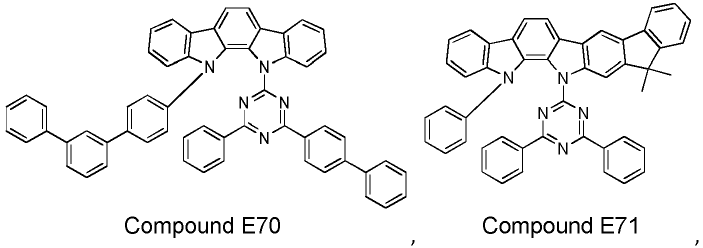

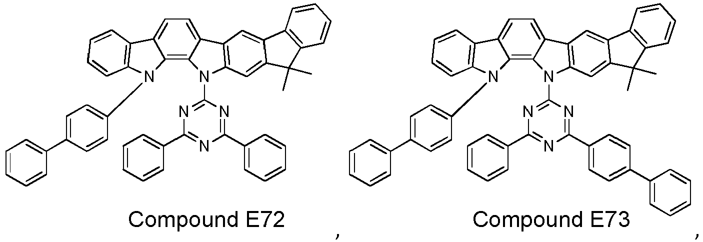

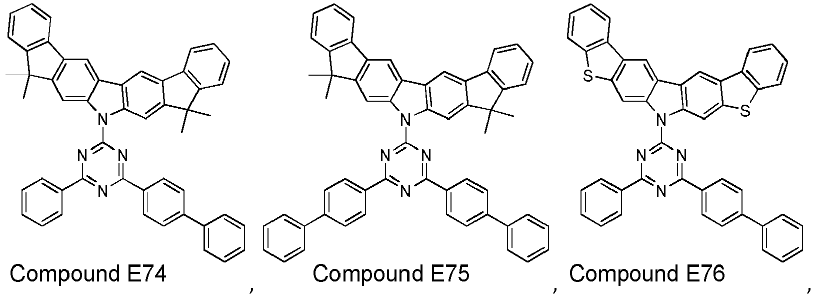

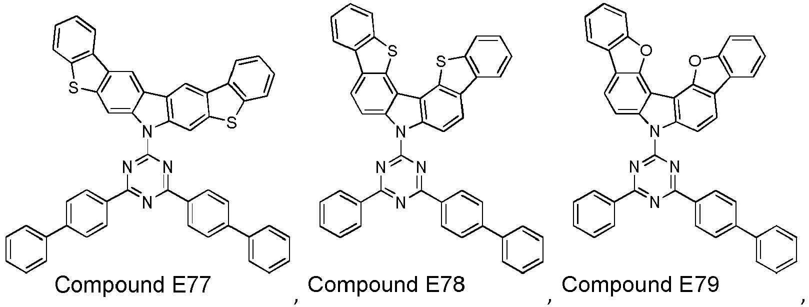

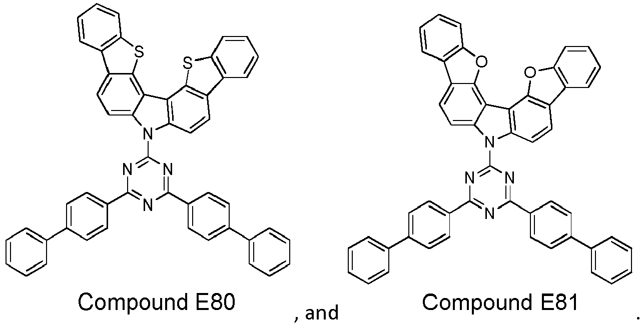

In one embodiment of the host composition, the mixture of the first compound and the second compound is selected from the group consisting of: (Compound 2 and Compound E2), (Compound 3 and Compound E4), (Compound 1 and Compound E5), (Compound 6 and Compound E7), (Compound 7 and Compound E7), (Compound 9 and Compound E8), (Compound 17 and Compound E8), and (Compound 15 and Compound E18).

According to another aspect of the present disclosure, a first device comprising a phosphorescent organic light-emitting device is disclosed. The phosphorescent organic light-emitting device comprises: an anode; a cathode; and an organic layer, disposed between the anode and the cathode, comprising an organic composition comprising the mixture of the first compound and the second compound according to the present disclosure.

In the first device, the first compound has a structure according to the formula (I): 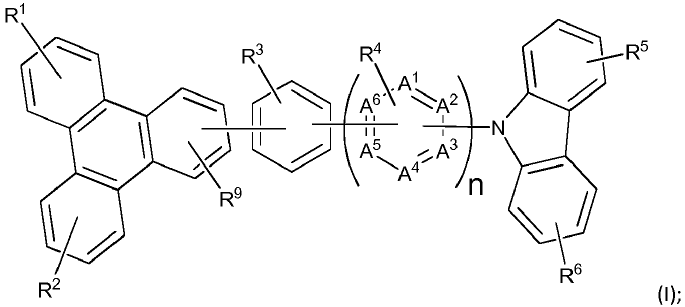
wherein R¹, R², R³, R⁴, R⁵, and R⁶ each represent mono, di, tri, tetra substitutions, or no substitution;
R⁹ represents mono, di, tri substitutions, or no substitution;
R¹, R², R³, R⁴, R⁵, R⁶, and R⁹ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof;
A¹, A², A³, A⁴, A⁵, and A⁶ are each independently selected from N or C; and
n is an integer from 1 to 20;
and the second compound has the formula (II): 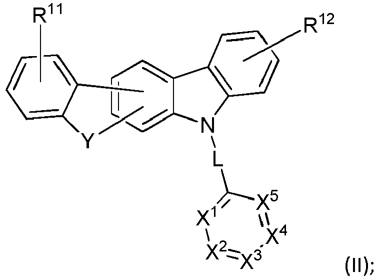
wherein R¹¹ and R¹² each represent mono, di, tri, tetra substitutions, or no substitution;
Y is selected from the group consisting of O, S. Se, NR', and CR*"*R*"'*;
L is a single bond or comprises an aryl or heteroaryl group having from 5-24 carbon atoms, which is optionally further substituted;
X¹, X², X³, X⁴, and X⁵ are each independently selected from group consisting of CR and N;
at least one of X¹, X², X³, X⁴, and X⁵ is N; and
R, R', R", and R*'"* are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof.

In one embodiment of the first device, in the formula (II) for the second compound, X¹, X³, and X⁵ are N; and wherein X² and X⁴ are CR.

In one embodiment of the first device, the organic layer is an emissive layer and the organic composition is a host. In one embodiment, the organic layer is a hole transporting layer.

In one embodiment of the first device, the organic layer can further comprise a phosphorescent emissive dopant. The phosphorescent emissive dopant can be a transition metal complex having at least one ligand or part of the ligand, if the ligand is more than bidentate, selected from the group consisting of: 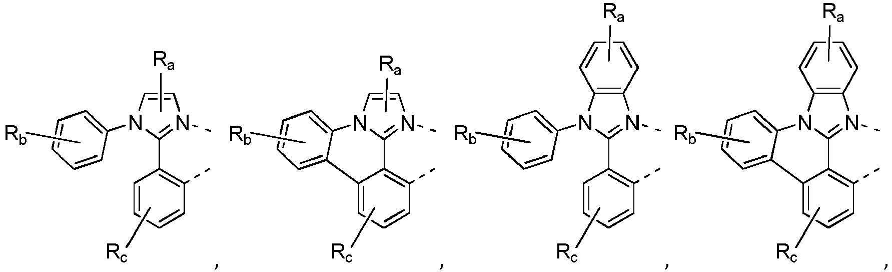 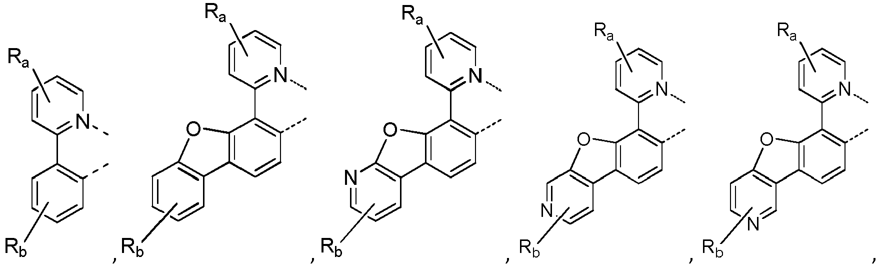 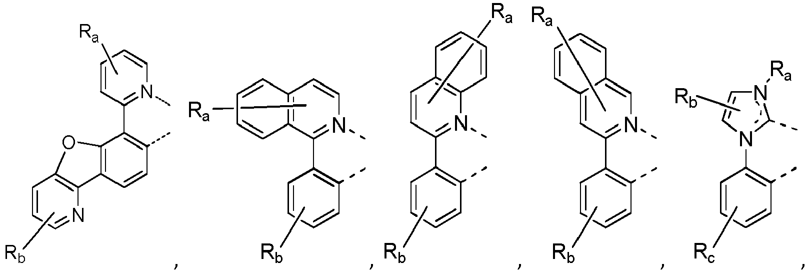 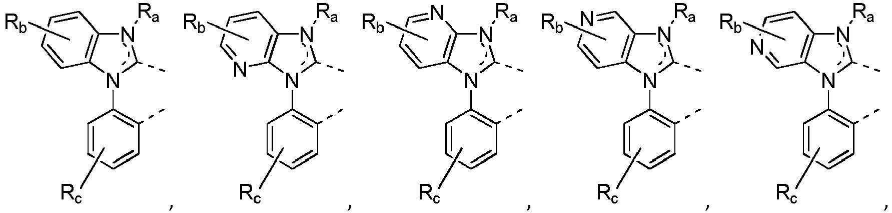 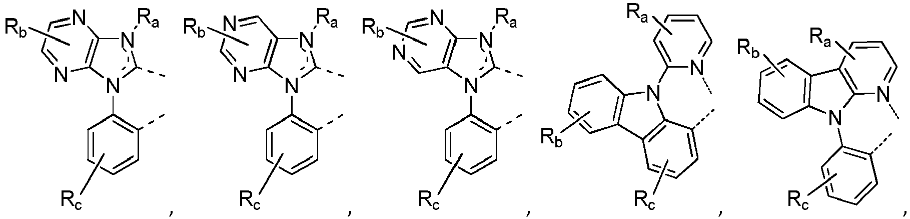 and 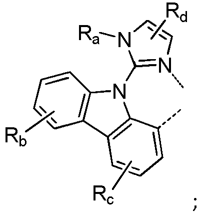 wherein Rₐ, R_{b}, R_{c}, and R_{d} may represent mono, di, tri, or tetra substitution, or no substitution; wherein Rₐ, R_{b}, R_{c}, and R_{d} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof; and wherein two adjacent substituents of Rₐ, R_{b}, R_{c}, and R_{d} are optionally joined to form a fused ring or form a multidentate ligand.

A method of fabricating an organic light emitting device comprising a first electrode, a second electrode, and an organic layer disposed between the first electrode and the second electrode, wherein the organic layer comprises the composition comprising a mixture of the first compound and the second compound according to the present disclosure, comprises:
providing a substrate having the first electrode disposed thereon;
depositing the organic composition over the first electrode; and
depositing the second electrode over the organic layer.

In one method, the organic composition is deposited in a vacuum thermal evaporation system having a pressure level in the range of 1x10⁻⁸ Torr to 1x10⁻¹² Torr. In one method, the organic composition leaves a residue corresponding to less than 5 wt% of the organic composition's original charge in the vacuum thermal evaporation system's sublimation crucible after the organic composition is depleted during the deposition of the organic composition over the first electrode.

In the method, the first compound has the formula (I): 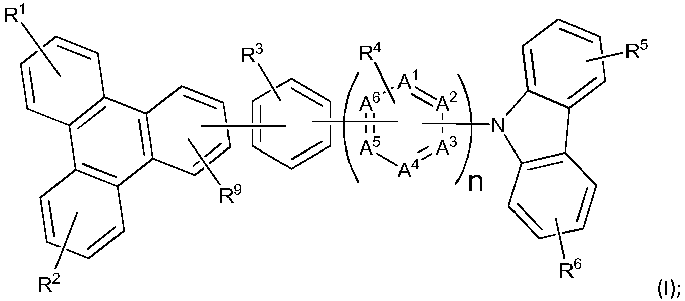
wherein R¹, R², R³, R⁴, R⁵, and R⁶ each represent mono, di, tri, tetra substitutions, or no substitution; R⁹ represents mono, di, tri substitutions, or no substitution;
R¹, R², R³, R⁴, R⁵, R⁶, and R⁹ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof;
A¹, A², A³, A⁴, A⁵, and A⁶ are each independently selected from N or C; and
n is an integer from 1 to 20;
and the second compound has the formula (II): 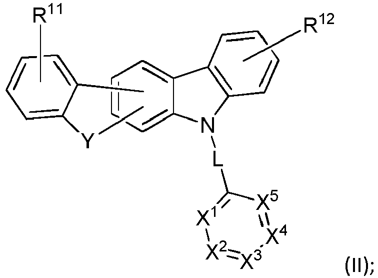
wherein R¹¹ and R¹² each represent mono, di, tri, tetra substitutions, or no substitution;
Y is selected from the group consisting of O, S. Se, NR', and CR*"*R*"'*;
L is a single bond or comprises an aryl or heteroaryl group having from 5-24 carbon atoms, which is optionally further substituted;
X¹, X², X³, X⁴, and X⁵ are each independently selected from group consisting of CR and N;
at least one of X¹, X², X³, X⁴, and X⁵ is N; and
R, R', R", and R*'"* are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof.

In one embodiment of the first device, the organic layer is a blocking layer and the organic composition is a blocking material in the organic layer. In another embodiment, the organic layer is an electron transporting layer and the organic composition is an electron transporting material in the organic layer.

In one embodiment of the first device, the first device is a consumer product. In another embodiment, the first device is an organic light-emitting device. In another embodiment, the first device can comprise a lighting panel.

### SYNTHESIS OF THE COMPOUNDS

Synthesis of Compound E2
(A) 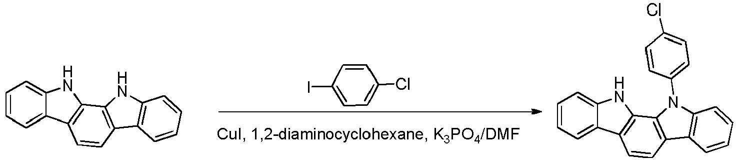 A solution of 11,12-dihydroindolo[2,3-*α*]carbazole (7.44 g, 29.0 mmol), 1-chloro-4-iodobenzene (20.77 g, 87 mmol), CuI (0.829 g, 4.35 mmol), cyclohexane-1,2-diamine (1.058 ml, 8.71 mmol), and K₃PO₄ (15.40 g, 72.6 mmol) in DMF (200 Ml) was heated under nitrogen at 145 °C overnight. After cooling to room temperature, the reaction mixture was diluted with EtOAc and water. The organic layer was isolated, washed with brine and water, dried over MgSO₄ and evaporated. The residue was purified by column chromatography on silica gel with hexane/EtOAc (95/5, v/v) as eluent to yield 11-(4-chlorophenyl)-11,12-dihydroindolo[2,3-*α*]carbazole (7.3 g, 68%) as a solid.
(B) 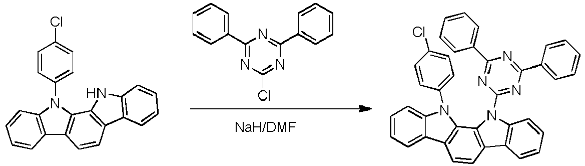 NaH (1.178 g, 29.4 mmol) in one portion was added to a solution of 11-(4-chlorophenyl)-11,12-dihydroindolo[2,3-*α*]carbazole (7.2 g, 19.63 mmol) in DMF (120 ml), at room temperature. The reaction mixture was stirred at room temperature for 2 hours. Then, 2-chloro-4,6-diphenyl-1,3,5-triazine (6.83 g, 25.5 mmol) was added to the reaction mixture. The reaction mixture was stirred overnight and the resulting solid was collected by filtration. The solid was dissolved in boiling toluene and the insoluble portion was removed by filtration. The filtrate was concentrated and the precipitation was collected by filtration to yield 11-(4-chlorophenyl)-12-(4,6-diphenyl-1,3,5-triazin-2-yl)-11,12-dihydroindolo[2,3-*α*]carbazole (8.6 g, 73.3 % yield) as a yellow powder.
(C) 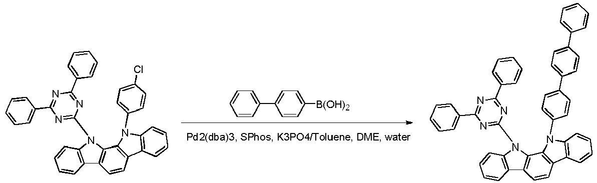 A solution of 11-(4-chlorophenyl)-12-(4,6-diphenyl-1,3,5-triazin-2-yl)-11,12-dihydroindolo[2,3-*α*]carbazole (4 g, 6.69 mmol), [1,1'-biphenyl]-4-ylboronic acid (3.97 g, 20.06 mmol), Pd₂(dba)₃ (0.61 g, 0.67 mmol), SPhos (0.55 g, 1.34 mmol), and K₃PO₄ (8.52 g, 40.1 mmol) in toluene (400 ml), DME (100 ml), and water (20 ml) was refluxed at 110 °C under nitrogen for 6 days. The hot reaction mixture was then filtered through a filter paper. The filtrate was evaporated; the residue was redissolved in boiling toluene, and filtered through a short plug of silica gel. Upon evaporation of the solvent, the solid was triturated with boiling EtOAc, toluene and xylene to yield Compound E2(2.3 g, 48%) as a yellow solid.

### COMBINATION WITH OTHER MATERIALS

The materials described herein as useful for a particular layer in an organic light emitting device may be used in combination with a wide variety of other materials present in the device. For example, emissive dopants disclosed herein may be used in conjunction with a wide variety of hosts, transport layers, blocking layers, injection layers, electrodes and other layers that may be present. The materials described or referred to below are non-limiting examples of materials that may be useful in combination with the compounds disclosed herein, and one of skill in the art can readily consult the literature to identify other materials that may be useful in combination.

### HIL/HTL:

A hole injecting/transporting material to be used in the present invention is not particularly limited, and any compound may be used as long as the compound is typically used as a hole injecting/transporting material. Examples of the material include, but not limit to: a phthalocyanine or porphyrin derivative; an aromatic amine derivative; an indolocarbazole derivative; a polymer containing fluorohydrocarbon; a polymer with conductivity dopants; a conducting polymer, such as PEDOT/PSS; a self-assembly monomer derived from compounds such as phosphonic acid and silane derivatives; a metal oxide derivative, such as MoOₓ; a p-type semiconducting organic compound, such as 1,4,5,8,9,12-Hexaazatriphenylenehexacarbonitrile; a metal complex, and a cross-linkable compound.

Examples of aromatic amine derivatives used in HIL or HTL include, but not limit to the following general structures:

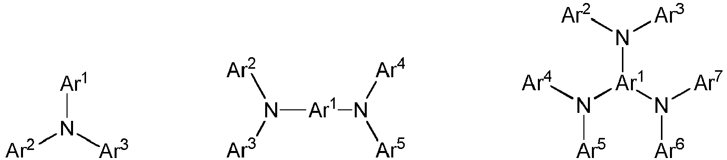

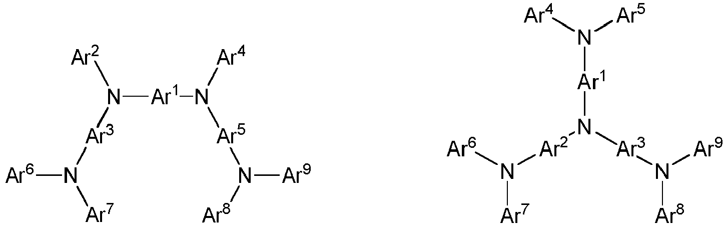

Each of Ar¹ to Ar⁹ is selected from the group consisting of aromatic hydrocarbon cyclic compounds such as benzene, biphenyl, triphenyl, triphenylene, naphthalene, anthracene, phenalene, phenanthrene, fluorene, pyrene, chrysene, perylene, azulene; group consisting aromatic heterocyclic compounds such as dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and groups consisting of 2 to 10 cyclic structural units which are groups of the same type or different types selected from the aromatic hydrocarbon cyclic group and the aromatic heterocyclic group and are bonded to each other directly or via at least one of oxygen atom, nitrogen atom, sulfur atom, silicon atom, phosphorus atom, boron atom, chain structural unit and the aliphatic cyclic group. Wherein each Ar is further substituted by a substituent selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof.

In one aspect, Ar¹ to Ar⁹ is independently selected from the group consisting of: 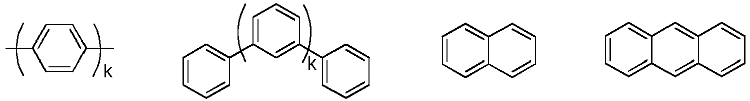 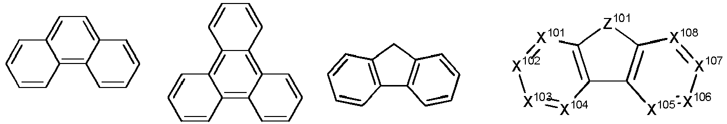 wherein k is an integer from 1 to 20; X¹⁰¹ to X¹⁰⁸ is C (including CH) or N; Z¹⁰¹ is NAr¹, O, or S; Ar¹ has the same definition as above.

Examples of metal complexes used in HIL or HTL include, but not limit to the following general formula: 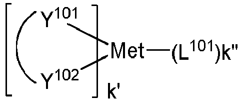 wherein Met is a metal, which can have an atomic weight greater than 40; (Y¹⁰¹-Y¹⁰²) is a bidentate ligand, Y¹⁰¹ and Y¹⁰² are independently selected from C, N, O, P, and S; L¹⁰¹ is an ancillary ligand; k' is an integer value from 1 to the maximum number of ligands that may be attached to the metal; and k'+k" is the maximum number of ligands that may be attached to the metal.

In one aspect, (Y¹⁰¹-Y¹⁰²) is a 2-phenylpyridine derivative. In another aspect, (Y¹⁰¹-Y¹⁰²) is a carbene ligand. In another aspect, Met is selected from Ir, Pt, Os, and Zn. In a further aspect, the metal complex has a smallest oxidation potential in solution vs. Fc⁺/Fc couple of less than about 0.6 V.

### Host:

The light emitting layer of the organic EL device of the present invention preferably contains at least a metal complex as light emitting material, and may contain a host material using the metal complex as a dopant material. Examples of the host material are not particularly limited, and any metal complexes or organic compounds may be used as long as the triplet energy of the host is larger than that of the dopant. While the Table below categorizes host materials as preferred for devices that emit various colors, any host material may be used with any dopant so long as the triplet criteria is satisfied.

Examples of metal complexes used as host are preferred to have the following general formula: 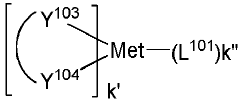 wherein Met is a metal; (Y¹⁰³-Y¹⁰⁴) is a bidentate ligand, Y¹⁰³ and Y¹⁰⁴ are independently selected from C, N, O, P, and S; L¹⁰¹ is an another ligand; k' is an integer value from 1 to the maximum number of ligands that may be attached to the metal; and k'+k" is the maximum number of ligands that may be attached to the metal.

In one aspect, the metal complexes are: 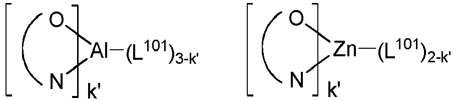 wherein (O-N) is a bidentate ligand, having metal coordinated to atoms O and N.

In another aspect, Met is selected from Ir and Pt. In a further aspect, (Y¹⁰³-Y¹⁰⁴) is a carbene ligand.

Examples of organic compounds used as host are selected from the group consisting aromatic hydrocarbon cyclic compounds such as benzene, biphenyl, triphenyl, triphenylene, naphthalene, anthracene, phenalene, phenanthrene, fluorene, pyrene, chrysene, perylene, azulene; group consisting aromatic heterocyclic compounds such as dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and group consisting 2 to 10 cyclic structural units which are groups of the same type or different types selected from the aromatic hydrocarbon cyclic group and the aromatic heterocyclic group and are bonded to each other directly or via at least one of oxygen atom, nitrogen atom, sulfur atom, silicon atom, phosphorus atom, boron atom, chain structural unit and the aliphatic cyclic group. Wherein each group is further substituted by a substituent selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof.

In one aspect, a host compound contains at least one of the following groups in the molecule: 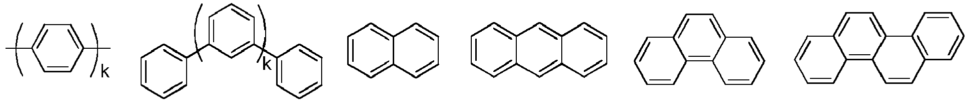 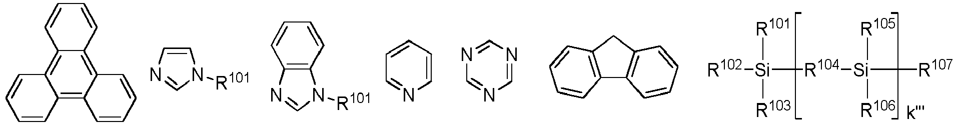 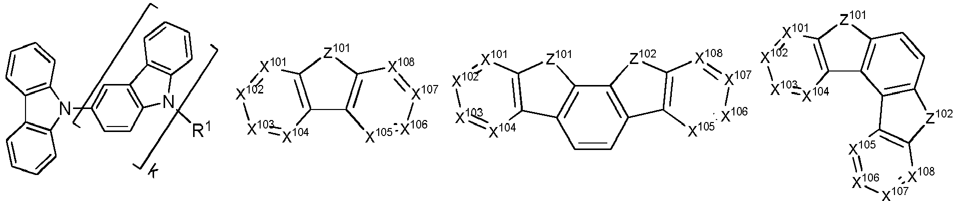 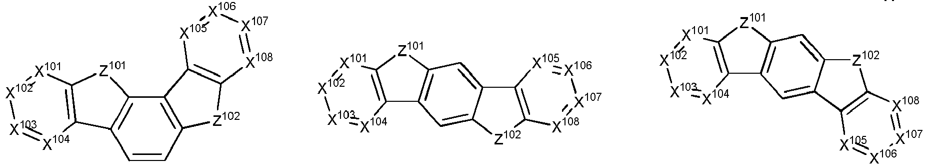 wherein R¹⁰¹ to R¹⁰⁷ is independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof, when it is aryl or heteroaryl, it has the similar definition as Ar's mentioned above. k is an integer from 0 to 20 or 1 to 20; k*"'* is an integer from 0 to 20. X¹⁰¹ to X¹⁰⁸ is selected from C (including CH) or N.
Z¹⁰¹ and Z¹⁰² is selected from NR¹⁰¹, O, or S.

### HBL:

A hole blocking layer (HBL) may be used to reduce the number of holes and/or excitons that leave the emissive layer. The presence of such a blocking layer in a device may result in substantially higher efficiencies as compared to a similar device lacking a blocking layer. Also, a blocking layer may be used to confine emission to a desired region of an OLED.

In one aspect, a compound used in HBL contains the same molecule or the same functional groups used as host described above.

In another aspect, a compound used in HBL contains at least one of the following groups in the molecule: 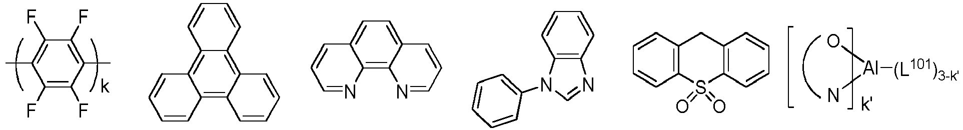 wherein k is an integer from 1 to 20; L¹⁰¹ is another ligand, k' is an integer from 1 to 3.

### ETL:

Electron transport layer (ETL) may include a material capable of transporting electrons. Electron transport layer may be intrinsic (undoped), or doped. Doping may be used to enhance conductivity. Examples of the ETL material are not particularly limited, and any metal complexes or organic compounds may be used as long as they are typically used to transport electrons.

In one aspect, a compound used in ETL contains at least one of the following groups in the molecule: 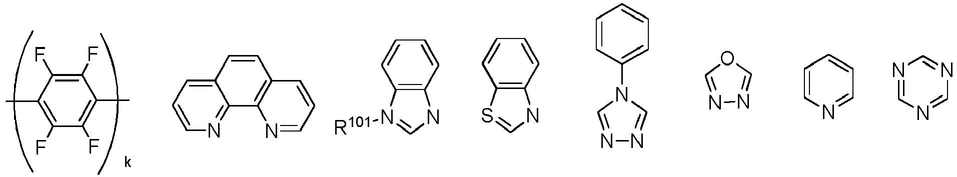 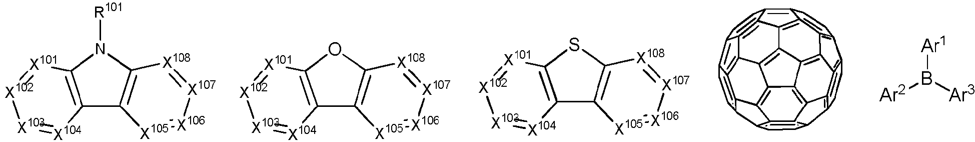 wherein R¹⁰¹ is selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof, when it is aryl or heteroaryl, it has the similar definition as Ar's mentioned above. Ar¹ to Ar³ has the similar definition as Ar's mentioned above. k is an integer from 1 to 20. X¹⁰¹ to X¹⁰⁸ is selected from C (including CH) or N.

In another aspect, the metal complexes used in ETL contains, but not limit to the following general formula: 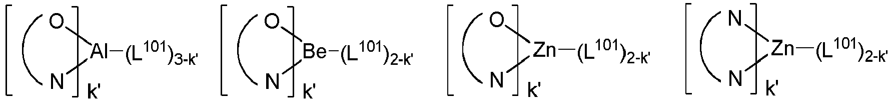 wherein (O-N) or (N-N) is a bidentate ligand, having metal coordinated to atoms O, N or N, N; L¹⁰¹ is another ligand; k' is an integer value from 1 to the maximum number of ligands that may be attached to the metal.

In any above-mentioned compounds used in each layer of the OLED device, the hydrogen atoms can be partially or fully deuterated. Thus, any specifically listed substituent, such as, without limitation, methyl, phenyl, pyridyl, etc. encompasses undeuterated, partially deuterated, and fully deuterated versions thereof. Similarly, classes of substituents such as, without limitation, alkyl, aryl, cycloalkyl, heteroaryl, etc. also encompass undeuterated, partially deuterated, and fully deuterated versions thereof.

In addition to and/or in combination with the materials disclosed herein, many hole injection materials, hole transporting materials, host materials, dopant materials, exciton/hole blocking layer materials, electron transporting and electron injecting materials may be used in an OLED. Non-limiting examples of the materials that may be used in an OLED in combination with materials disclosed herein are listed in Table 4 below. Table 4 lists non-limiting classes of materials, non-limiting examples of compounds for each class, and references that disclose the materials.

**TABLE 4**

| **MATERIAL** | **EXAMPLES OF MATERIAL** | **PUBLICATIONS** |
|---|---|---|
| **Hole injection materials** | | |
| Phthalocyanine and porphyrin compounds | 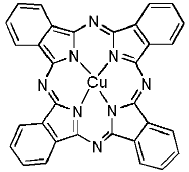 | Appl. Phys. Lett. 69, 2160(1996) |
| Starburst triarylamines | 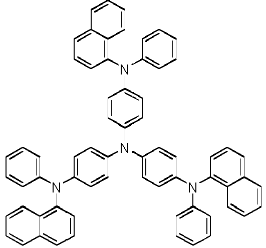 | J. Lumin. 72-74, 985 (1997) |
| CFₓ Fluorohydrocarbon polymer | 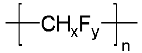 | Appl. Phys. Lett. 78, 673 (2001) |
| Conducting polymers | 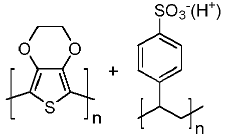 | Synth. Met. 87, 171 (1997) |
| (e.g., PEDOT:PSS, polyaniline, polythiophene) | | WO2007002683 |
| Phosphonic acid and silane SAMs | 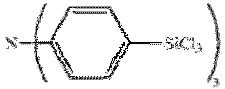 | US20030162053 |
| Triarylamine or polythiophene polymers with conductivity dopants | 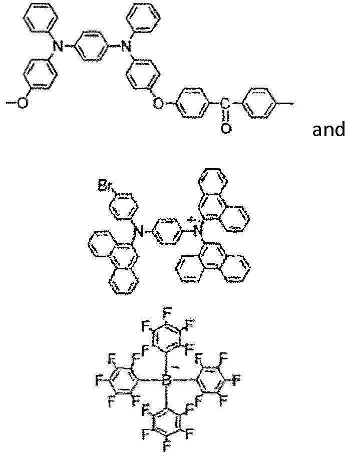 | EP1725079A1 |
| Organic compounds with conductive inorganic compounds, such as molybdenum and tungsten oxides | 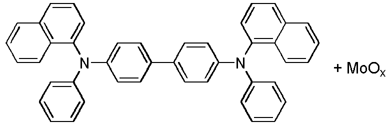 | US20050123751 |
| | | SID Symposium Digest, 37, 923 (2006) |
| | | WO2009018009 |
| n-type semiconducting organic complexes | 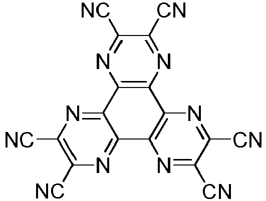 | US20020158242 |
| Metal organometallic complexes | 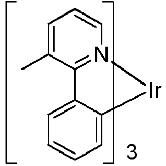 | US20060240279 |
| Cross-linkable compounds | 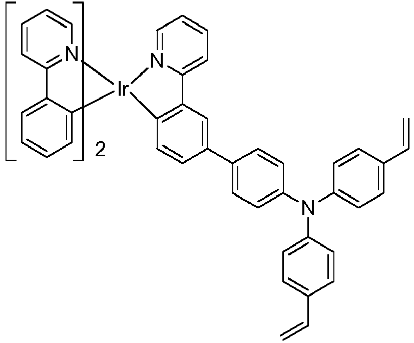 | US20080220265 |
| Polythiophene based polymers and copolymers | 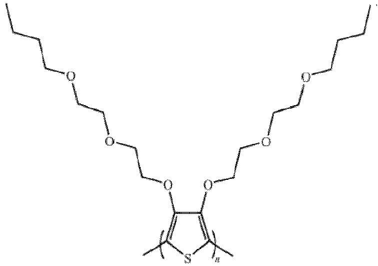 | WO 2011075644 |
| | | EP2350216 |
| **Hole transporting materials** | | |
| Triarylamines (e.g., TPD, α-NPD) | 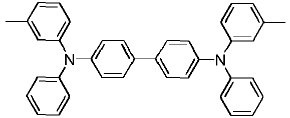 | Appl. Phys. Lett. 51, 913 (1987) |
| | 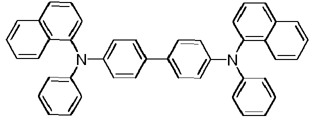 | US5061569 |
| | 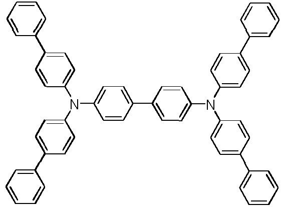 | EP650955 |
| | 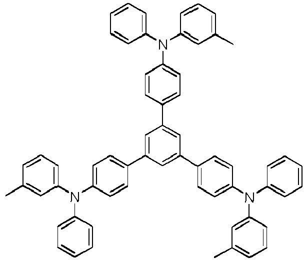 | J. Mater. Chem. 3, 319 (1993) |
| | 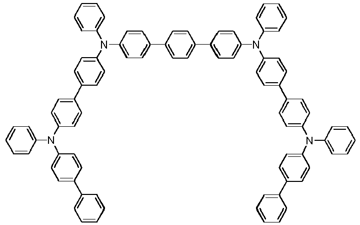 | Appl. Phys. Lett. 90, 183503 (2007) |
| | 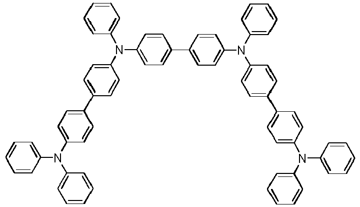 | Appl. Phys. Lett. 90, 183503 (2007) |
| Triarylamine on spirofluorene core | 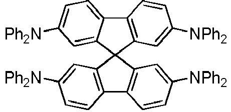 | Synth. Met. 91, 209 (1997) |
| Arylamine carbazole compounds | 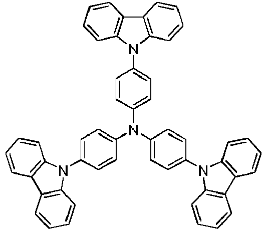 | Adv. Mater. 6, 677 (1994), US20080124572 |
| Triarylamine with (di)benzothiophene/(di)benz ofuran | 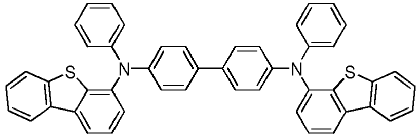 | US20070278938, US20080106190 |
| | | US20110163302 |
| Indolocarbazoles | 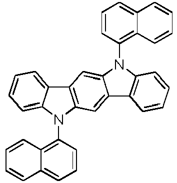 | Synth. Met. 111, 421 (2000) |
| Isoindole compounds | 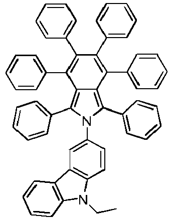 | Chem. Mater. 15, 3148 (2003) |
| Metal carbene complexes | 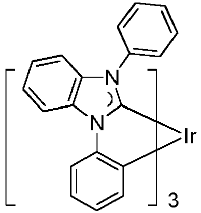 | US20080018221 |
| **Phosphorescent OLED host materials** | | |
| **Red hosts** | | |
| Arylcarbazoles | 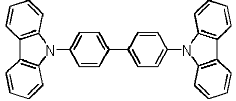 | Appl. Phys. Lett. 78, 1622 (2001) |
| Metal 8-hydroxyquinolates (e.g., Alq₃, BAlq) | 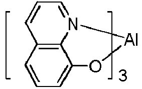 | Nature 395, 151 (1998) |
| | 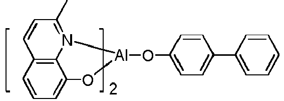 | US20060202194 |
| | 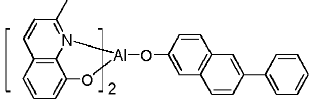 | WO2005014551 |
| | 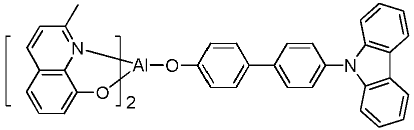 | WO2006072002 |
| Metal phenoxybenzothiazole compounds | 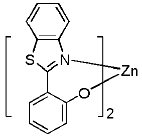 | Appl. Phys. Lett. 90, 123509 (2007) |
| Conjugated oligomers and polymers | 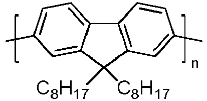 | Org. Electron. 1, 15 (2000) |
| (e.g., polyfluorene) | | |
| Aromatic fused rings | 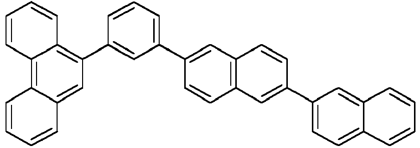 | WO2009066779, WO2009066778, WO2009063833, US20090045731, US20090045730, WO2009008311, US20090008605, US20090009065 |
| Zinc complexes | 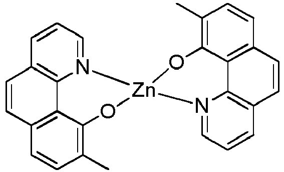 | WO2010056066 |
| Chrysene based compounds | 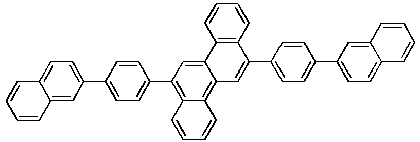 | WO2011086863 |
| Green hosts | | |
| Arylcarbazoles | 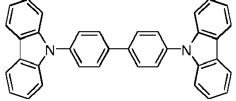 | Appl. Phys. Lett. 78, 1622 (2001) |
| | 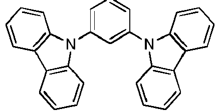 | US20030175553 |
| | 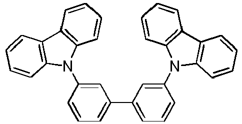 | WO2001039234 |
| Aryltriphenylene compounds | 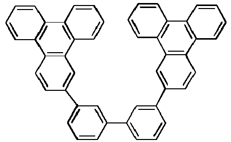 | US20060280965 |
| | 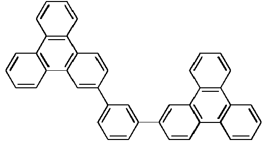 | US20060280965 |
| | 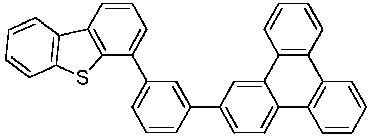 | WO2009021126 |
| Poly-fused heteroaryl compounds | 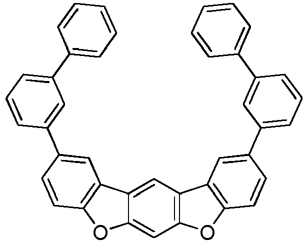 | US20090309488 |
| | | US20090302743 |
| | | US20100012931 |
| | | |
| Donor acceptor type molecules | 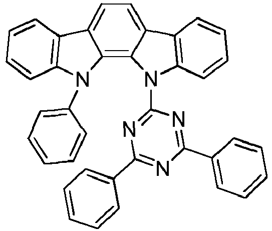 | WO2008056746 |
| | 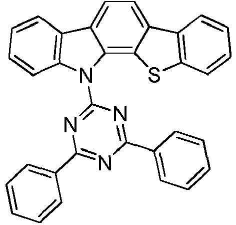 | WO2010107244 |
| Aza-carbazole/DBT/DBF | 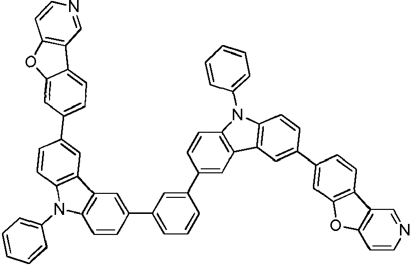 | JP2008074939 |
| | 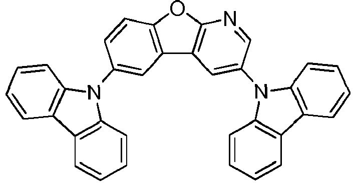 | US20100187984 |
| Polymers (e.g., PVK) | 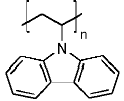 | Appl. Phys. Lett. 77, 2280(2000) |
| Spirofluorene compounds | 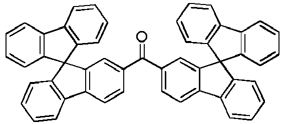 | WO2004093207 |
| Metal phenoxybenzooxazole compounds | 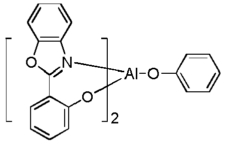 | WO2005089025 |
| | 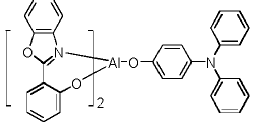 | WO2006132173 |
| | 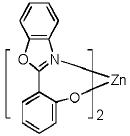 | JP200511610 |
| Spirofluorene-carbazole compounds | 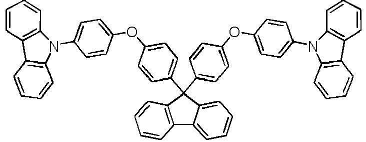 | JP2007254297 |
| | 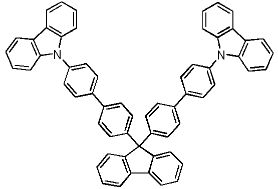 | JP2007254297 |
| Indolocarbazoles | 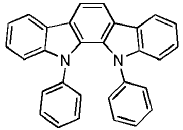 | WO2007063796 |
| | 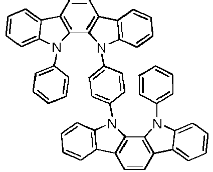 | WO2007063754 |
| 5-member ring electron deficient heterocycles (e.g., triazole, oxadiazole) | 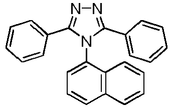 | J. Appl. Phys. 90, 5048 (2001) |
| | 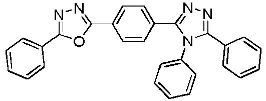 | WO2004107822 |
| Tetraphenylene complexes | 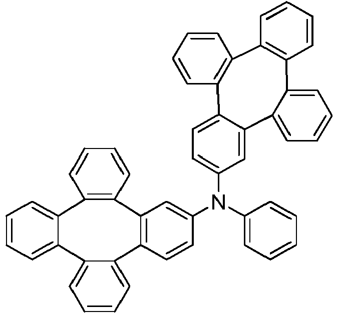 | US20050112407 |
| Metal phenoxypyridine compounds | 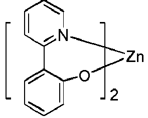 | WO2005030900 |
| Metal coordination complexes (e.g., Zn, Al with N^N ligands) | 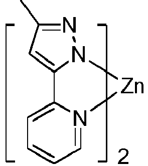 | US20040137268, US20040137267 |
| Blue hosts | | |
| Arylcarbazoles | 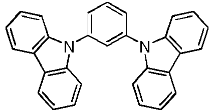 | Appl. Phys. Lett, 82, 2422(2003) |
| | 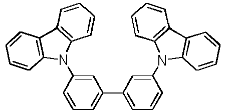 | US20070190359 |
| Dibenzothiophene/Dibenzof uran-carbazole compounds | 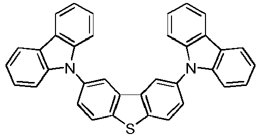 | WO2006114966, US20090167162 |
| | 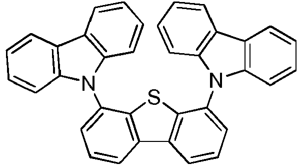 | US20090167162 |
| | 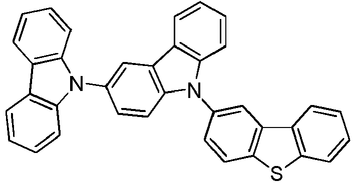 | WO2009086028 |
| | 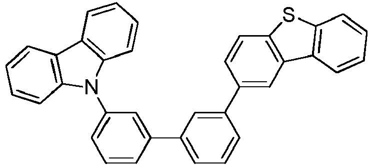 | US20090030202, US20090017330 |
| | 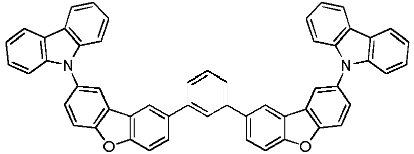 | US20100084966 |
| Silicon aryl compounds | 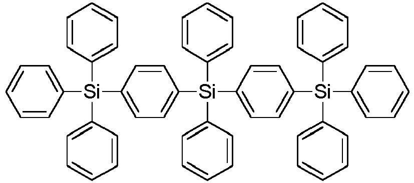 | US20050238919 |
| | 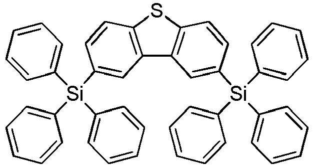 | WO2009003898 |
| Silicon/Germanium aryl compounds | 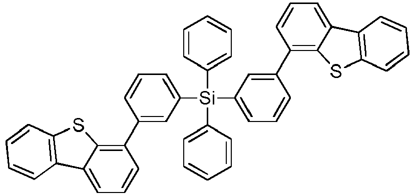 | EP2034538A |
| Aryl benzoyl ester | 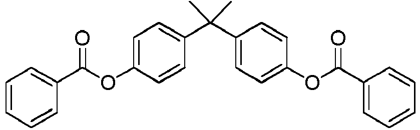 | WO2006100298 |
| Carbazole linked by nonconjugated groups | 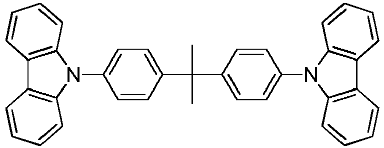 | US20040115476 |
| Aza-carbazoles | 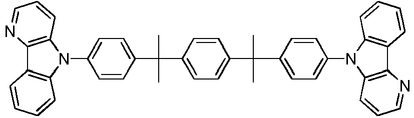 | US20060121308 |
| High triplet metal organometallic complex | 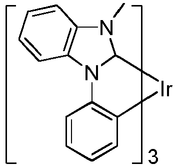 | US7154114 |
| **Phosphorescent dopants** | | |
| **Red dopants** | | |
| Heavy metal porphyrins (e.g., PtOEP) | 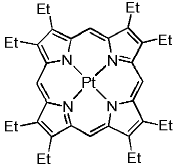 | Nature 395, 151 (1998) |
| Iridium(III) organometallic complexes | 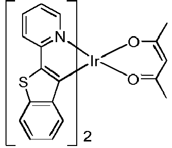 | Appl. Phys. Lett. 78, 1622 (2001) |
| | 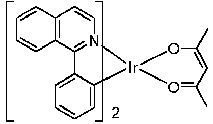 | US20030072964 |
| | 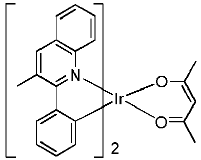 | US20030072964 |
| | 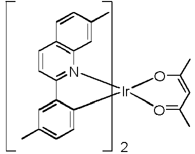 | US20060202194 |
| | 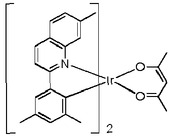 | US20060202194 |
| | 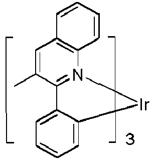 | US20070087321 |
| | 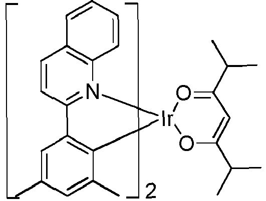 | US20080261076 |
| | | US20100090591 |
| | 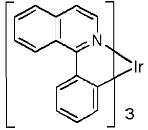 | US20070087321 |
| | 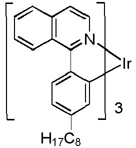 | Adv. Mater. 19, 739 (2007) |
| | 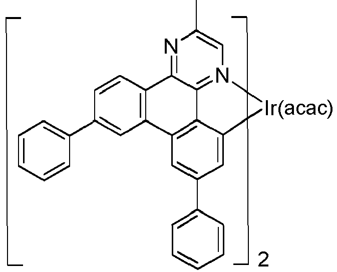 | WO2009100991 |
| | 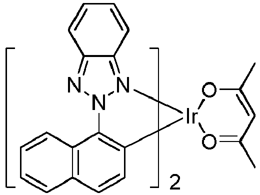 | WO2008101842 |
| | 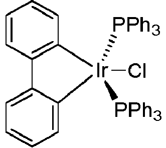 | US7232618 |
| Platinum(II) organometallic complexes | 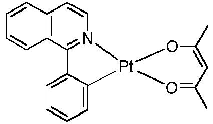 | WO2003040257 |
| | 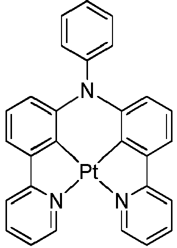 | US20070103060 |
| Osmium(III) complexes | 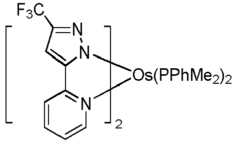 | Chem. Mater. 17, 3532 (2005) |
| Ruthenium(II) complexes | 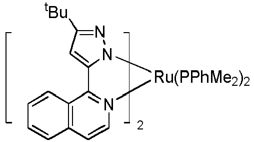 | Adv. Mater. 17, 1059 (2005) |
| Rhenium (I), (II), and (III) complexes | 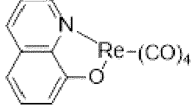 | US20050244673 |
| Green dopants | | |
| Iridium(III) organometallic complexes | 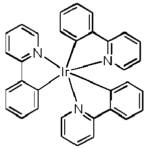 | Inorg. Chem. 40, 1704 (2001) |
| | and its derivatives | |
| | 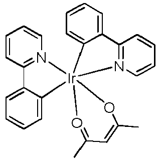 | US20020034656 |
| | 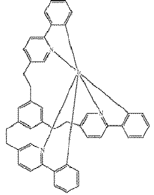 | US7332232 |
| | 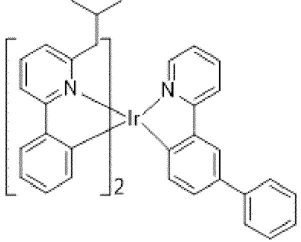 | US20090108737 |
| | 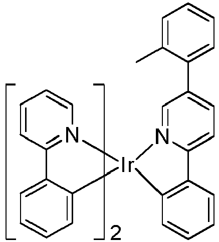 | WO2010028151 |
| | 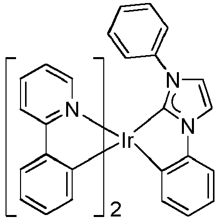 | EP1841834B |
| | 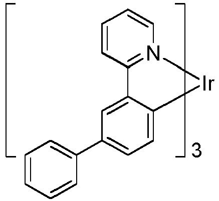 | US20060127696 |
| | 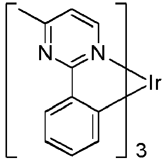 | US20090039776 |
| | 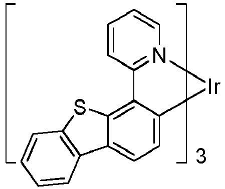 | US6921915 |
| | 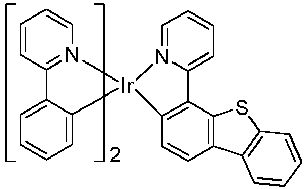 | US20100244004 |
| | 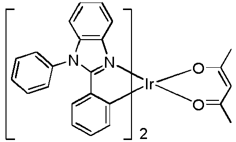 | US6687266 |
| | 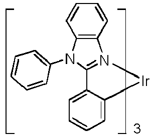 | Chem. Mater. 16,2480 (2004) |
| | 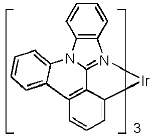 | US20070190359 |
| | 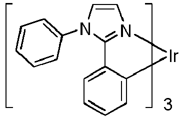 | US 20060008670 |
| | | JP2007123392 |
| | 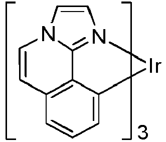 | WO2010086089, WO2011044988 |
| | 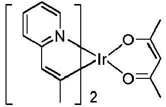 | Adv. Mater. 16, 2003 (2004) |
| | 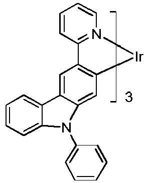 | Angew. Chem. Int. Ed. 2006,45,7800 |
| | 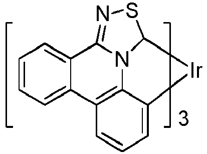 | WO2009050290 |
| | 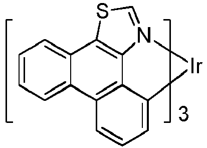 | US20090165846 |
| | 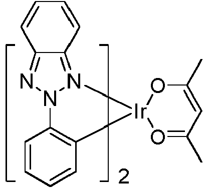 | US20080015355 |
| | 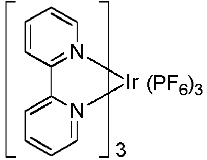 | US20010015432 |
| | 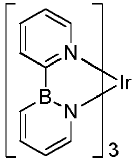 | US20100295032 |
| Monomer for polymeric metal organometallic compounds | 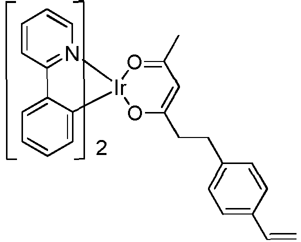 | US7250226, US7396598 |
| Pt(II) organometallic complexes, including polydentated ligands | 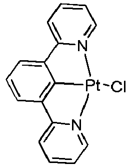 | Appl. Phys. Lett. 86, 153505(2005) |
| | 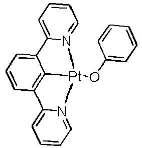 | Appl. Phys. Lett. 86, 153505(2005) |
| | 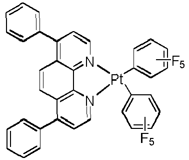 | Chem. Lett. 34, 592 (2005) |
| | 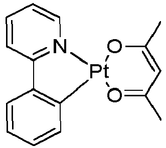 | WO2002015645 |
| | 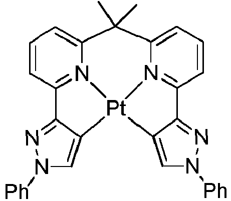 | US20060263635 |
| | 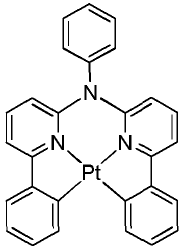 | US20060182992 |
| | | US20070103060 |
| Cu complexes | 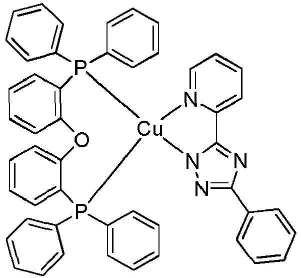 | WO2009000673 |
| | 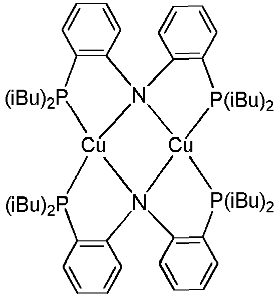 | US20070111026 |
| Gold complexes | 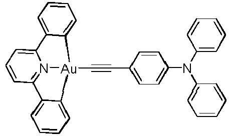 | Chem. Commun. 2906 (2005) |
| Rhenium(III) complexes | 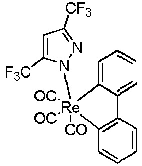 | Inorg. Chem. 42, 1248 (2003) |
| Osmium(II) complexes | 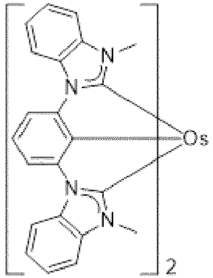 | US7279704 |
| Deuterated organometallic complexes | 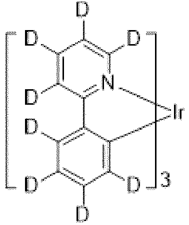 | US20030138657 |
| Organometallic complexes with two or more metal centers | 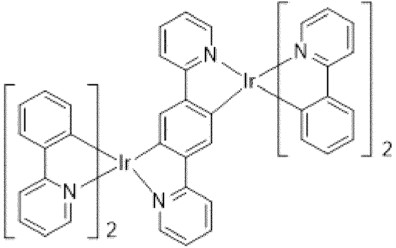 | US20030152802 |
| | 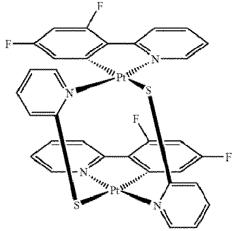 | US7090928 |
| **Blue dopants** | | |
| Iridium(III) organometallic complexes | 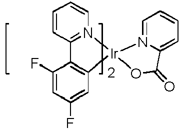 | WO2002002714 |
| | 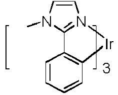 | WO2006009024 |
| | 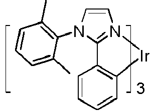 | US20060251923 |
| | | US20110057559 |
| | | US20110204333 |
| | 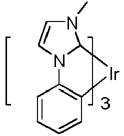 | US7393599, WO2006056418, US20050260441, WO2005019373 |
| | 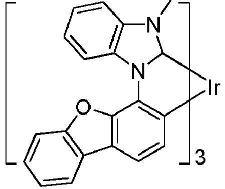 | US7534505 |
| | 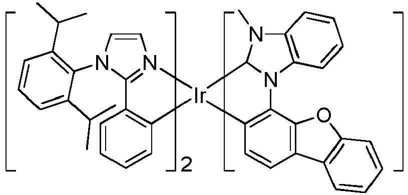 | WO2011051404 |
| | 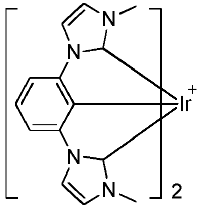 | US7445855 |
| | 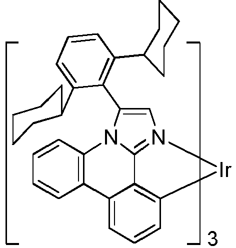 | US20070190359, US20080297033 |
| | | US20100148663 |
| | 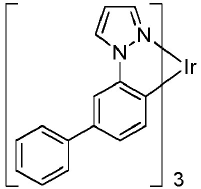 | US7338722 |
| | 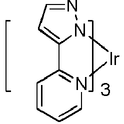 | US20020134984 |
| | 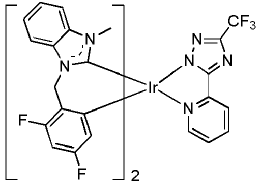 | Angew. Chem. Int. Ed. 47, 4542 (2008) |
| | 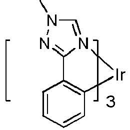 | Chem. Mater. 18, 5119 (2006) |
| | 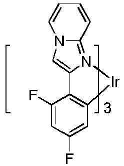 | Inorg. Chem. 46, 4308 (2007) |
| | 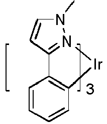 | WO2005123873 |
| | 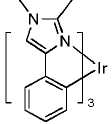 | WO2005123873 |
| | 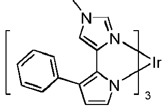 | WO2007004380 |
| | 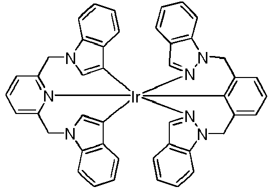 | WO2006082742 |
| Osmium(II) complexes | 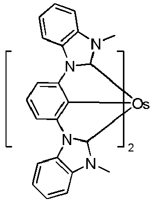 | US7279704 |
| | 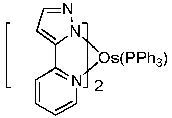 | Organometallics 23, 3745(2004) |
| Gold complexes | 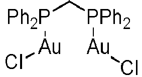 | Appl. Phys. Lett.74,1361 (1999) |
| Platinum(II) complexes | 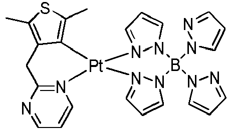 | WO2006098120, WO2006103874 |
| Pt tetradentate complexes with at least one metal-carbene bond | 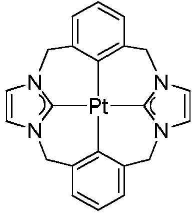 | US7655323 |
| **Exciton/hole blocking layer materials** | | |
| Bathocuprine compounds (e.g., BCP, BPhen) | 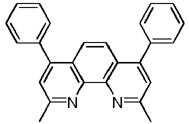 | Appl. Phys. Lett. 75, 4 (1999) |
| | 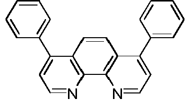 | Appl. Phys. Lett. 79, 449 (2001) |
| Metal 8-hydroxyquinolates (e.g., BAlq) | 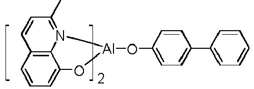 | Appl. Phys. Lett. 81, 162 (2002) |
| 5-member ring electron deficient heterocycles such as triazole, oxadiazole, imidazole, benzimidazole | 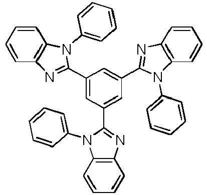 | Appl. Phys. Lett. 81, 162 (2002) |
| Triphenylene compounds | 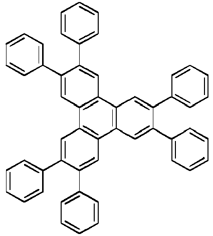 | US20050025993 |
| Fluorinated aromatic compounds | 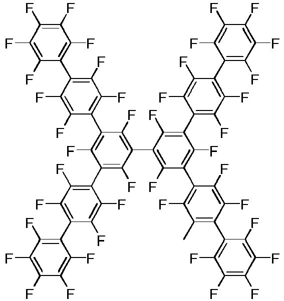 | Appl. Phys. Lett. 79, 156 (2001) |
| Phenothiazine-S-oxide | 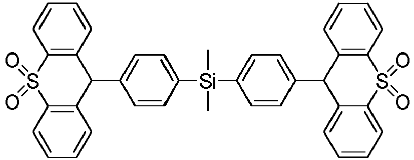 | WO2008132085 |
| Silylated five-membered nitrogen, oxygen, sulfur or phosphorus dibenzoheterocycles | 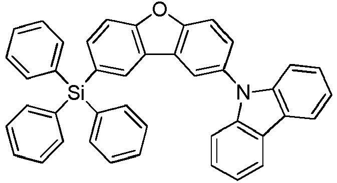 | WO2010079051 |
| Aza-carbazoles | 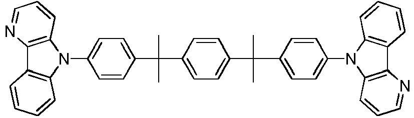 | US20060121308 |
| **Electron transporting materials** | | |
| Anthracene-benzimidazole compounds | 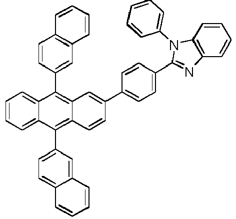 | WO2003060956 |
| | 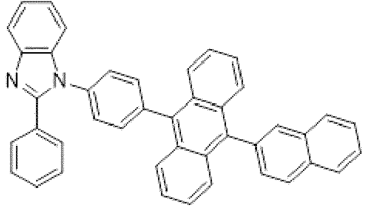 | US20090179554 |
| Aza triphenylene derivatives | 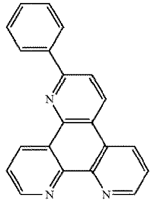 | US20090115316 |
| Anthracene-benzothiazole compounds | 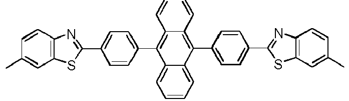 | Appl. Phys. Lett. 89, 063504 (2006) |
| Metal 8-hydroxyquinolates (e.g., Alq₃, Zrq₄) | 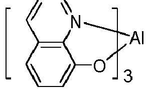 | Appl. Phys. Lett. 51, 913 (1987) US7230107 |
| Metal hydroxybenzoquinolates | 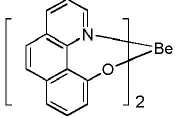 | Chem. Lett. 5, 905 (1993) |
| Bathocuprine compounds such as BCP, BPhen, etc | 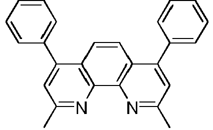 | Appl. Phys. Lett. 91, 263503 (2007) |
| | 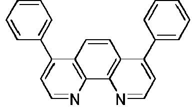 | Appl. Phys. Lett. 79, 449 (2001) |
| 5-member ring electron deficient heterocycles (e.g., triazole, oxadiazole, imidazole, benzimidazole) | 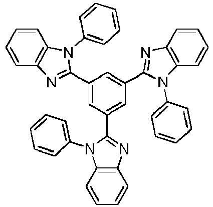 | Appl. Phys. Lett. 74, 865 (1999) |
| | 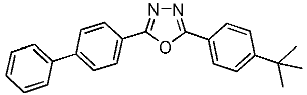 | Appl. Phys. Lett. 55, 1489(1989) |
| | 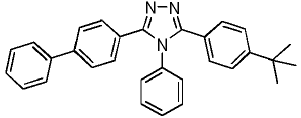 | Jpn. J. Apply. Phys. 32, L917 (1993) |
| Silole compounds | 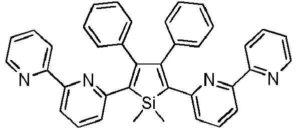 | Org. Electron. 4, 113 (2003) |
| Arylborane compounds | 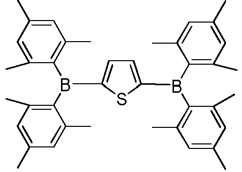 | J. Am. Chem. Soc. 120, 9714(1998) |
| Fluorinated aromatic compounds | 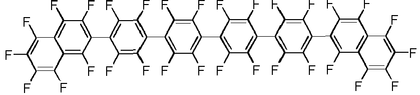 | J. Am. Chem. Soc. 122, 1832(2000) |
| Fullerene (e.g., C60) | 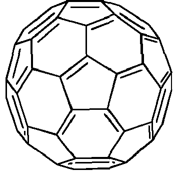 | US20090101870 |
| Triazine complexes | 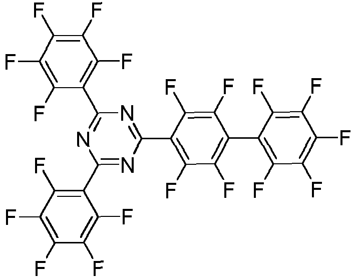 | US20040036077 |
| Zn (N^N) complexes | 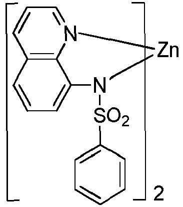 | US6528187 |

## Claims

1. A composition comprising:
a mixture of a first compound and a second compound, wherein the first compound has a different chemical structure than the second compound;
wherein the first compound is capable of functioning as a hole transporting material in an organic light emitting device at room temperature;
wherein the first compound comprises at least one carbazole group;
wherein the first compound has an evaporation temperature T1 of 150 to 350 °C;
wherein the second compound has an evaporation temperature T2 of 150 to 350 °C;
wherein the evaporation temperatures are measured in a vacuum deposition tool at a constant pressure, between 1x10⁻⁶ Torr to 1x10⁻⁹ Torr, at a 2Å/sec deposition rate;
wherein the absolute value of T1-T2 is less than 20 °C;
wherein the first compound has a concentration C1 in said mixture, and the first compound has a concentration C2 in a film formed by evaporating the mixture in a vacuum deposition tool at a constant pressure between 1x10⁻⁶ Torr to 1x10⁻⁹ Torr, at a 2Å/sec deposition rate on a surface positioned at a predefined distance away from the mixture being evaporated;
wherein the absolute value of (C1-C2)/C1 is less than 5%, and
wherein the first compound has a structure according to a formula (I): 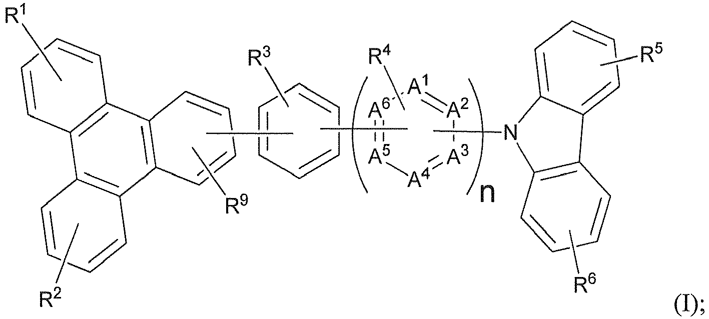 wherein
R¹, R², R³, R⁴, R⁵, and R⁶ each represent mono, di, tri, tetra substitutions, or no substitution;
R⁹ represents mono, di, tri substitutions, or no substitution;
R¹, R², R³, R⁴, R⁵, R⁶, and R⁹ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof;
A¹, A², A³, A⁴, A⁵, and A⁶ are each independently selected from N or C; and
n is an integer from 1 to 20;
wherein the second compound has a structure according to a formula (II): 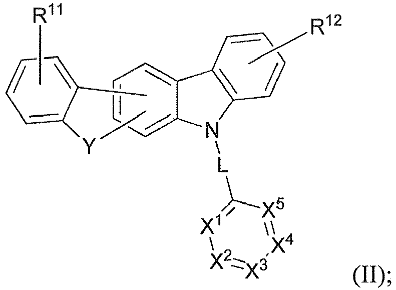 wherein
R¹¹ and R¹² each represent mono, di, tri, tetra substitutions, or no substitution;
Y is selected from the group consisting of O, S, Se, NR', and CR"R"';
L is a single bond or comprises an aryl or heteroaryl group having from 5-24 carbon atoms, which is optionally further substituted;
X¹, X², X³, X⁴, and X⁵ are each independently selected from the group consisting of CR and N;
at least one of X¹, X², X³, X⁴, and X⁵ is N; and
R, R', R", and R''' are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acid, ester, nitrile, isonitrile, sulfanyl, sulfinyl, sulfonyl, phosphino, and combinations thereof.

2. The composition of claim 1, wherein the first compound has an evaporation temperature T1 of 200 to 350 °C and the second compound has an evaporation temperature of T2 of 200 to 350 °C.

3. The composition of claim 1 or 2, wherein the absolute value of (C1-C2)/C1 is less than 3%.

4. The composition of any one of claims 1 to 3, wherein the first compound has a vapor pressure of P1 at T1, the second compound has a vapor pressure of P2 at T2; and
wherein the ratio of P1/P2 is within the range of 0.90 to 1.10.

5. The composition of any one of claims 1 to 4, wherein the first compound has a first mass loss rate and the second compound has a second mass loss rate, wherein the ratio between the first mass loss rate and the second mass loss rate is within the range of 0.90 to 1.10.

6. The composition of any one of claims 1 to 5, wherein the first compound further comprises at least one of the chemical groups selected from the group consisting of triphenylene, dibenzothiophene, dibenzofuran, and dibenzoselenophene.

7. The composition of any one of claims 1 to 6, wherein the second compound comprises at least one of the chemical groups selected from the group consisting of aza-triphenylene, aza-carbazole, aza-dibenzothiophene, aza-dibenzofuran, aza-dibenzoselenophene, and non-fused N-containing six-member aromatic rings.

8. The composition of any one of claims 1 to 7, wherein the first compound has a HOMO energy level higher than -5.8 eV.

9. The composition of any of claims 1 to 8, wherein the composition further comprises a third compound, wherein the third compound has a different chemical structure than the first and second compounds, wherein the third compound has an evaporation temperature T3 of 150 to 350 °C, and wherein the absolute value of T1-T3 is less than 20 °C.

10. The composition of any one of claims 1 to 9, wherein the first compound is selected from the group consisting of: 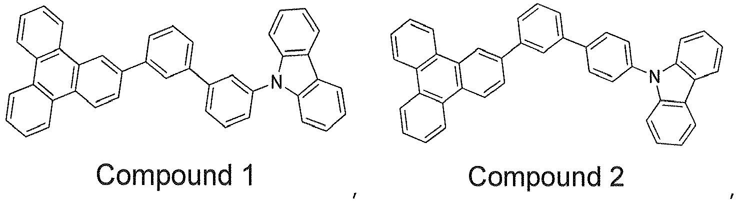 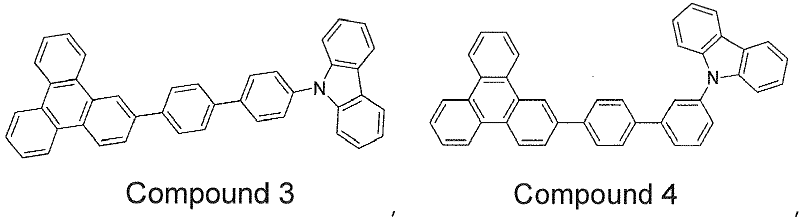 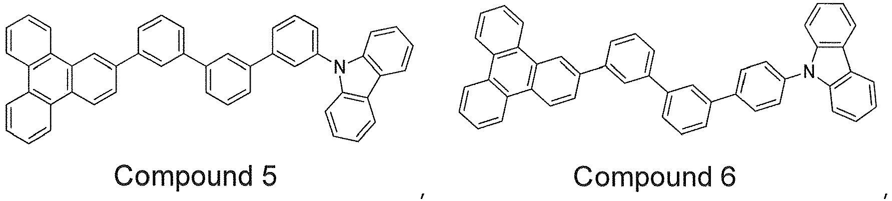 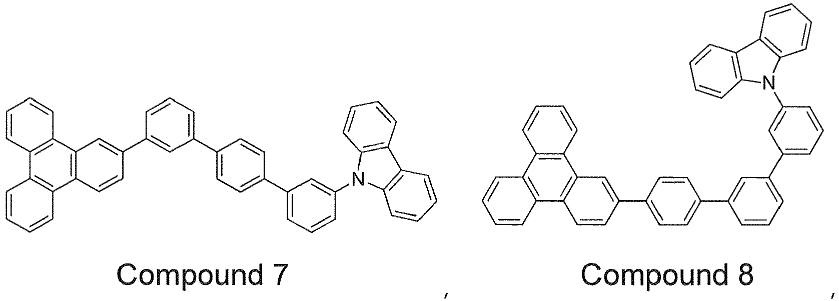 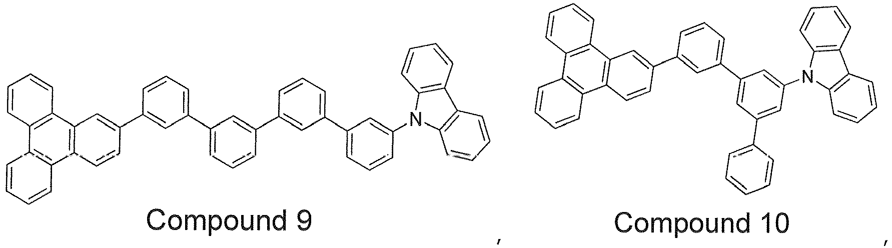 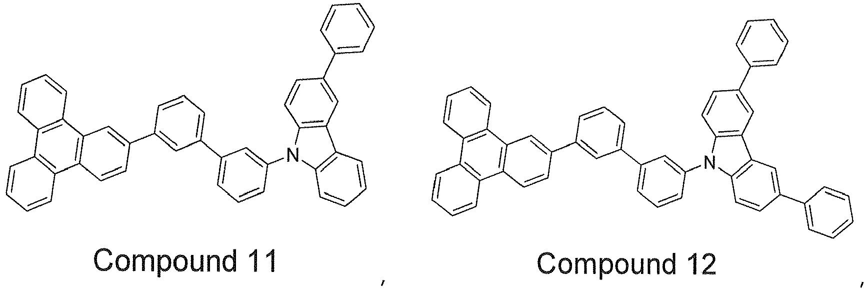 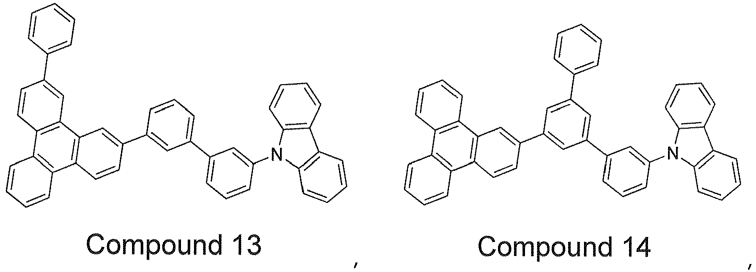 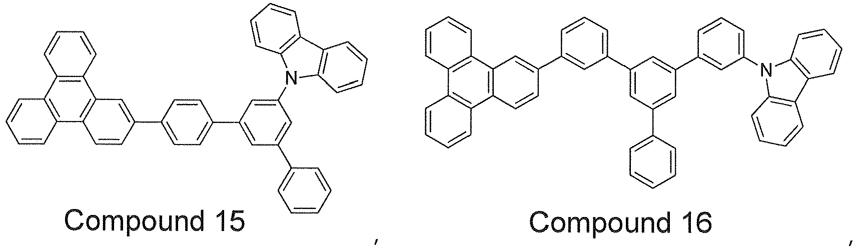 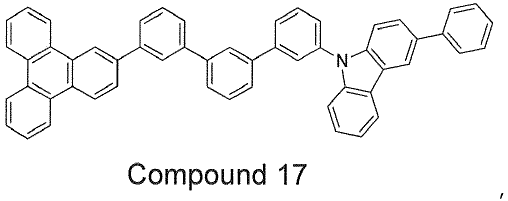 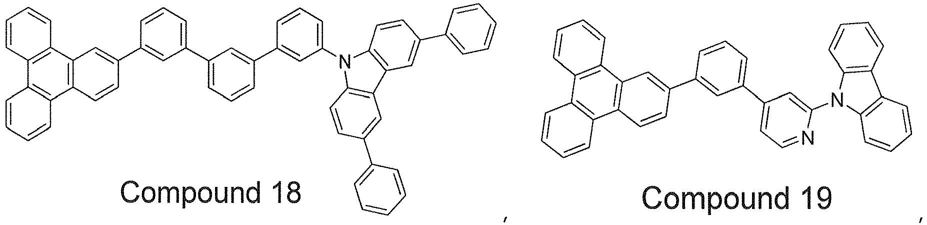 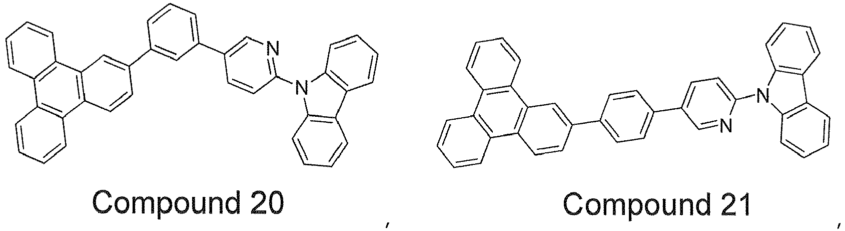 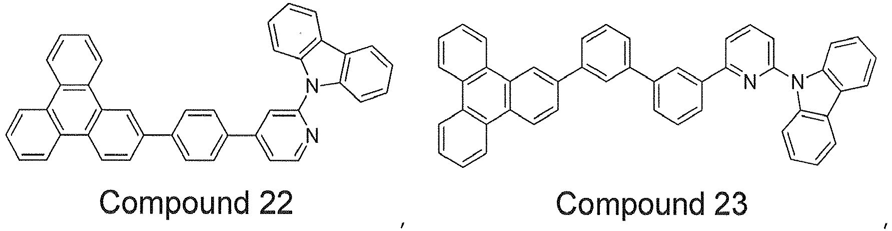 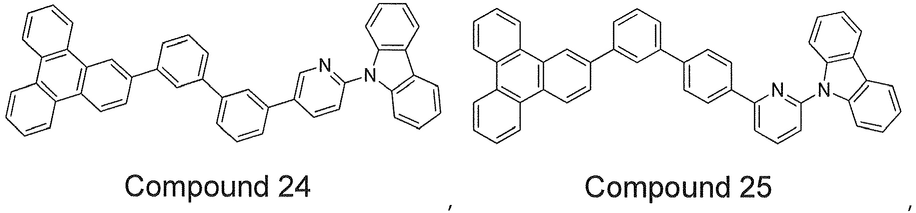 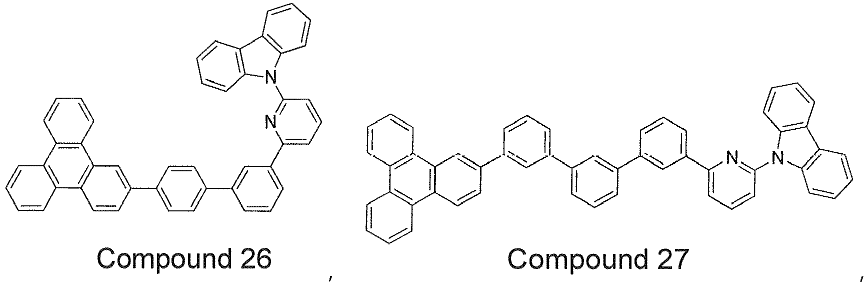 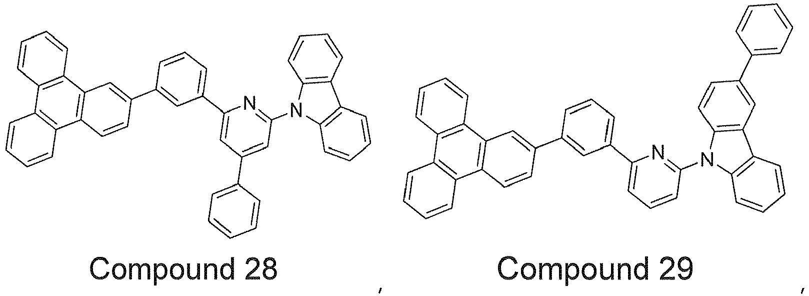 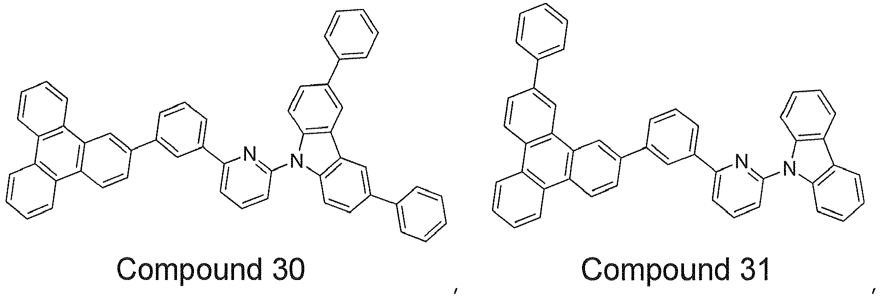 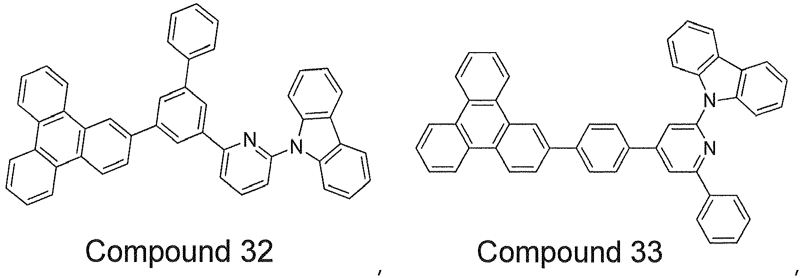 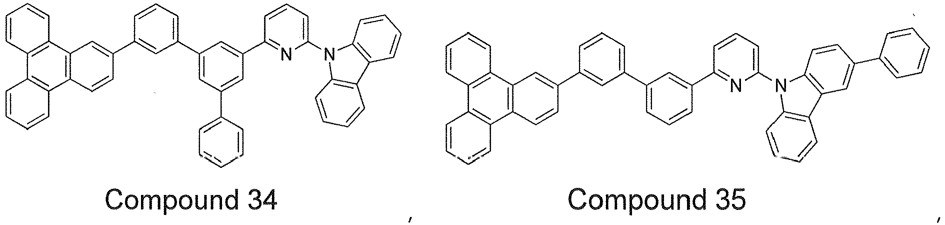 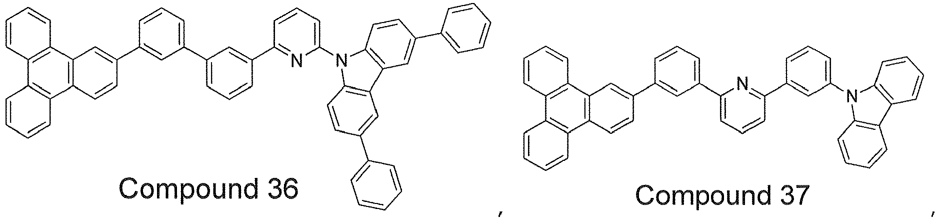 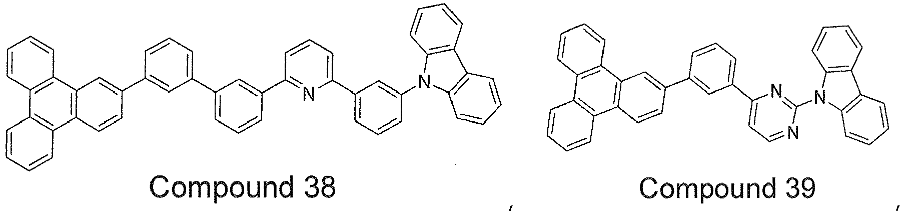 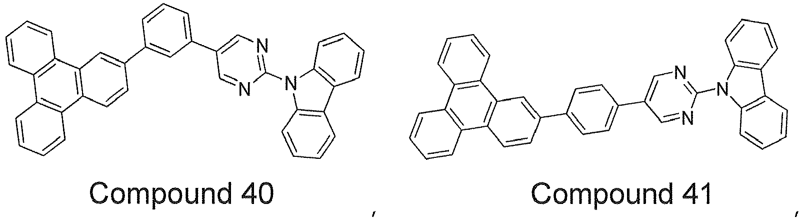 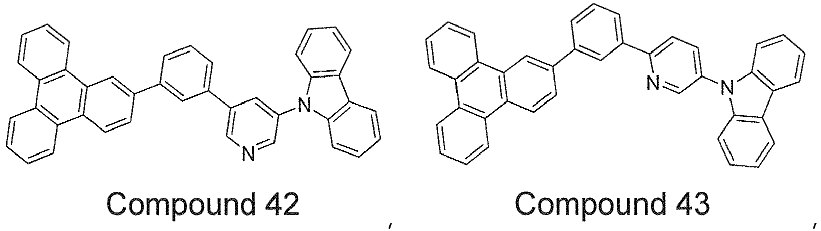 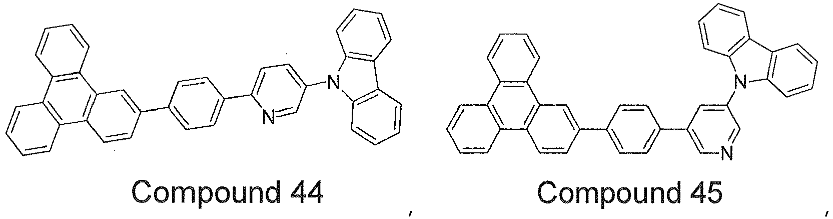 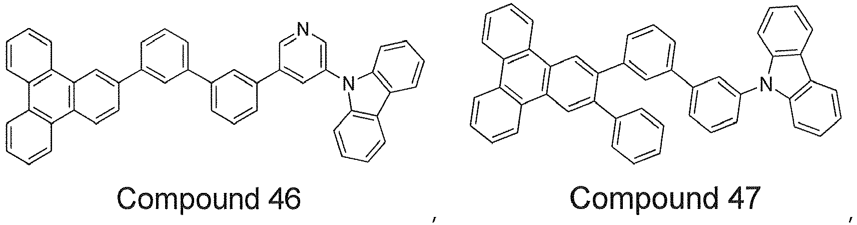 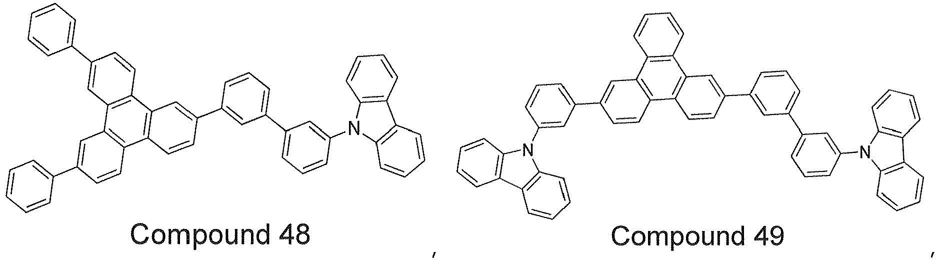 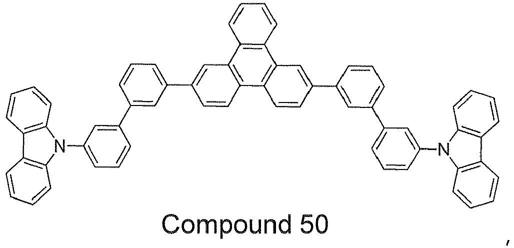 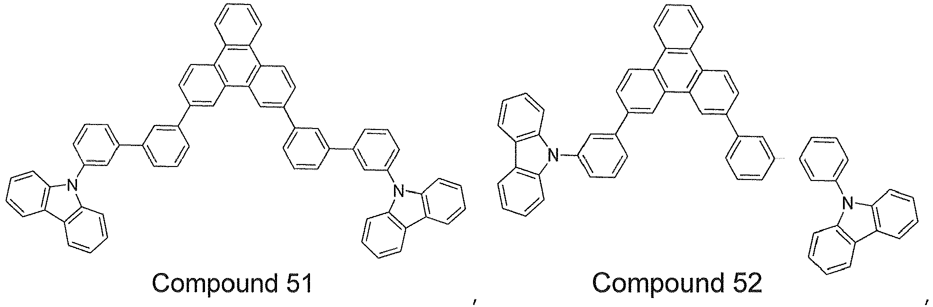 and

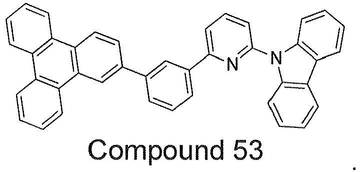

11. The composition of any one of claims 1 to 9, wherein X¹, X³, and X⁵ are N; and wherein X² and X⁴ are CR.

12. The composition of any one of claims 1 to 11, wherein the second compound is selected from the group consisting of:

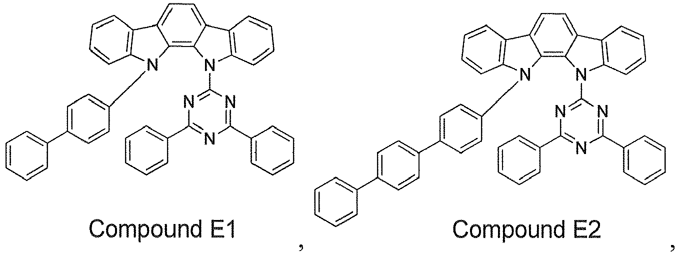

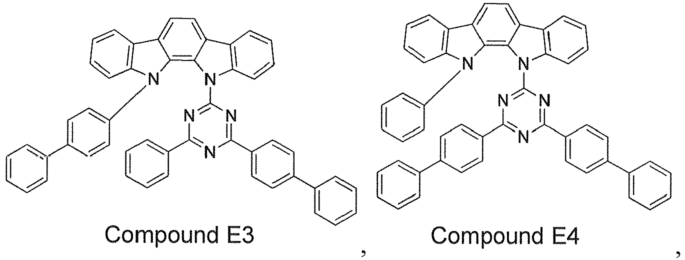

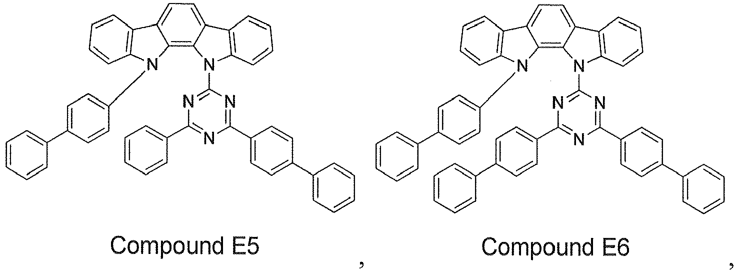

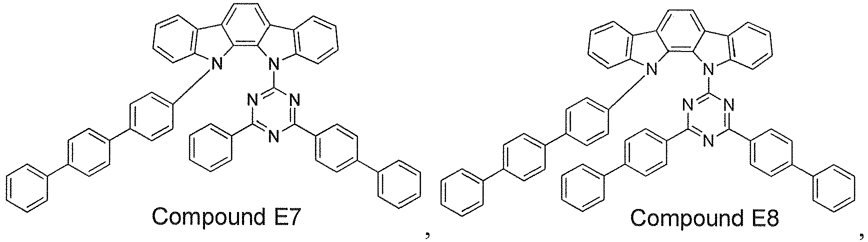

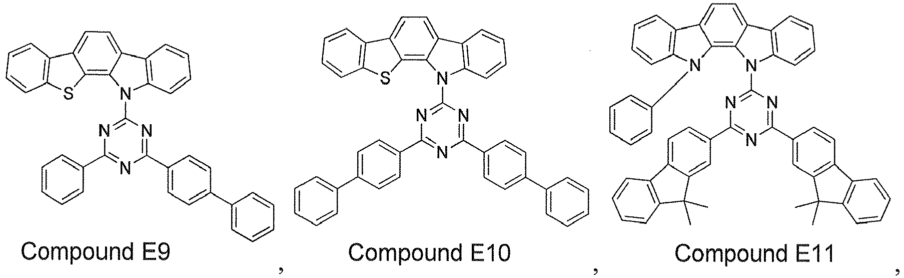

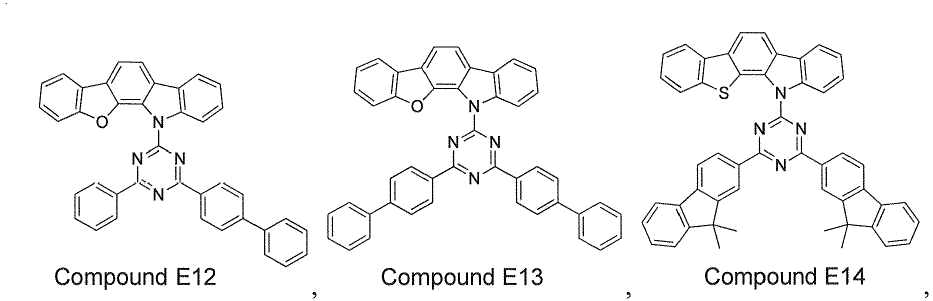

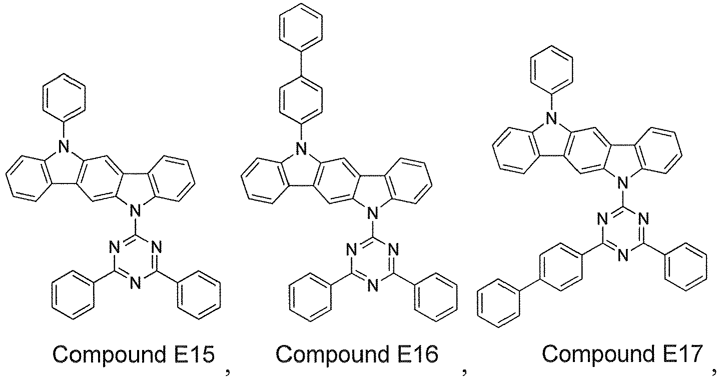

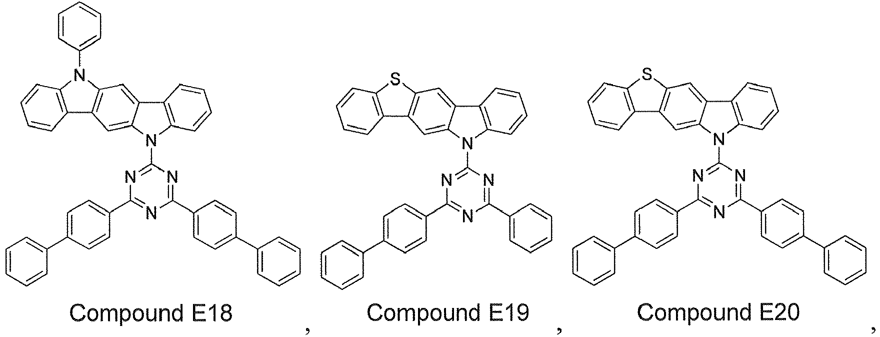

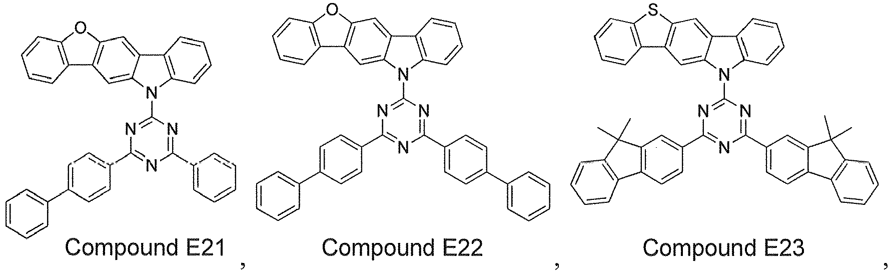

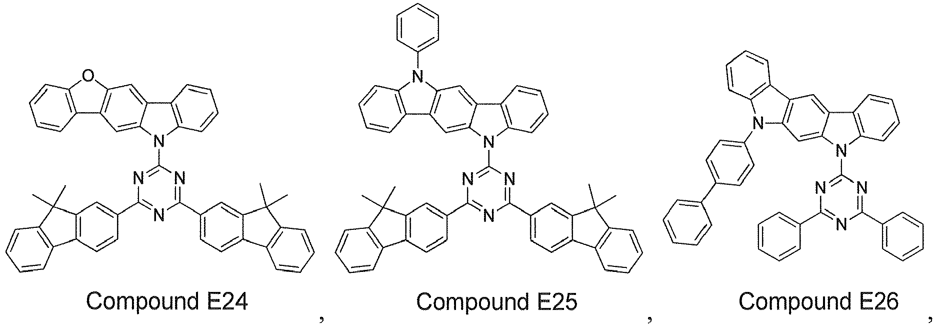

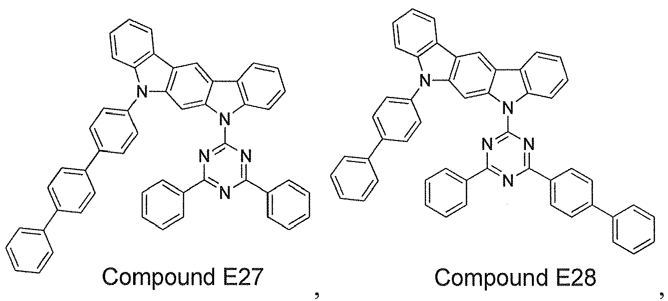

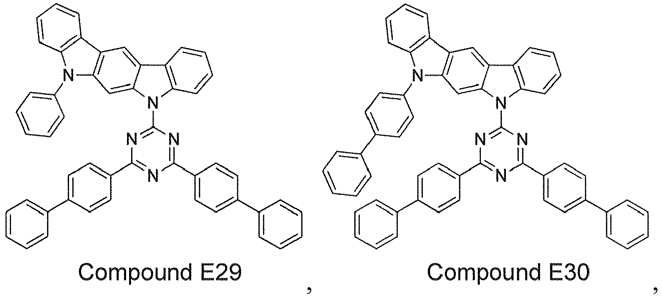

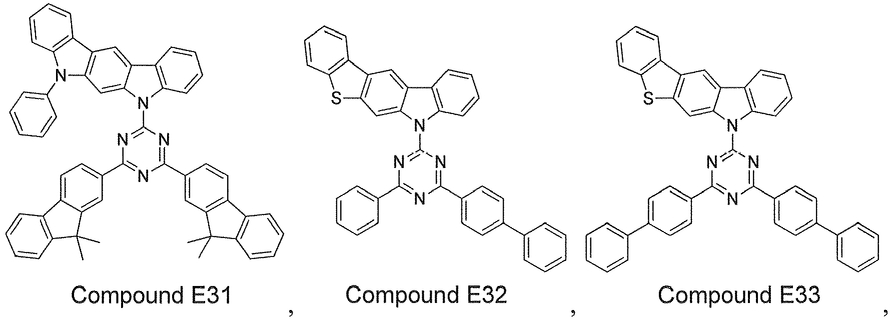

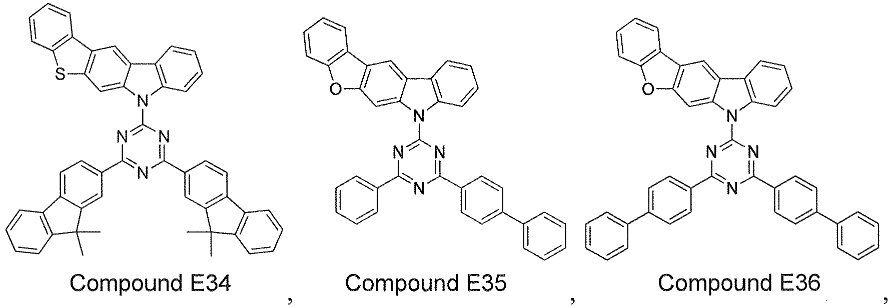

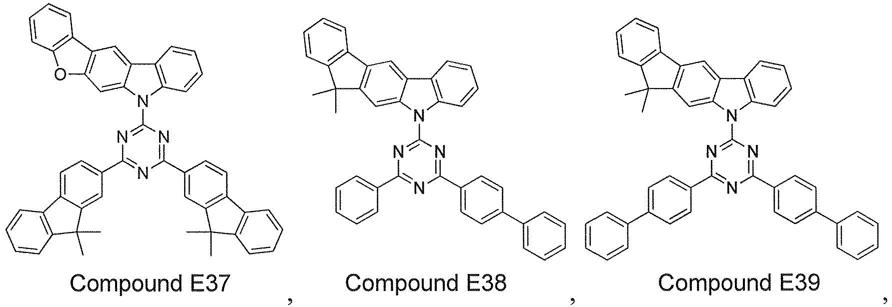

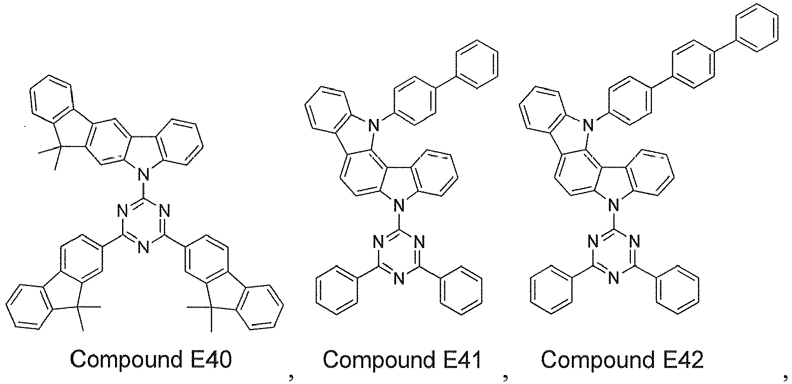

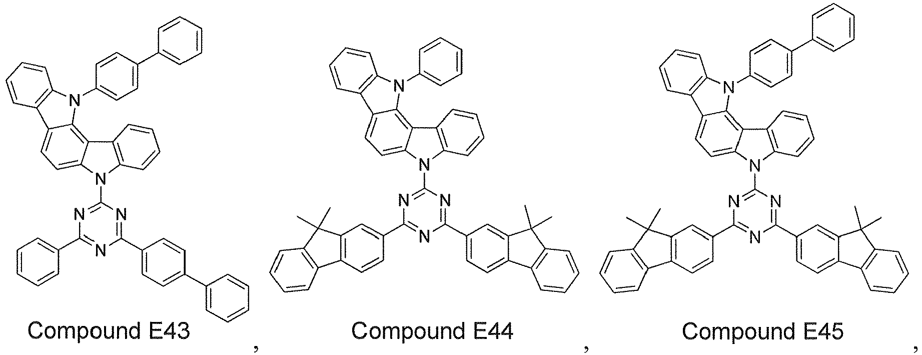

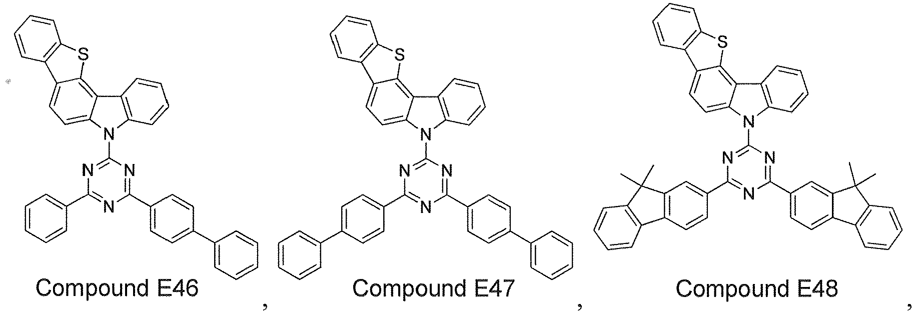

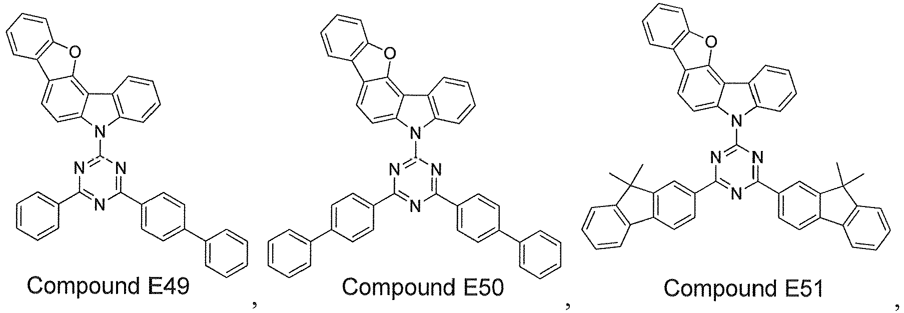

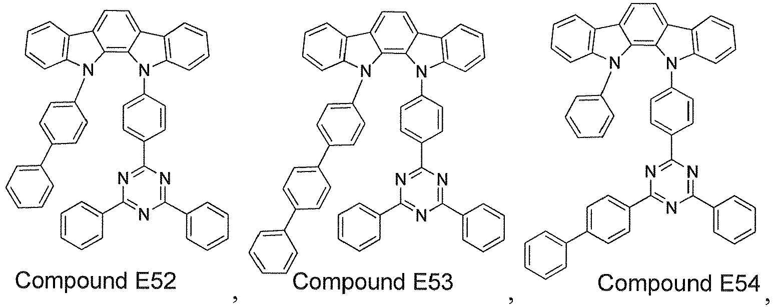

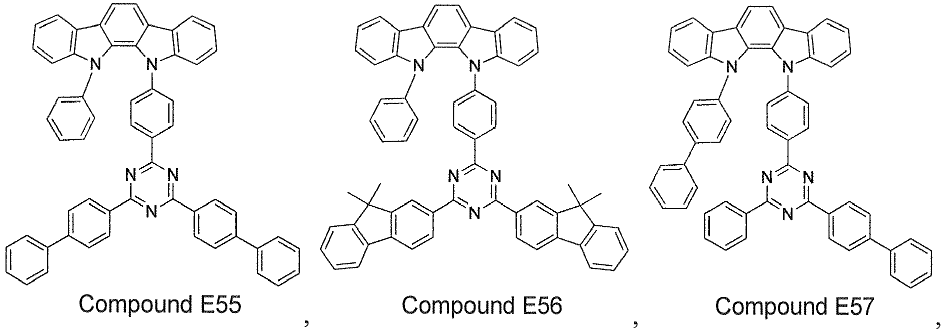

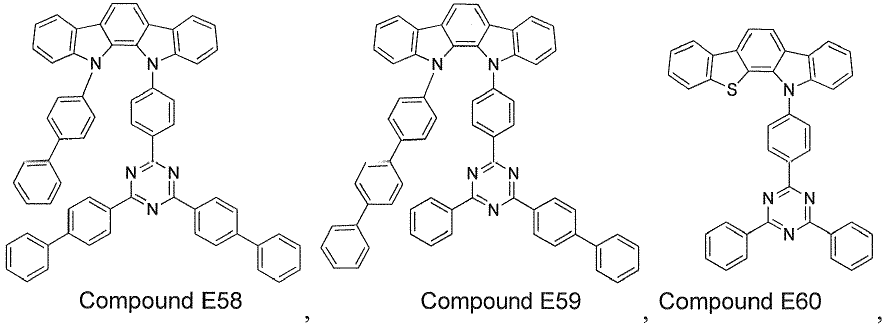

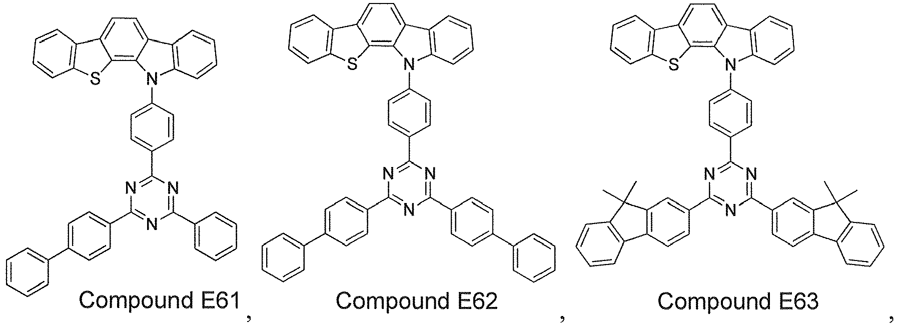

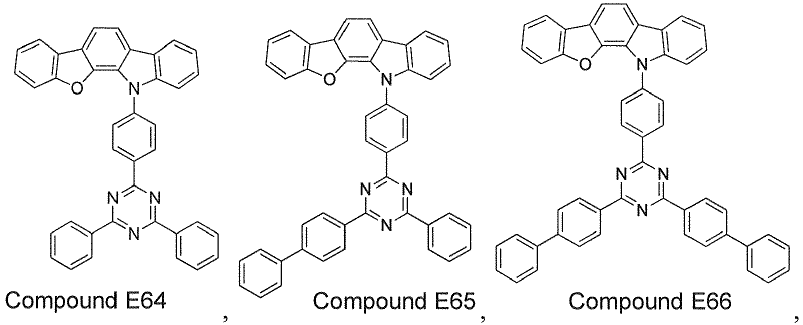

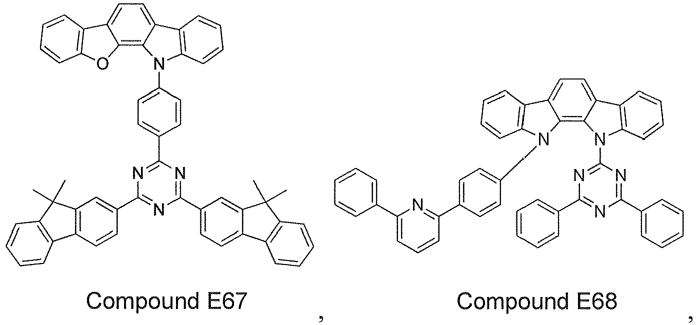

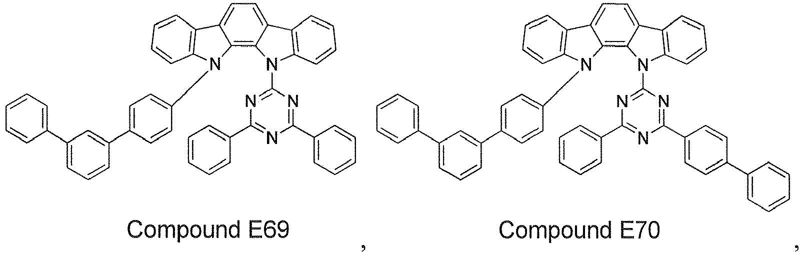

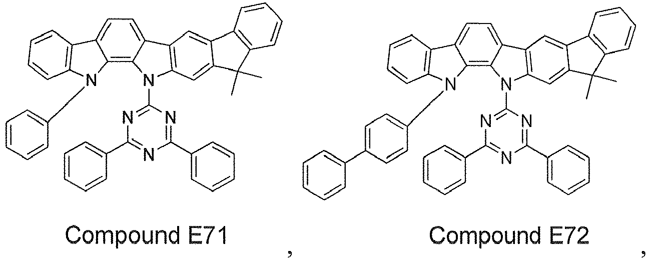

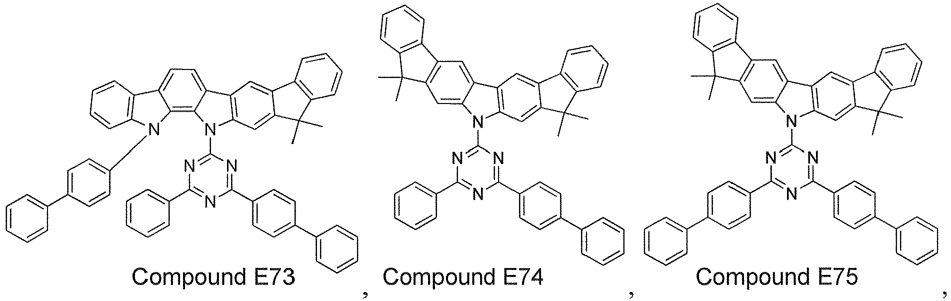

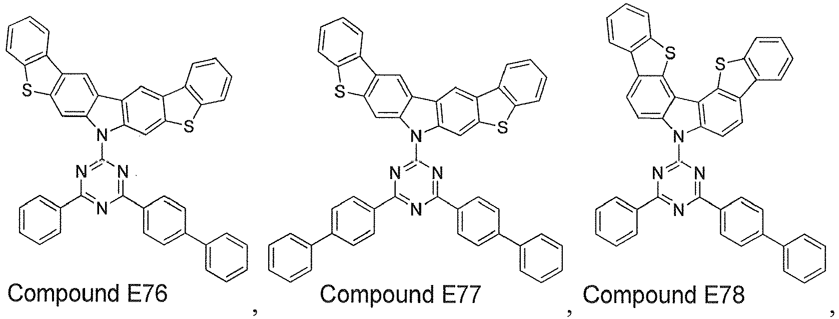

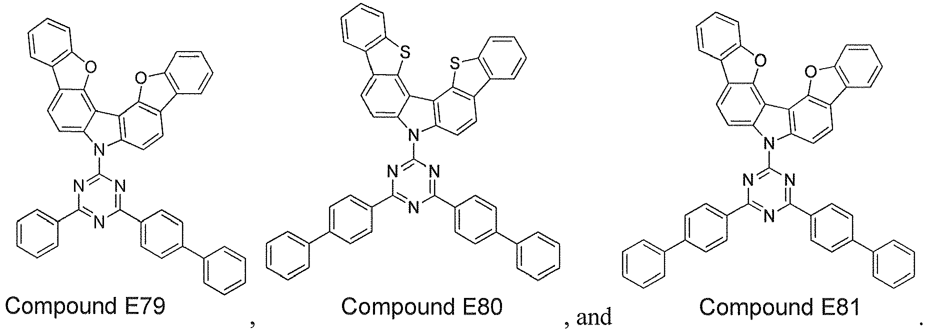

13. The composition of any one of claims 1 to 12, wherein the mixture of the first compound and the second compound is selected from the group consisting of: (Compound 2 and Compound E2), (Compound 3 and Compound E4), (Compound 1 and Compound E5), (Compound 6 and Compound E7), (Compound 7 and Compound E7), (Compound 9 and Compound E8), (Compound 17 and Compound E8), and (Compound 15 and Compound E18).

14. A first device comprising a first organic light emitting device, the first organic light emitting device comprising:
an anode;
a cathode; and
an organic layer, disposed between the anode and the cathode, comprising a composition according to any one of claims 1 to 13.

## Patentansprüche

1. Eine Zusammensetzung umfassend:
eine Mischung aus einer ersten Verbindung und einer zweiten Verbindung, wobei die erste Verbindung eine von der zweiten Verbindung verschiedene chemische Struktur aufweist;
wobei die erste Verbindung in der Lage ist als Lochtransportmaterial in einem organischen Licht emittierenden Gerät bei Raumtemperatur zu funktionieren;
wobei die erste Verbindung mindestens eine Carbazolgruppe umfasst;
wobei die erste Verbindung eine Verdampfungstemperatur T1 von 150 bis 350 °C aufweist;
wobei die zweite Verbindung eine Verdampfungstemperatur T2 von 150 bis 350 °C aufweist;
wobei die Verdampfungstemperaturen in einem Vakuumabscheidegerät bei einem konstanten Druck zwischen 1x10⁻⁶ Torr bis 1x10⁻⁹ Torr bei einer 2Å/sec Abscheiderate gemessen werden;
wobei der absolute Wert von T1-T2 weniger als 20 °C beträgt;
wobei die erste Verbindung eine Konzentration C1 in der Mischung aufweist, und die erste Verbindung eine Konzentration C2 in einem Film aufweist, der durch Verdampfen der Mischung in einem Valcuumabscheidegerät bei einem konstanten Druck zwischen 1x10⁻⁶ Torr bis 1x10⁻⁹ Torr bei einer 2Å/sec Abscheiderate auf eine Oberfläche positioniert bei einer vorbestimmten Distanz von der Mischung, die verdampft wird, gebildet wird;
wobei der absolute Wert von (C1-C2)/C1 weniger als 5% beträgt, und
wobei die erste Verbindung eine Struktur gemäß Formel (I) aufweist: 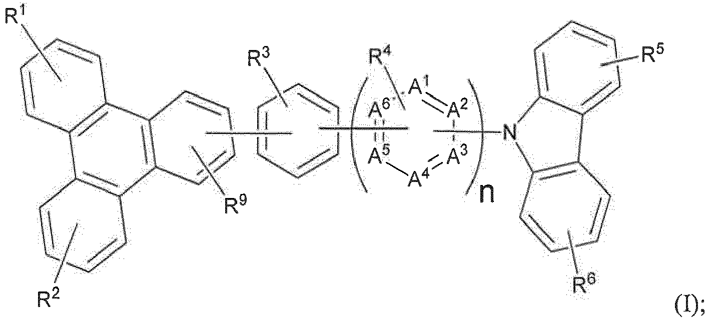 wobei
R¹, R², R³, R⁴, R⁵ und R⁶ jeweils für mono-, di-, tri-, tetra-Substitutionen oder keine Substitutionen stehen;
R⁹ steht für mono-, di-, tri-Substitutionen oder keine Substitutionen;
R¹, R², R³, R⁴, R⁵, R⁶ und R⁹ sind jeweils unabhängig ausgewählt aus der Gruppe bestehend aus Wasserstoff, Deuterium, Halogenid, Alkyl, Cycloalkyl, Heteroalkyl, Arylalkyl, Alkoxy, Aryloxy, Amino, Silyl, Alkenyl, Cycloalkenyl, Heteroalkenyl, Alkinyl, Aryl, Heteroaryl, Acyl, Carbonyl, Carbonsäure, Ester, Nitril, Isonitril, Sulfanyl, Sulfinyl, Sulfonyl, Phosphino und deren Kombinationen;
A¹, A², A³, A⁴, A⁵ und A⁶ sind jeweils unabhängig ausgewählt aus N oder C; und
n ist eine ganze Zahl von 1 bis 20;
wobei die zweite Verbindung eine Struktur gemäß Formel (II) aufweist: 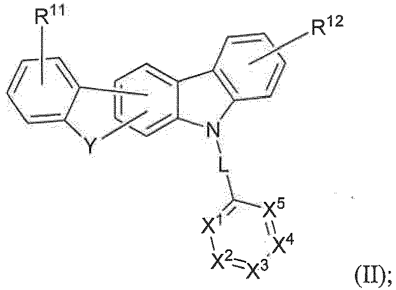
wobei
R¹¹ und R¹² jeweils für mono-, di-, tri-, tetra-Substitutionen oder keine Substitutionen stehen;
Y ist ausgewählt aus der Gruppe bestehend aus O, S, Se, NR' und CR"R"';
L ist eine Einfachbindung oder umfasst eine Aryl- oder Heteroarylgruppe mit 5-24 Kohlenstoffatomen, die gegebenenfalls weiter substituiert ist;
X¹, X², X³, X⁴ und X⁵ sind jeweils unabhängig ausgewählt aus der Gruppe bestehend aus CR und N;
mindestens einer von X¹, X², X³, X⁴ und X⁵ ist N; und
R, R', R" und R''' sind jeweils unabhängig ausgewählt aus der Gruppe bestehend aus Wasserstoff, Deuterium, Halogenid, Allyl, Cycloalkyl, Heteroallcyl, Arylalkyl, Alkoxy, Aryloxy, Amino, Silyl, Alkenyl, Cycloallcenyl, Heteroalkenyl, Alkinyl, Aryl, Heteroaryl, Acyl, Carbonyl, Carbonsäure, Ester, Nitril, Isonitril, Sulfanyl, Sulfinyl, Sulfonyl, Phosphino und deren Kombinationen.

2. Die Zusammensetzung nach Anspruch 1, wobei die erste Verbindung eine Verdampfungstemperatur T1 von 200 bis 350 °C und die zweite Verbindung eine Verdampfungstemperatur von T2 von 200 bis 350 °C aufweist.

3. Die Zusammensetzung nach Anspruch 1 oder 2, wobei der absolute Wert von (C1-C2)/C1 weniger als 3% beträgt.

4. Die Zusammensetzung nach einem der Ansprüche 1 bis 3, wobei die erste Verbindung einen Dampfdruck P1 bei T1, die zweite Verbindung einen Dampfdruck P2 bei T2 aufweist; und
wobei das Verhältnis von P1/P2 innerhalb des Bereichs von 0,90 bis 1,10 liegt.

5. Die Zusammensetzung nach einem der Ansprüche 1 bis 4, wobei die erste Verbindung eine erste Massenverlustrate und die zweite Verbindung eine zweite Massenverlustrate aufweist, wobei das Verhältnis zwischen der ersten Massenverlustrate und der zweiten Massenverlustrate innerhalb des Bereichs von 0,90 bis 1,10 liegt.

6. Die Zusammensetzung nach einem der Ansprüche 1 bis 5, wobei die erste Verbindung ferner mindestens eine der chemischen Gruppen ausgewählt aus der Gruppe bestehend aus Triphenylen, Dibenzothiophen, Dibenzofuran und Dibenzoselenophen umfasst.

7. Die Zusammensetzung nach einem der Ansprüche 1 bis 6, wobei die zweite Verbindung mindestens eine der chemischen Gruppen ausgewählt aus der Gruppe bestehend aus Aza-triphenylen, Aza-carbazol, Aza-dibenzothiophen, Aza-dibenzofuran, Aza-dibenzoselenophen und nicht fusionierte N-enthaltende sechs-gliedrige aromatische Ringe umfasst.

8. Die Zusammensetzung nach einem der Ansprüche 1 bis 7, wobei die erste Verbindung ein HOMO Energieniveau von mehr als -5.8 eV aufweist.

9. Die Zusammensetzung nach einem der Ansprüche 1 bis 8, wobei die Zusammensetzung ferner eine dritte Verbindung umfasst, wobei die dritte Verbindung eine von der ersten und der zweiten Verbindung unterschiedliche chemische Struktur aufweist, wobei die dritte Verbindung eine Verdampfungstemperatur T3 von 150 bis 350 °C aufweist, und wobei der absolute Wert von T1-T3 weniger als 20 °C beträgt.

10. Die Zusammensetzung nach einem der Ansprüche 1 bis 9, wobei die erste Verbindung ausgewählt ist aus der Gruppe bestehend aus:

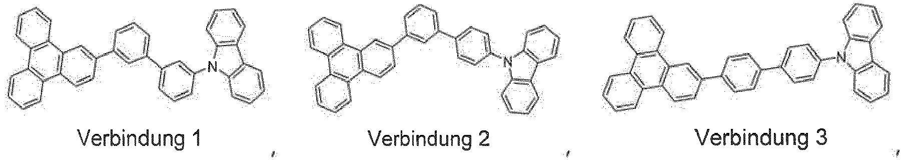

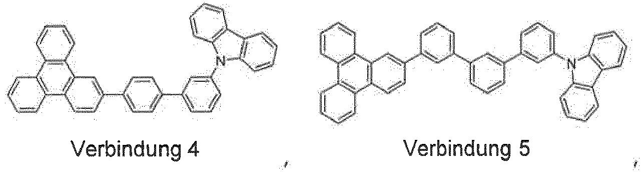

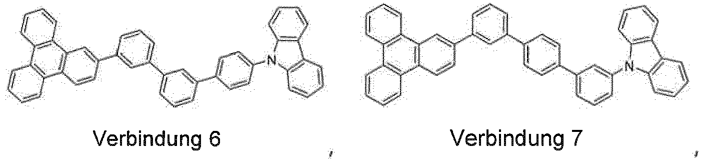

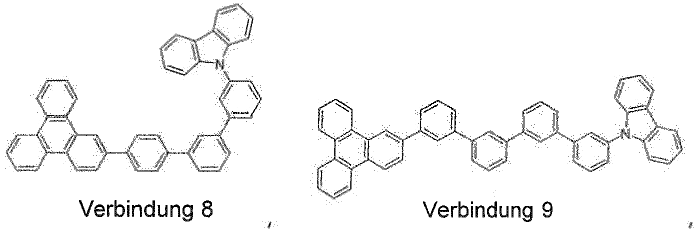

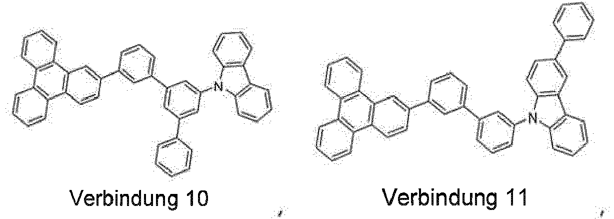

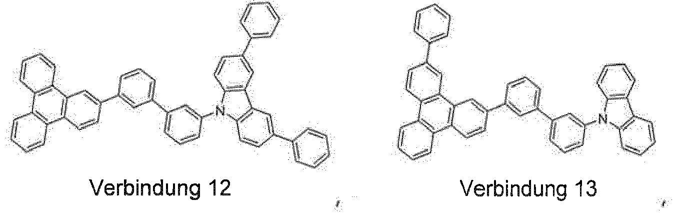

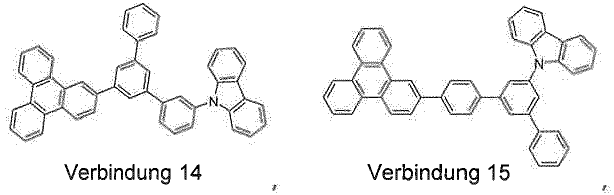

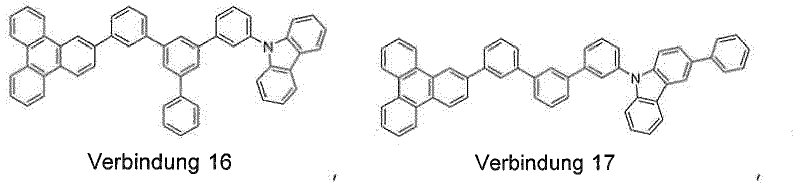

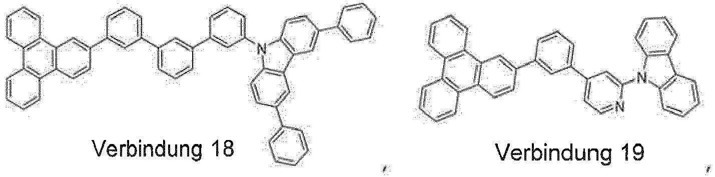

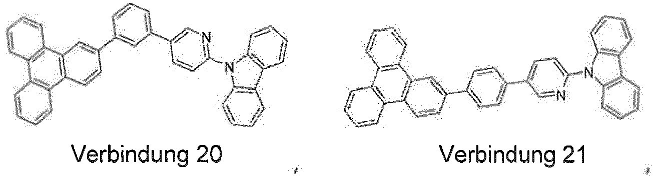

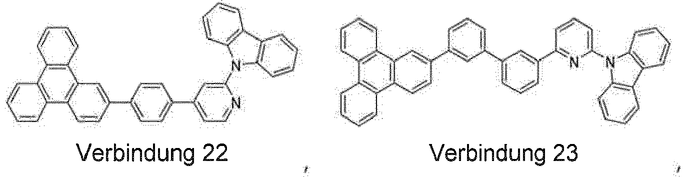

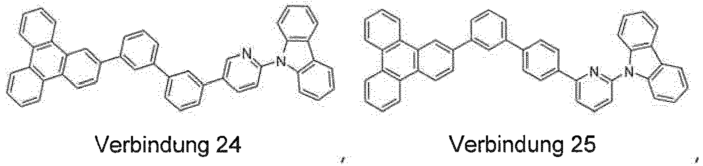

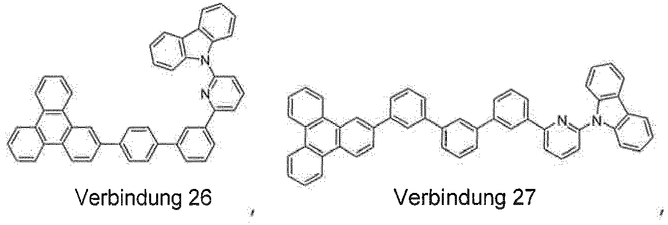

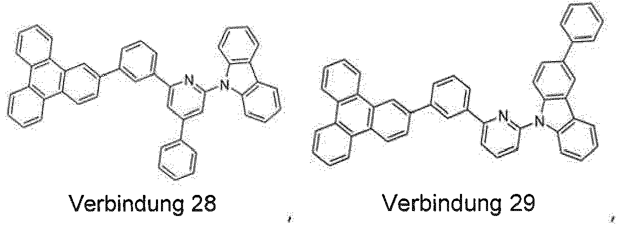

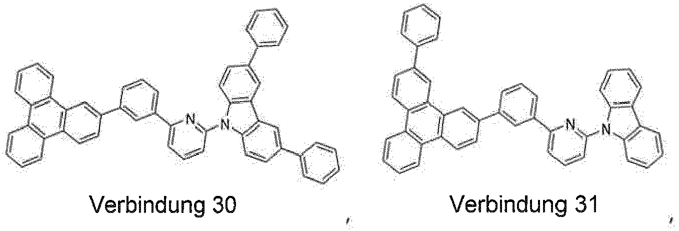

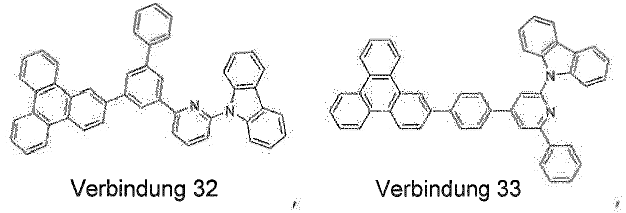

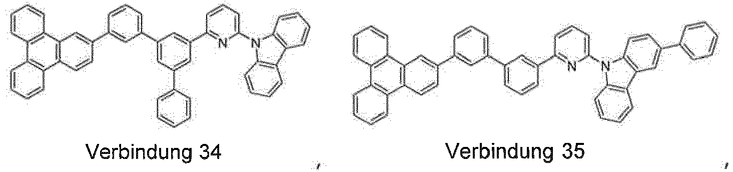

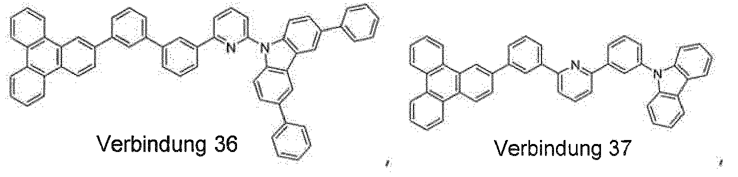

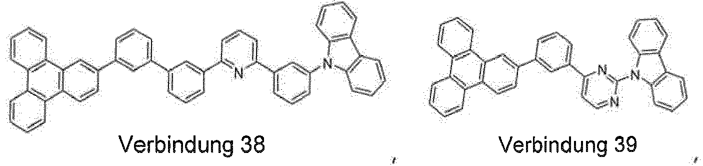

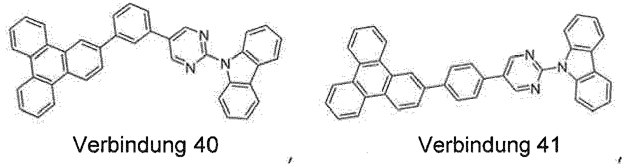

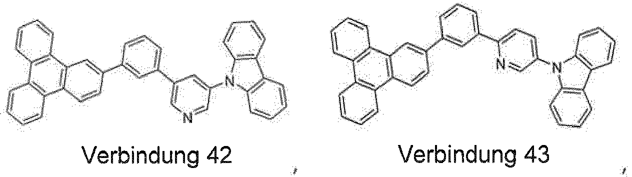

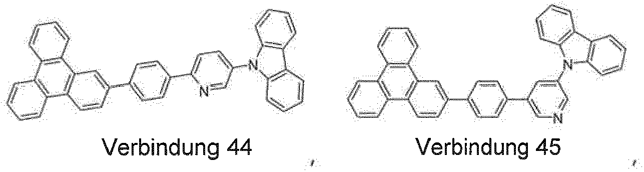

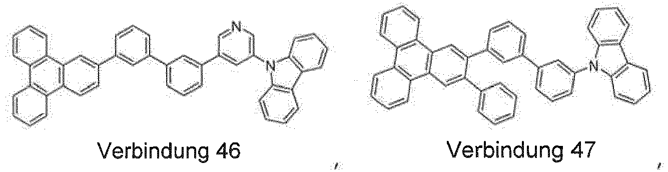

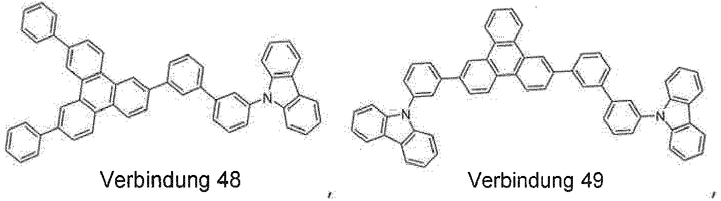

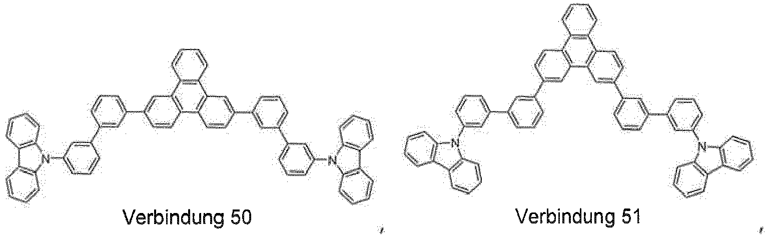

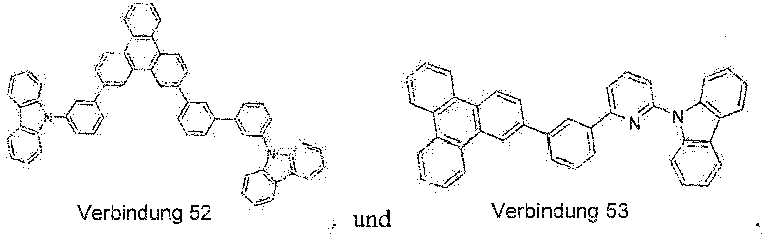

11. Die Zusammensetzung nach einem der Ansprüche 1 bis 9, wobei X¹, X³ und X⁵ gleich N sind; und wobei X² und X⁴ gleich CR sind.

12. Die Zusammensetzung nach einem der Ansprüche 1 bis 11, wobei die zweite Verbindung ausgewählt ist aus der Gruppe bestehend aus:

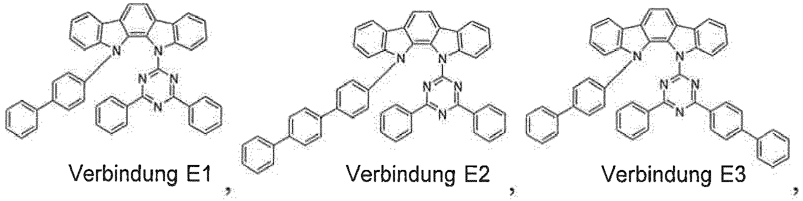

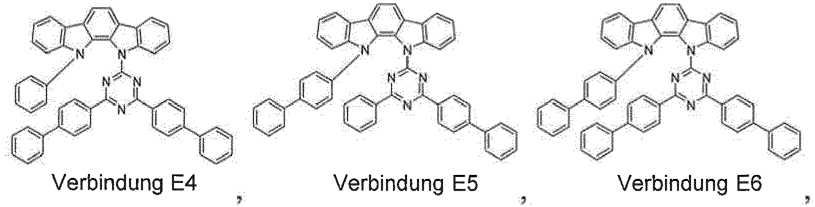

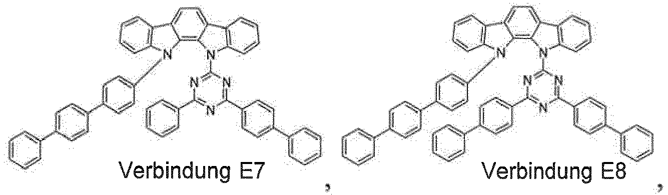

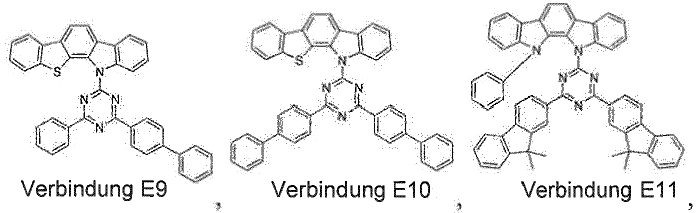

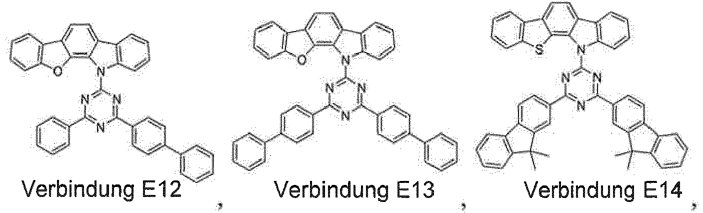

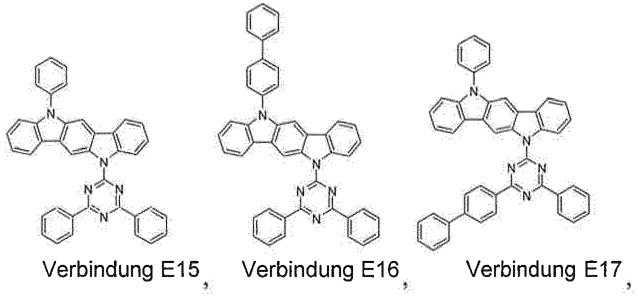

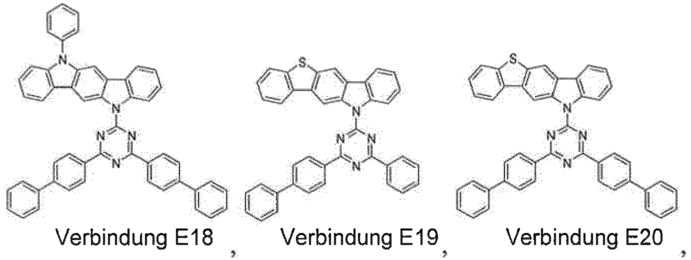

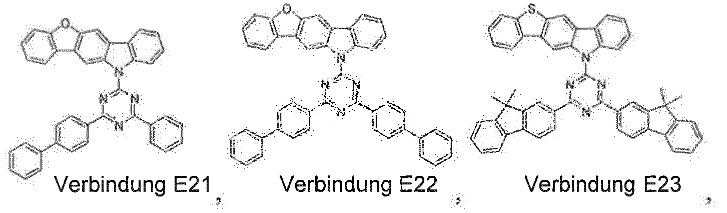

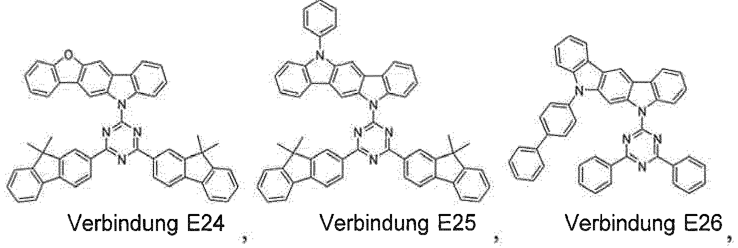

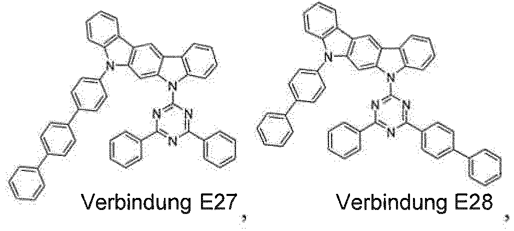

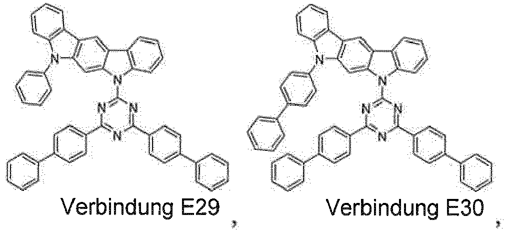

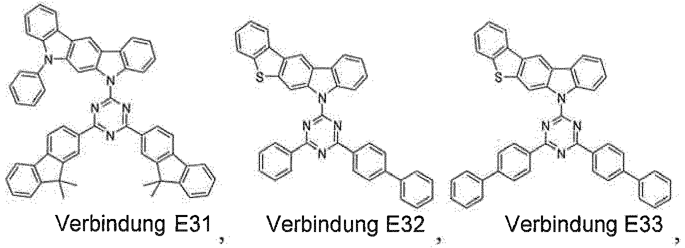

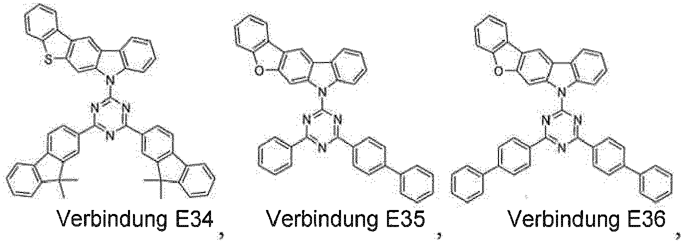

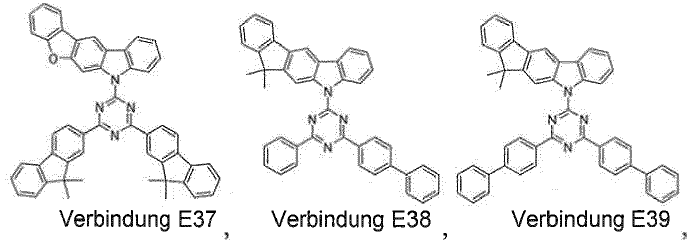

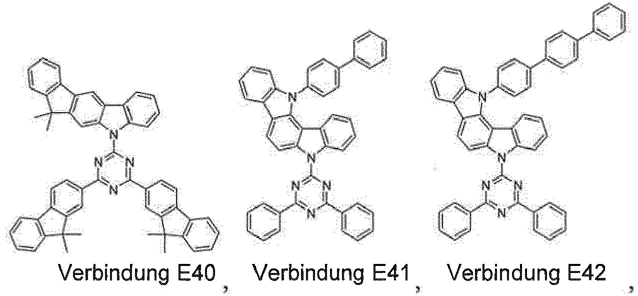

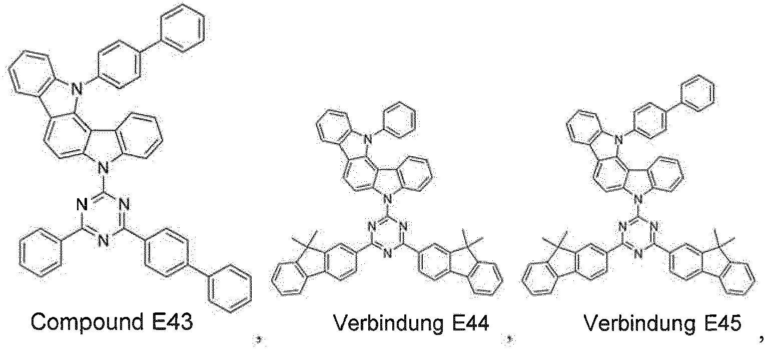

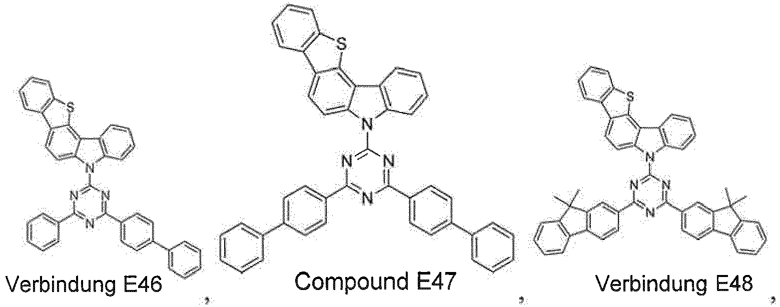

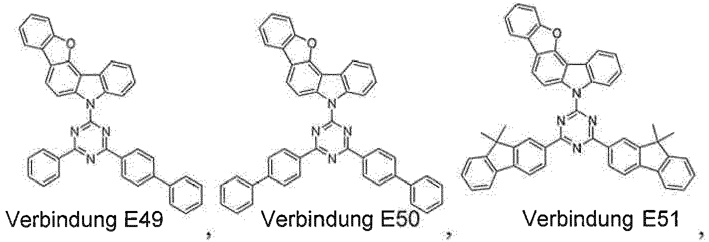

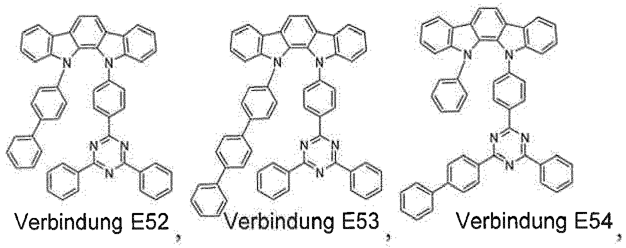

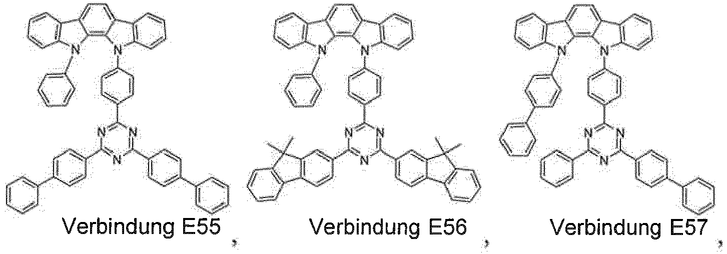

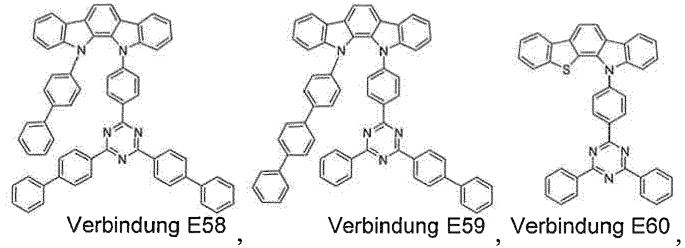

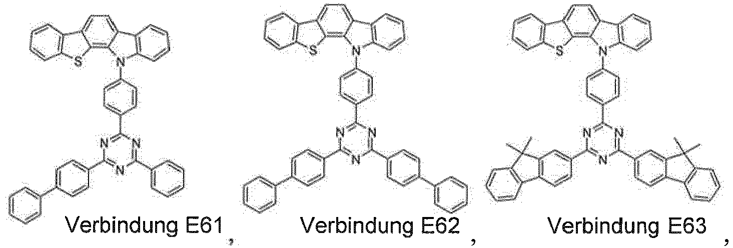

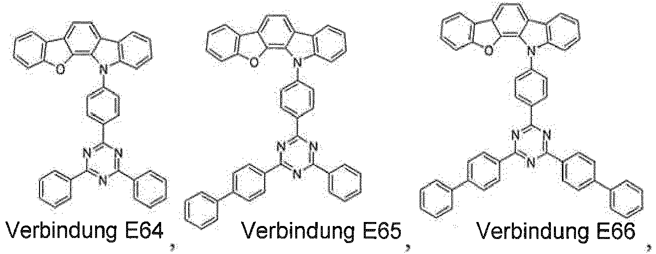

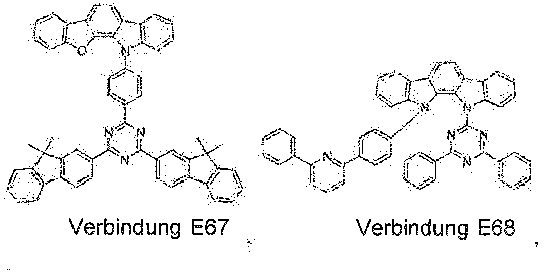

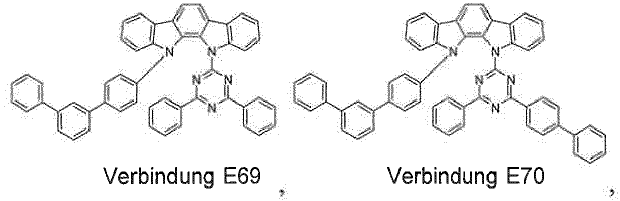

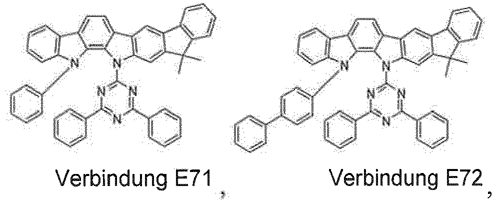

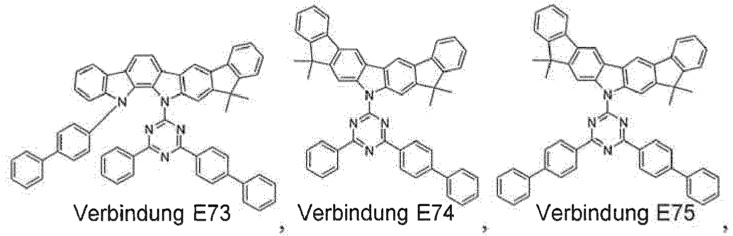

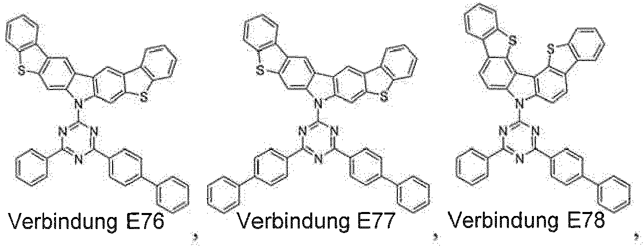

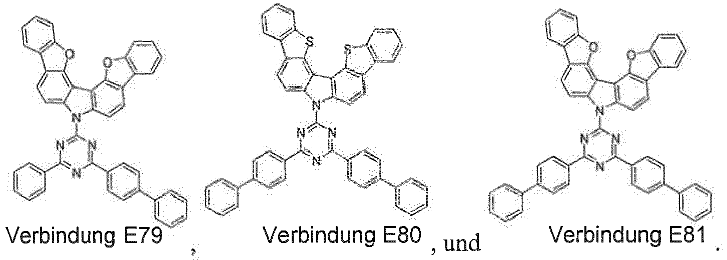

13. Die Zusammensetzung nach einem der Ansprüche 1 bis 12, wobei die Mischung aus der ersten Verbindung und der zweiten Verbindung ausgewählt ist aus der Gruppe bestehend aus: (Verbindung 2 und Verbindung E2), (Verbindung 3 und Verbindung E4), (Verbindung 1 und Verbindung E5), (Verbindung 6 und Verbindung E7), (Verbindung 7 und Verbindung E7), (Verbindung 9 und Verbindung E8), (Verbindung 17 und Verbindung E8), und (Verbindung 15 und Verbindung E18).

14. Eine erste Vorrichtung umfassend eine erste organische Licht emittierende Vorrichtung, die erste organische Licht emittierende Vorrichtung umfassend:
eine Anode;
eine Kathode; und
eine organische Schicht angeordnet zwischen der Anode und der Kathode, umfassend eine Zusammensetzung nach einem der Ansprüche 1 bis 13.

## Revendications

1. Composition comprenant :
un mélange d'un premier composé et d'un deuxième composé, le premier composé ayant une structure chimique différente du deuxième composé ;
dans laquelle le premier composé est capable de fonctionner en tant que matériau transporteur de trou dans un dispositif électroluminescent organique à température ambiante ;
dans laquelle le premier composé comprend au moins un groupe carbazole ;
dans laquelle le premier composé a une température d'évaporation T1 de 150 à 350 °C ;
dans laquelle le deuxième composé a une température d'évaporation T2 de 150 à 350 °C ;
dans laquelle les températures d'évaporation sont mesurées dans un outil de dépôt sous vide à une pression constante, comprise entre 1x10⁻⁶ Torr et 1 x 10⁻⁹ Torr, à un taux de dépôt de 2 Å/s ;
dans laquelle la valeur absolue de T1-T2 est inférieure à 20 °C ;
dans laquelle le premier composé a une concentration C1 dans ledit mélange, et le premier composé a une concentration C2 dans un film formé par évaporation du mélange dans un outil de dépôt sous vide à une pression constante, comprise entre 1x10⁻⁶ Torr et 1 x 10⁻⁹ Torr, à un taux de dépôt de 2 Å/s sur une surface positionnée à une distance prédéfinie du mélange étant évaporé ;
dans laquelle la valeur absolue de (C₁-C₂)/C1 est inférieure à 5 %, et
dans laquelle le premier composé a une structure selon une formule (I) : 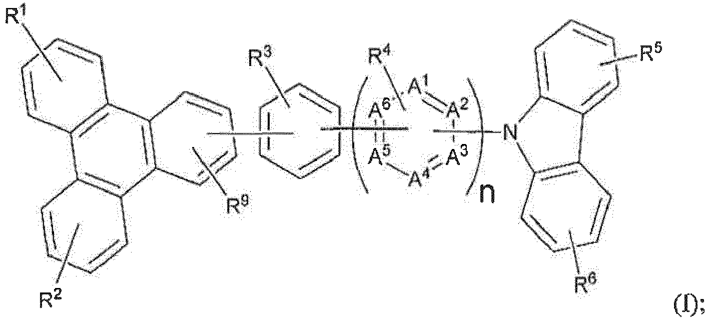 dans laquelle
R¹, R², R³, R⁴, R⁵ et R⁶ représentent chacun des mono, di, tri, tétra-substitutions, ou aucune substitution ;
R⁹ représente des mono, di, tri-substitutions, ou aucune substitution ; R¹, R², R³, R⁴, R⁵, R⁶ et R⁹ sont chacun indépendamment choisis dans le groupe constitué d'hydrogène, deutérium, halogénure, alkyle, cycloalkyle, hétéroalkyle, arylalkyle, alcoxy, aryloxy, amino, silyle, alcényle, cycloalcényle, hétéroalcényle, alcynyle, aryle, hétéroaryle, acyle, carbonyle, acide carboxylique, ester, nitrile, isonitrile, sulfanyle, sulfinyle, sulfonyle, phosphino, et des combinaisons de ceux-ci ;
A¹, A², A³, A⁴, A⁵ et A⁶ sont chacun indépendamment choisis parmi N ou C ; et
n est un entier de 1 à 20 ;
dans laquelle le deuxième composé a une structure selon une formule (II) : 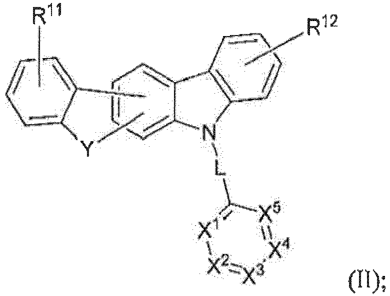
dans laquelle
R¹¹ et R¹² représentent chacun des mono, di, tri, tétra-substitutions, ou aucune substitution ;
Y est choisi dans le groupe constitué de O, S, Se, NR', et CR"R"' ;
L est une simple liaison ou comprend un groupe aryle ou hétéroaryle ayant de 5 à 24 atomes de carbone, qui est facultativement substitué plus avant ;
X¹, X², X³, X⁴ et X⁵ sont chacun indépendamment choisis dans le groupe constitué de CR et N ;
au moins l'un de X¹, X², X³, X⁴ et X⁵ est N ; et R, R', R" et R''' sont chacun indépendamment choisis dans le groupe constitué d'hydrogène, deutérium, halogénure, alkyle, cycloalkyle, hétéroalkyle, arylalkyle, alcoxy, aryloxy, amino, silyle, alcényle, cycloalcényle, hétéroalcényle, alcynyle, aryle, hétéroaryle, acyle, carbonyle, acide carboxylique, ester, nitrile, isonitrile, sulfanyle, sulfinyle, sulfonyle, phosphino, et des combinaisons de ceux-ci.

2. Composition de la revendication 1, dans laquelle le premier composé a une température d'évaporation T1 de 200 à 350 °C et le deuxième composé a une température d'évaporation T2 de 200 à 350 °C.

3. Composition de la revendication 1 ou 2, dans laquelle la valeur absolue de (C1-C2)/C1 est inférieure à 3 %.

4. Composition de l'une quelconque des revendications 1 à 3, dans laquelle le premier composé a une pression de vapeur de P1 à T1, le deuxième composé a une pression de vapeur de P2 à T2 ; et
dans laquelle le rapport P1/P2 est dans la plage de 0,90 à 1,10.

5. Composition de l'une quelconque des revendications 1 à 4, dans laquelle le premier composé a un premier taux de perte de masse et le deuxième composé a un deuxième taux de perte de masse, dans laquelle le rapport entre le premier taux de perte de masse et le deuxième taux de perte de masse est dans la plage de 0,90 à 1,10.

6. Composition de l'une quelconque des revendications 1 à 5, dans laquelle le premier composé comprend en outre au moins un des groupes chimiques choisis dans le groupe constitué des triphénylène, dibenzothiophène, dibenzofurane et dibenzosélénophène.

7. Composition de l'une quelconque des revendications 1 à 6, dans laquelle le deuxième composé comprend au moins un des groupes chimiques choisis dans le groupe constitué des aza-triphénylène, aza-carbazole, aza-dibenzothiophène, aza-dibenzofurane, aza-dibenzosélénophène et des cycles aromatiques à six chaînons contenant N non condensés.

8. Composition de l'une quelconque des revendications 1 à 7, dans laquelle le premier composé a un niveau d'énergie HOMO supérieur à -5,8 eV.

9. Composition de l'une quelconque des revendications 1 à 8, la composition comprenant en outre un troisième composé, dans laquelle le troisième composé a une structure chimique différente des premier et deuxième composés, dans laquelle le troisième composé a une température d'évaporation T3 de 150 à 350 °C, et dans laquelle la valeur absolue de T1-T3 est inférieure à 20 °C.

10. Composition de l'une quelconque des revendications 1 à 9, dans laquelle le premier composé est choisi dans le groupe constitué de : 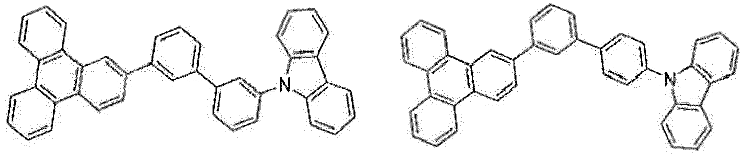 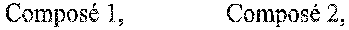 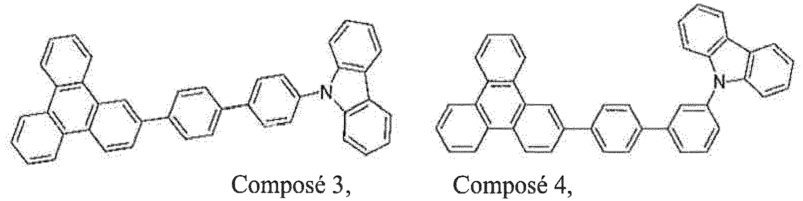 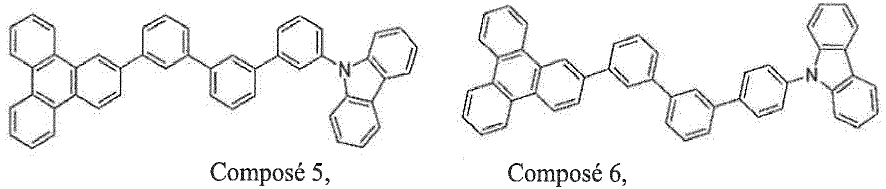 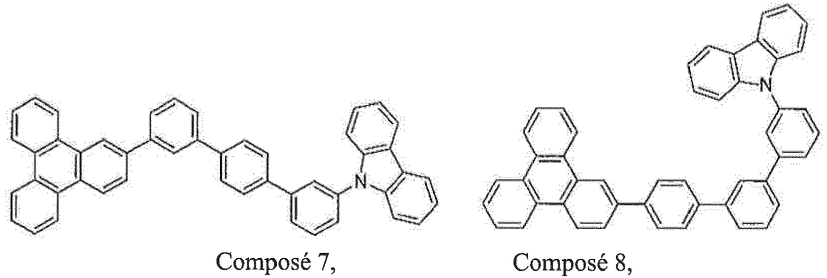 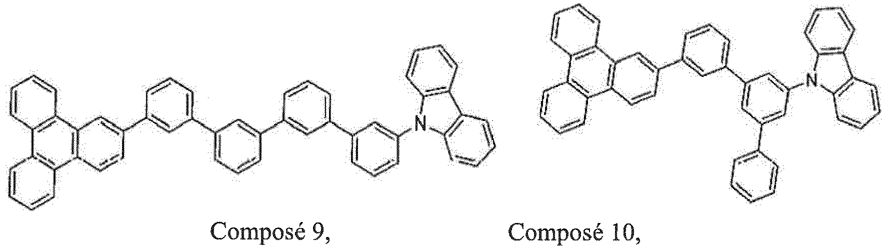 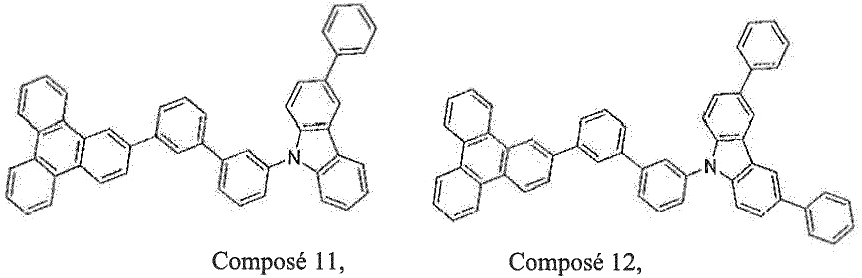 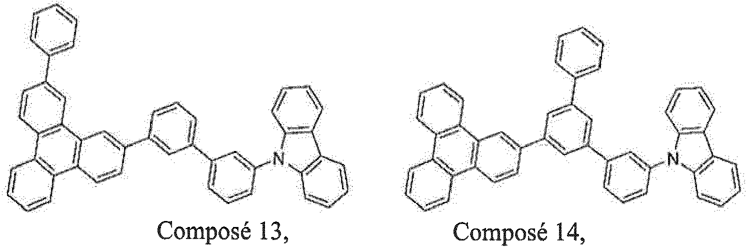 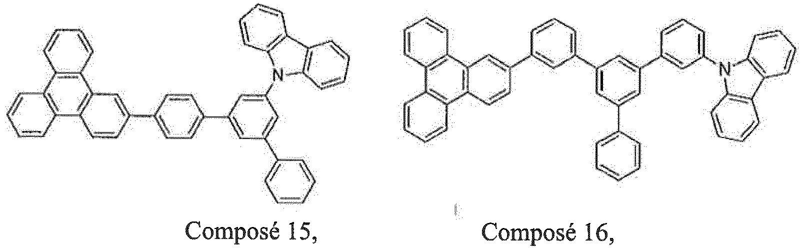 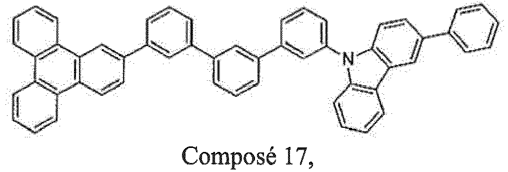 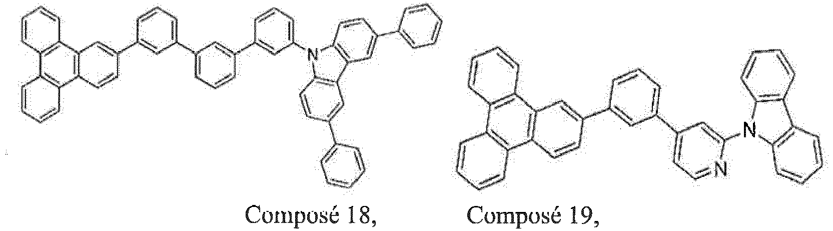 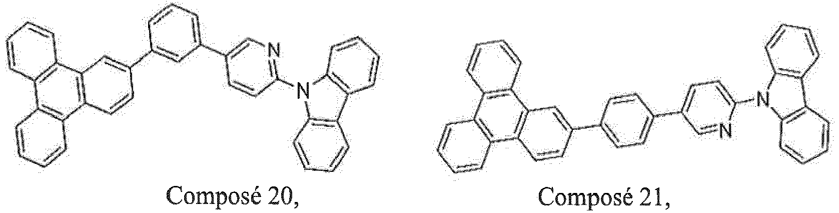 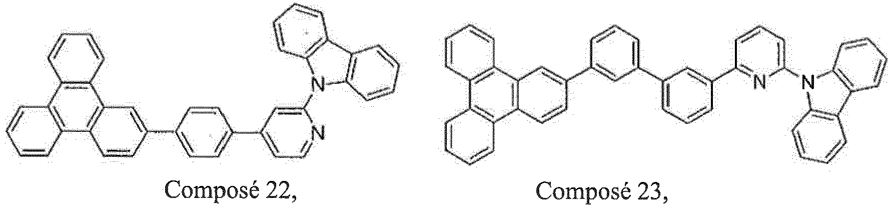 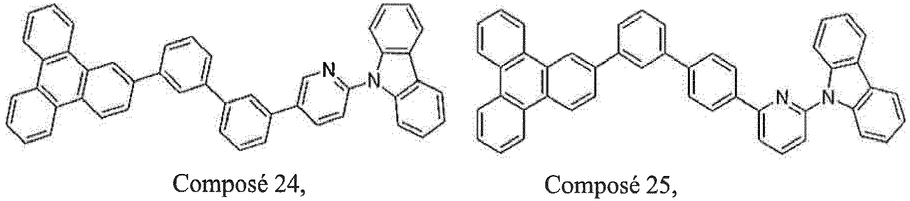 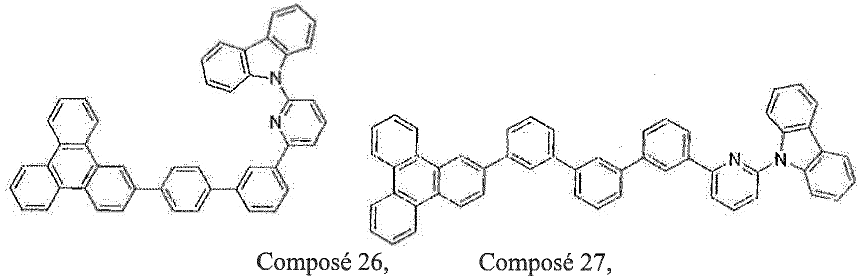 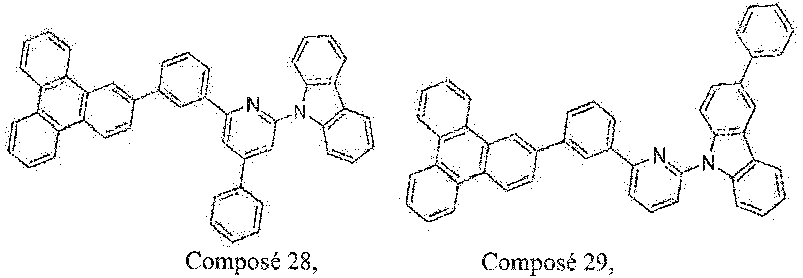 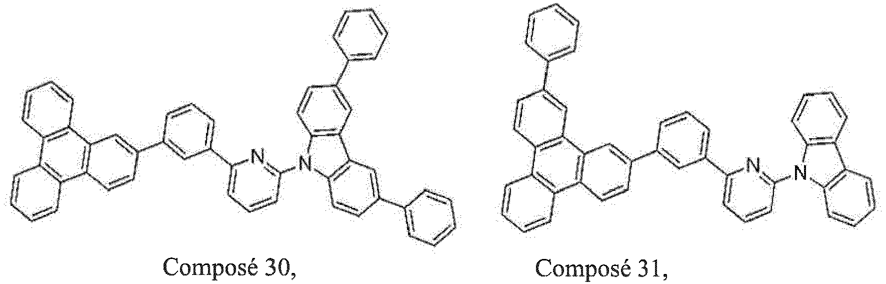 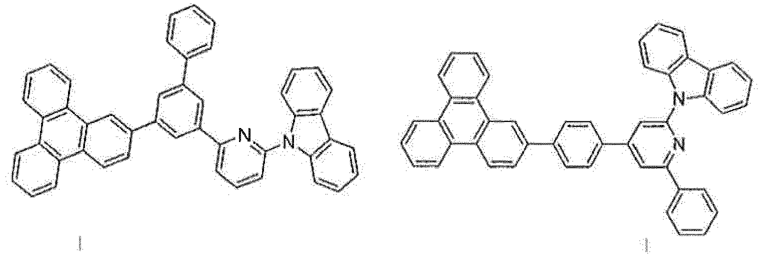 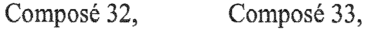 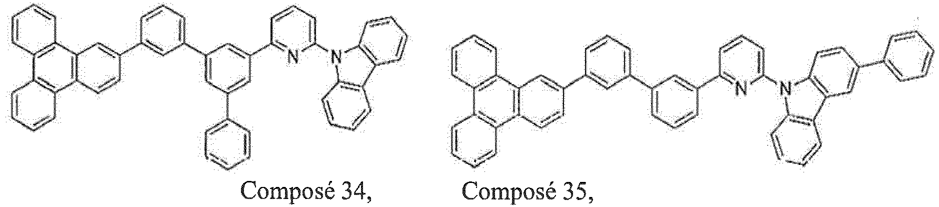 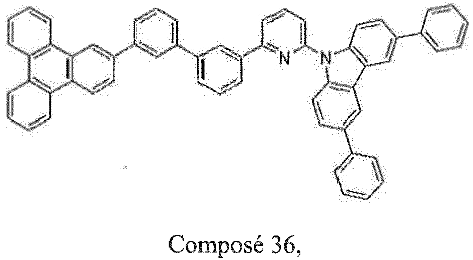 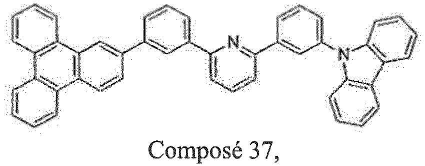 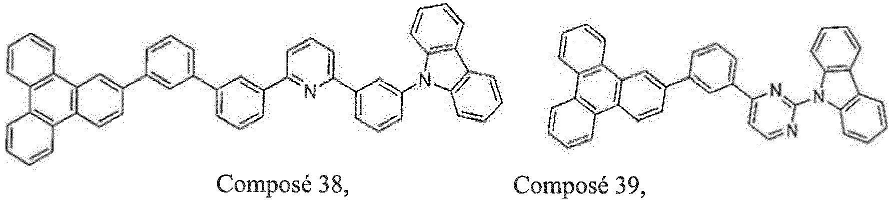 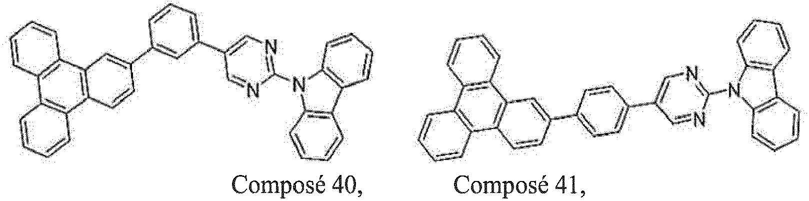 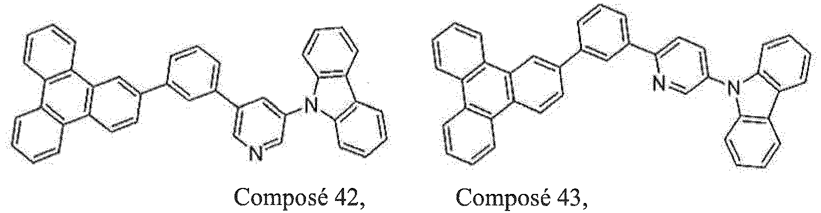 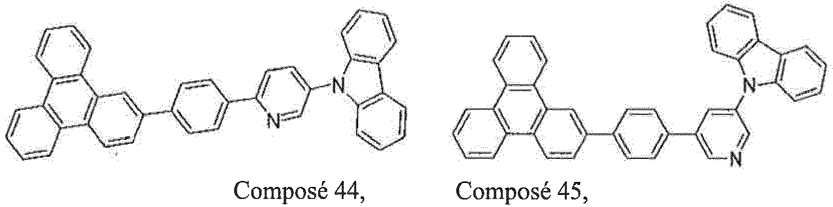 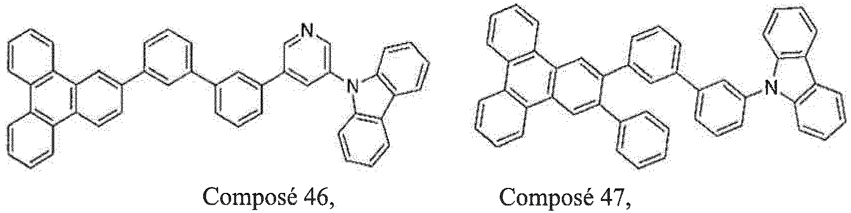 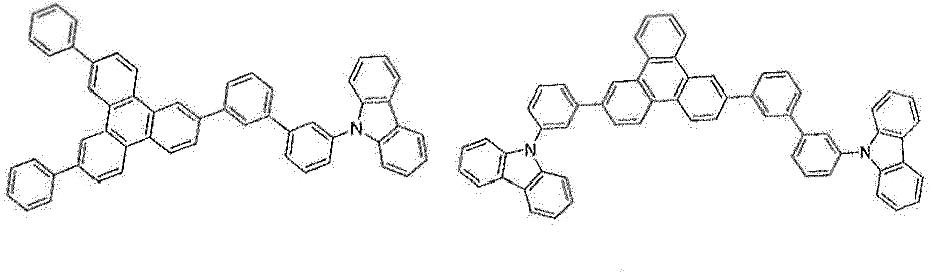 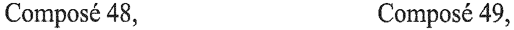 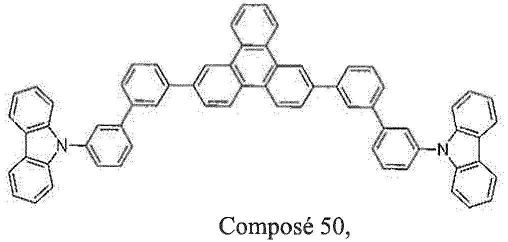 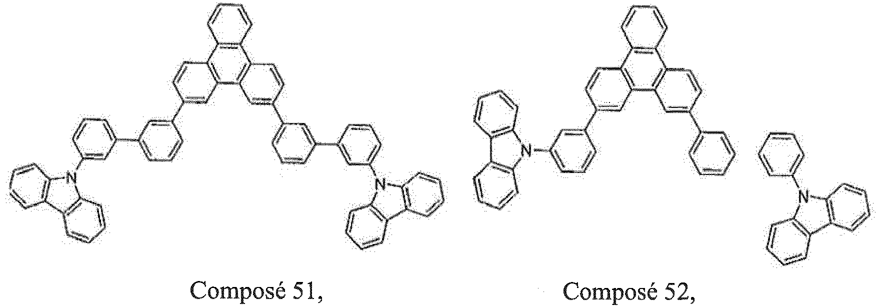
et

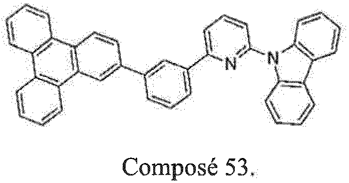

11. Composition de l'une quelconque des revendications 1 à 9, dans laquelle X¹, X³ et X⁵ sont N ; et dans laquelle X² et X⁴ sont CR.

12. Composition de l'une quelconque des revendications 1 à 11, dans laquelle le deuxième composé est choisi dans le groupe constitué de :

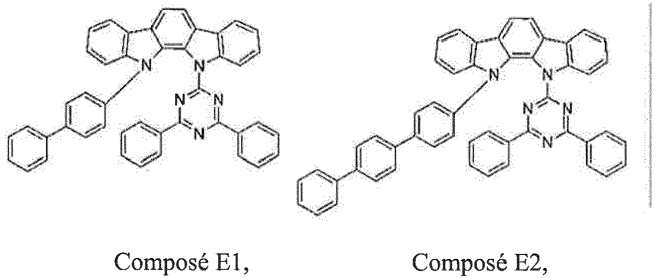

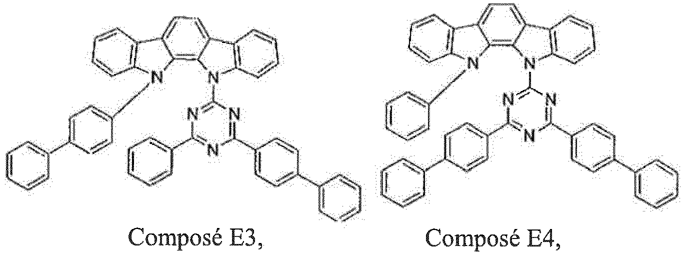

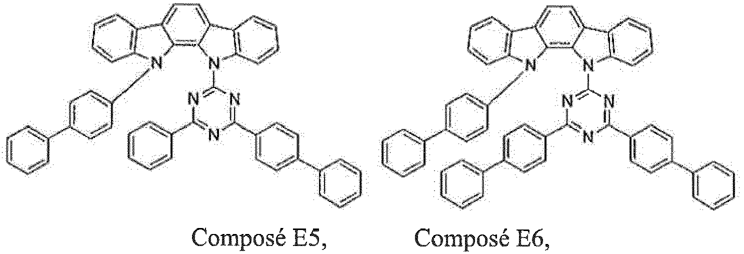

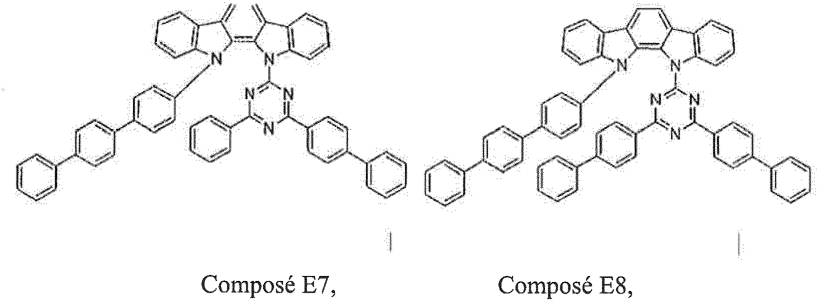

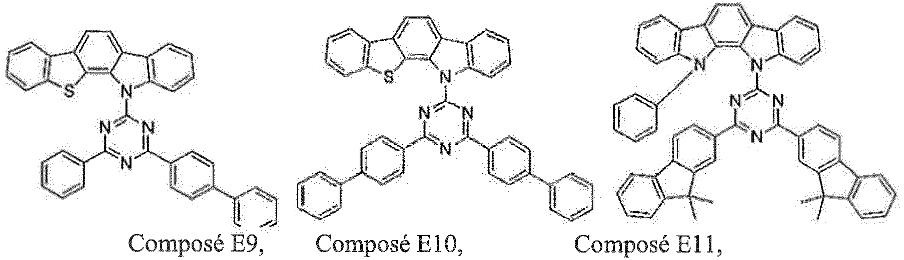

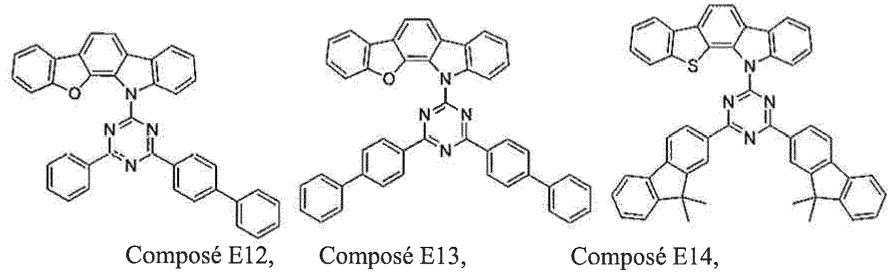

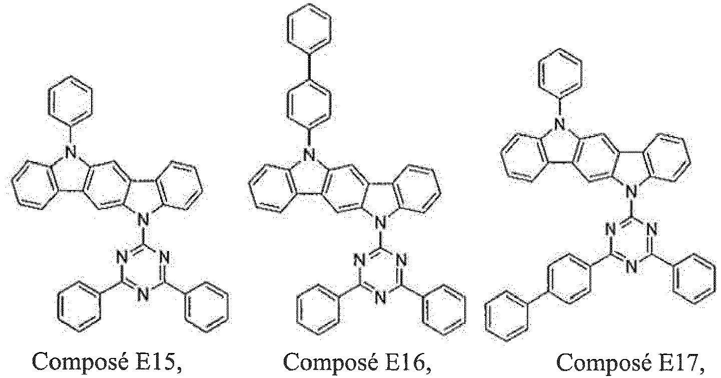

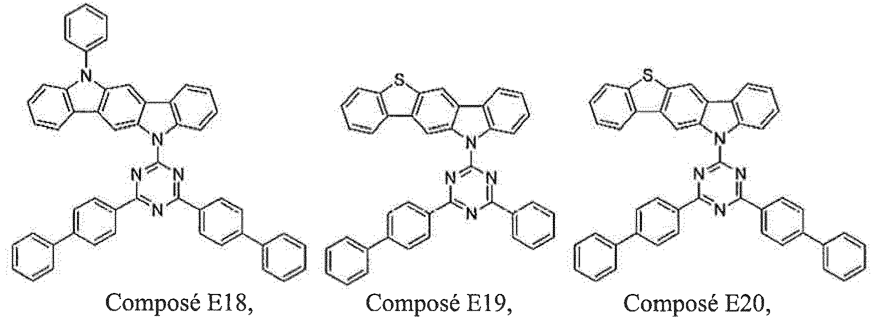

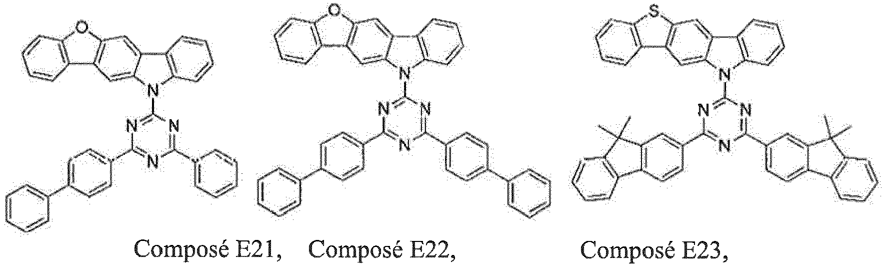

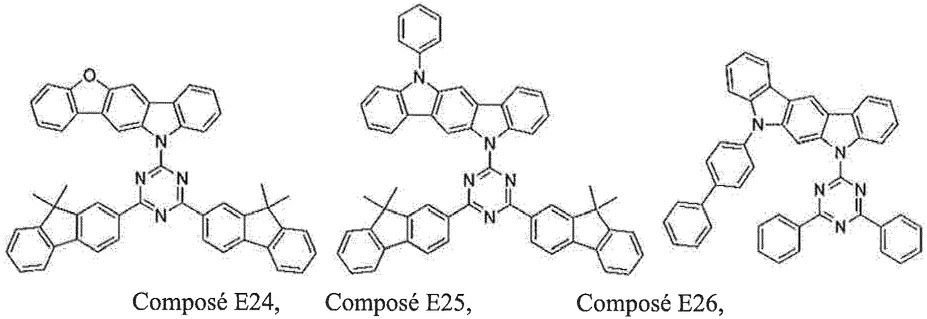

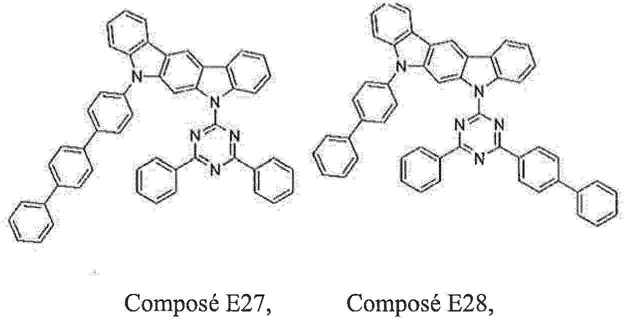

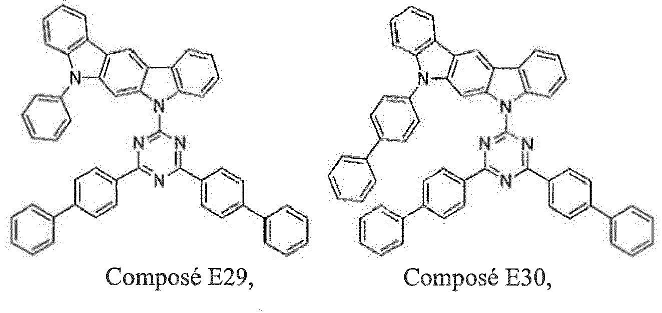

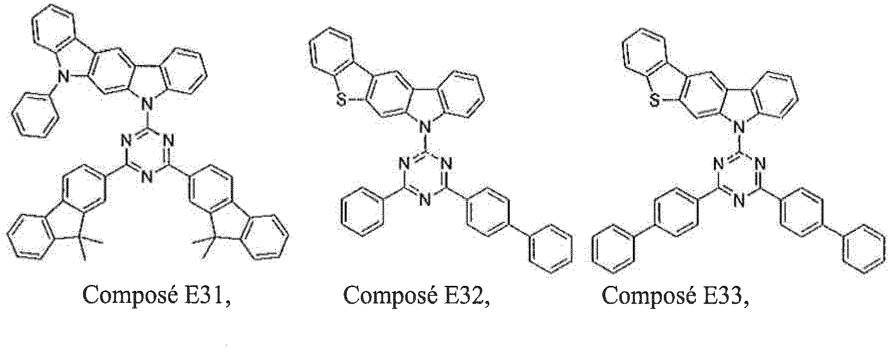

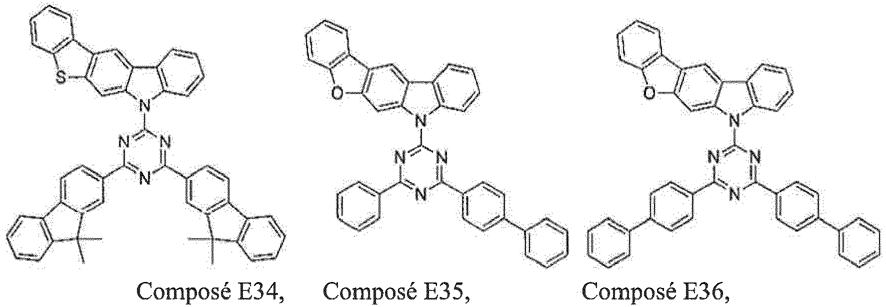

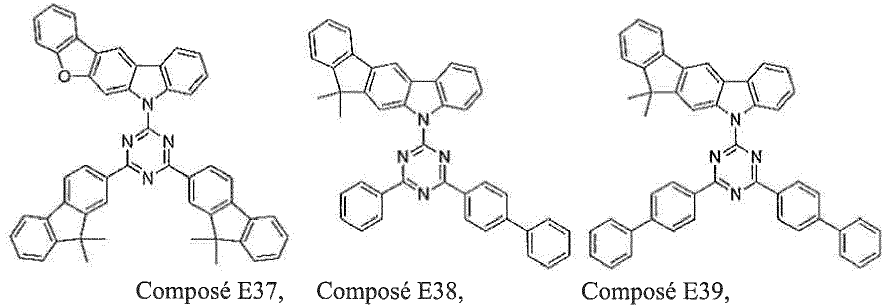

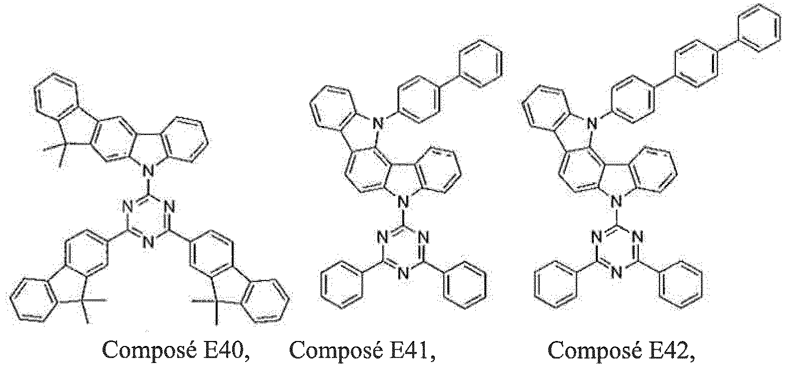

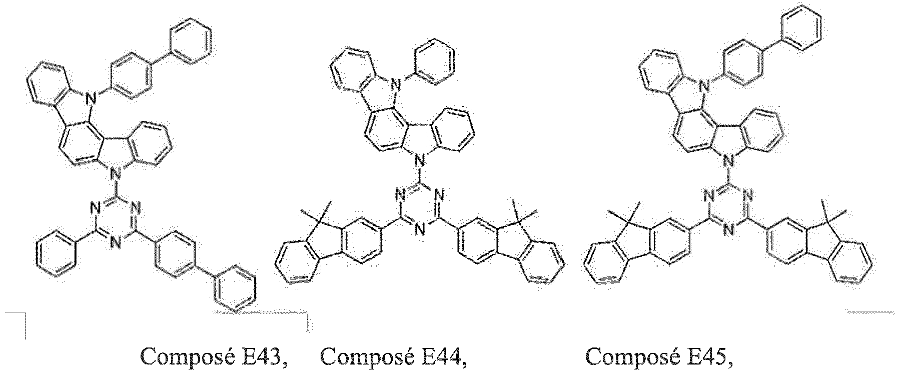

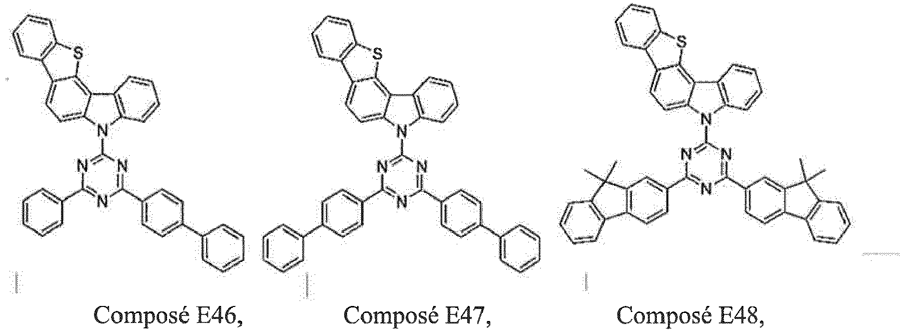

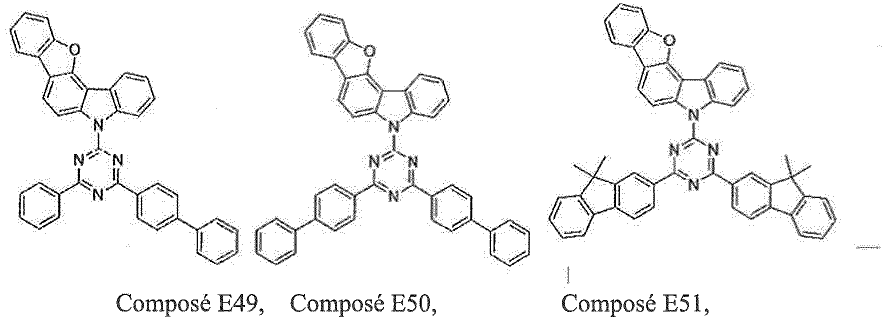

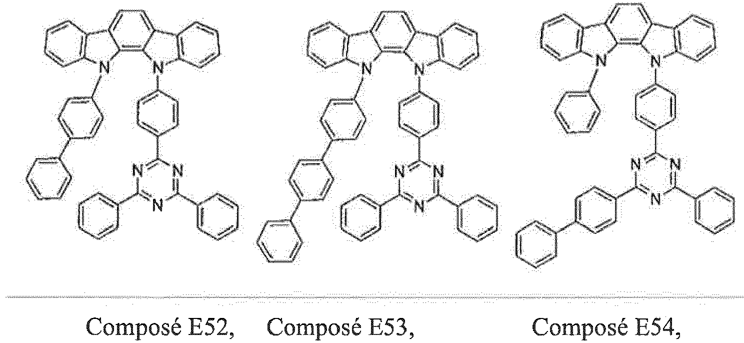

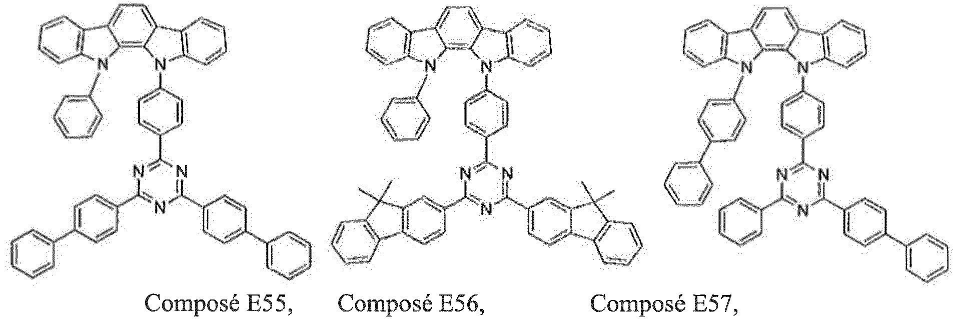

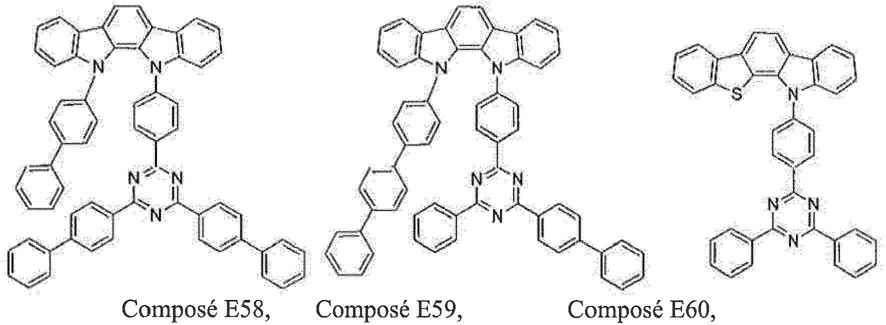

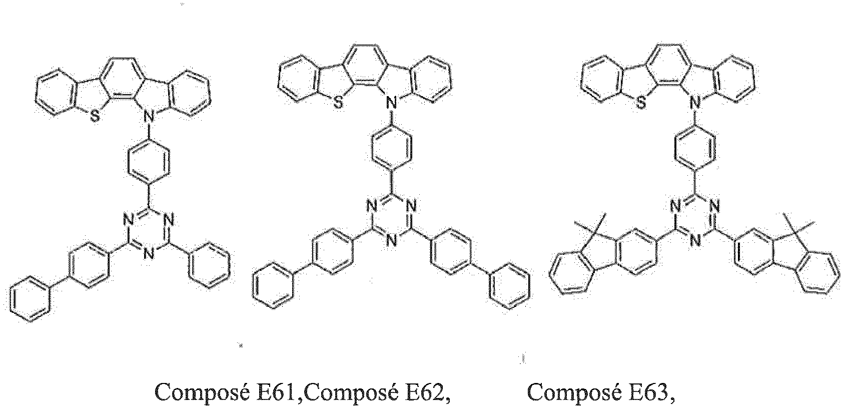

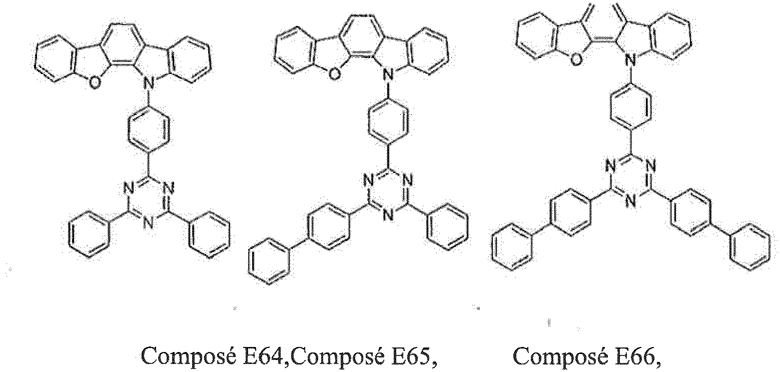

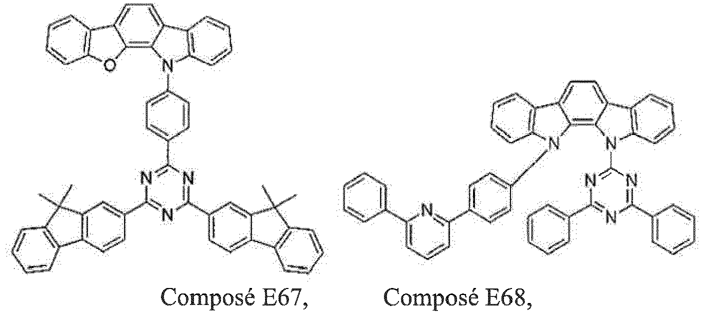

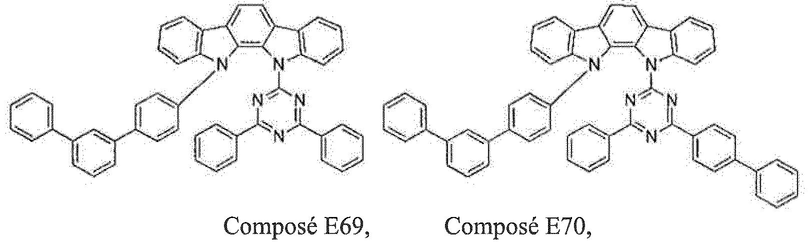

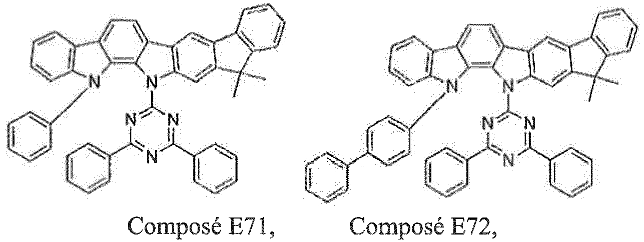

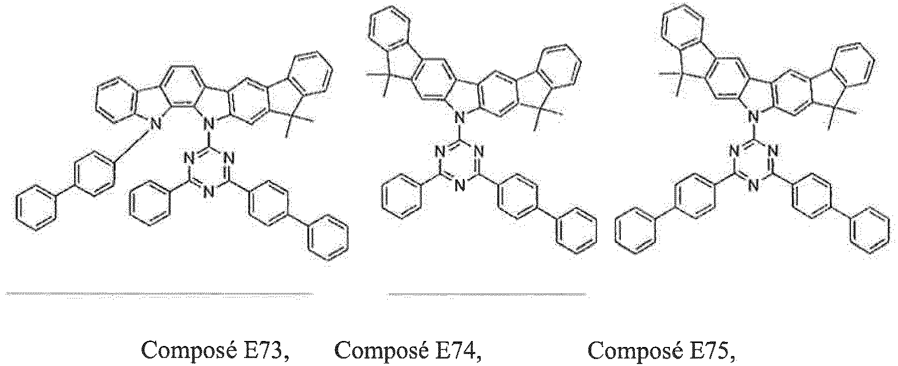

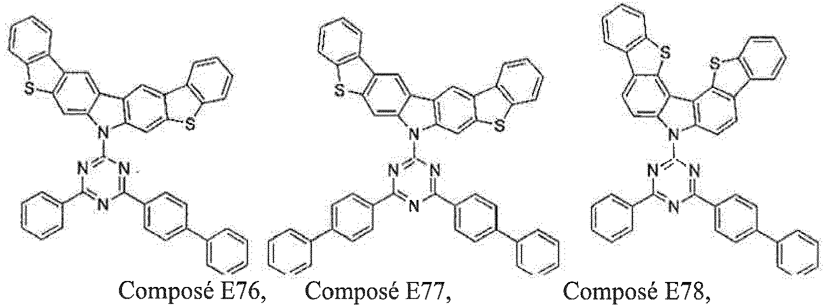

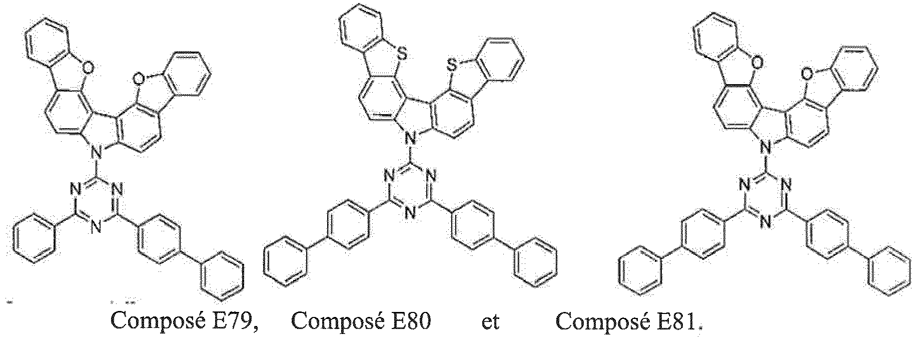

13. Composition de l'une quelconque des revendications 1 à 12, dans laquelle le mélange du premier composé et du deuxième composé est choisi dans le groupe constitué de : (composé 2 et composé E2), (composé 3 et composé E4), (composé 1 et composé E5), (composé 6 et composé E7), (composé 7 et composé E7), (composé 9 et composé E8), (composé 17 et composé E8), et (composé 15 et composé E18).

14. Premier dispositif comprenant un premier dispositif électroluminescent organique, le premier dispositif électroluminescent organique comprenant :
une anode ;
une cathode ; et
une couche organique, disposée entre l'anode et la cathode, comprenant une composition selon l'une quelconque des revendications 1 à 13.
